# EUROPEAN PATENT APPLICATION

(11) **EP 4 725 957 A1**
(43) Date of publication of application: **15.04.2026**
(21) Application number: 24818462.4
(22) Date of filing: 16.05.2024
(51) Int. Cl.: C07K 7/62, A61K 38/12, A61P 31/04

(54) **GROUP OF CYCLIC ALKALINE LIPOPEPTIDE COMPOUNDS, AND PREPARATION METHOD THEREFOR AND USE THEREOF**

(30) Priority: 07.06.2023 CN 202310668917
(71) Applicant: Institute of Medicinal Biotechnology, Chinese Academy of Medical Sciences, Beijing 100050 (CN)
(72) Inventor: LI, Zhuorong, Beijing 100050 (CN); CUI, Along, Beijing 100050 (CN); YANG, Hexian, Beijing 100050 (CN)
(74) Representative: Michalski Hüttermann & Partner Patentanwälte mbB
(86) International application number: PCT/CN2024/093551
(87) International publication number: WO 2024/250929

(57) **Abstract**

A cyclic basic lipopeptide compound, and a preparation method therefor and a use thereof. The compound has a structure represented by general formula I, wherein AA represents amino acid. The preparation method for a cyclic lipopeptide compound comprises the following steps: (1) reacting an Fmoc-AA-OP side chain free amino group of a protected basic amino acid with a halogenated resin to obtain Fmoc-AA-OP-resin; (2) coupling Fmoc-AA-OP-resin one by one to obtain a linear polypeptide-resin; (3) selectively removing a protective group from the linear polypeptide-resin and carrying out solid-phase cyclization on the linear polypeptide-resin to obtain a cyclic polypeptide-resin; (4) carrying out acidolysis and ether precipitation on the cyclic polypeptide-resin to obtain a cyclic polypeptide crude product; and (5) carrying out purification and/or salt conversion and lyophilization on the cyclic polypeptide crude product to obtain a cyclic polypeptide pure product. The cyclic lipopeptide compound can be used for preparing antibacterial drugs, and particularly used for preparing antibacterial drugs having improved antibacterial activity and reduced renal toxicity, comprising an antibacterial drug against carbapenem drug-resistant "super bacteria". R0-CO-AA1-AA2-D/L-AA3-ring(4-10)[Dab4-AA5-D/L-AA6-AA7-AA8-AA9-AA10] I.

## Description

### TECHNICAL FIELD

The present disclosure relates to a cyclic alkaline lipopeptide compound, a preparation method therefor and use thereof in the preparation of an antibacterial medicament, in particular to use thereof in the preparation of an antibacterial medicament with enhanced antibacterial activity and reduced nephrotoxicity, including the use in the preparation of broad-spectrum antibacterial medicaments against carbapenem-resistant "super bacteria" and *Pseudomonas aeruginosa,* and pharmaceutical compositions containing such compounds as active ingredients and use thereof. The present disclosure belongs to the field of biomedicine.

### BACKGROUND

Polymyxin, a cyclic alkaline lipopeptide antibiotic, was first reported in 1947. It refers to a series of cationic antimicrobial peptides produced by *Bacillus polymyxa,* including different structural types such as A, B, C, D, E, F, K, M, P, S and T, with a molecular weight of approximately 1200 Daltons. All polymyxin antibiotics share a common structural feature: they consist of three moieties, namely a cyclic heptapeptide, a linear tripeptide, and a side-chain acyl group linked to the linear tripeptide. They have similar antibacterial spectra and exert bactericidal effects by disrupting the cell membranes of Gram-negative bacteria, leading to the leakage of intracellular contents.

Polymyxin antibiotics have been used clinically since the 1950s. However, their clinical application has gradually declined due to their narrow antibacterial spectrum (effective only against Gram-negative bacteria), inherent nephrotoxicity, and more importantly, the emergence of new broad-spectrum antibacterial agents such as third-generation cephalosporins and carbapenems. In recent years, with the continuous emergence of multidrug-resistant Gram-negative bacteria, especially the increasing prevalence of carbapenem-resistant *Acinetobacter baumannii, Pseudomonas aeruginosa, Escherichia coli* and *Klebsiella pneumoniae,* polymyxins have become the last line of defense for the treatment of infections caused by carbapenem-resistant Gram-negative bacteria, thus regaining clinical attention.

Currently, the clinically used polymyxins are polymyxin B and colistin (polymyxin E), both of which are multi-component mixtures obtained by bacterial fermentation. The complex composition and severe nephrotoxicity of clinically used polymyxins have limited their clinical application. Therefore, the design and synthesis of cyclic antibacterial lipopeptides with high antibacterial activity and low nephrotoxicity are urgently needed.

Reported chemical preparation methods for polymyxin B include solid-phase condensation combined with liquid-phase cyclization strategies. Liquid-phase cyclization requires large amounts of solvents, complicates product separation and purification, and achieves a yield of less than 20%, which is even lower in actual synthesis processes. In the literature (de Visser P C, Kriek N M, van Hooft P A, et al. Solid-phase synthesis of polymyxin B1 and analogues via a safety-catch approach. J Pept Res, 2003, 61(6):298-306), Kenner's safety catch method was used for the solid-phase condensation and solid-phase cyclization to synthesize polymyxin B1, with an overall yield of merely 1.5%. The literature (Wei-Liang Xu, A-Long Cui, Xin-Xin Hu, et al. A new strategy for total solid-phase synthesis of polymyxins. Tetrahedron Letters, 2015, 56(33):4796-4799) reported the synthesis of polymyxin B2 and E2 via solid-phase condensation and solid-phase cyclization, achieving a yield of approximately 25%. WO2013156977A1 disclosed a method for solid-phase synthesis of insulin by linking the resin to the side-chain amino group of lysine. In the present disclosure, a protected alkaline amino acid Fmoc-AA-OP, which is structurally similar to lysine, is used to link the resin via its side-chain amino group, and solid-phase condensation and solid-phase cyclization are adopted to synthesize polymyxin derivatives. This method avoids the large consumption of solvents associated with liquid-phase cyclization, is environmentally friendly, and yields crude peptides with high purity that can be easily separated and purified, with an overall yield of over 30%.

Regarding the preparation of cyclic alkaline lipopeptide compounds, the present disclosure is the first to prepare novel derivatives with adjusted hydrophobicity of the side-chain acyl group (by changing R₀-CO-) by substituteing the length and volume of the side-chain acyl group. The novel derivatives also include those obtained by substituting the amino acids at positions 1 and/or 3 and/or 5 and/or 8 and/or 9 with alkaline or polar amino acids (by changing AA₁, AA₃, AA₅, AA₈, AA₉), those obtained by substituting the amino acids at positions 6 and/or 7 with hydrophobic or polar amino acids (by changing AA₆, AA₇), and those obtained by introducing strong polar groups such as sulfomethyl groups into the amino acid residues at positions 1, 2, 3, 5, 6, 7, 8, 9 and 10. By changing the alkalinity or hydrophobicity of the polymyxin molecule, the antibacterial activity is enhanced and nephrotoxicity is reduced.

Regarding the biological functions of the cyclic lipopeptide derivatives, the present disclosure has investigated their antibacterial activity and nephrotoxicity. Compared with positive controls, some of the cyclic lipopeptide derivatives exhibit improved antibacterial activity against Gram-negative bacteria and reduced nephrotoxicity. In particular, the compounds of the present disclosure possess broad-spectrum antibacterial activity against G⁻ bacteria including *Pseudomonas aeruginosa.*

### SUMMARY

It is an object of the present disclosure to provide a cyclic alkaline lipopeptide compound and a preparation method therefor, in particular to a method for synthesizing a cyclic lipopeptide compound by solid-phase condensation and solid-phase cyclization. The compound of the present disclosure has significant antibacterial effects, especially in the preparation of antibacterial medicaments against carbapenem-resistant "super bacteria".

In order to achieve the above object, the present disclosure adopts the following technical solutions:
The present disclosure provides a polymyxin compound or a pharmaceutically acceptable salt thereof, having a structure represented by general formula I:

R₀-CO-AA₁-AA₂-D/L-AA₃-ring(4-10)[Dab₄-AA₅-D/L-AA₆-AA₇-AA₈-AA₉-AA₁₀] I

Specifically, the present disclosure provides polymyxin compounds or pharmaceutically acceptable salts thereof according to the following embodiments.

A cyclic alkaline lipopeptide compound having the structure represented by General Formula I or a pharmaceutically acceptable salt thereof, wherein the compound consists of three moieties: a cyclic heptapeptide, a linear tripeptide, and a side-chain acyl chain (i.e., R₀-CO-) linked to the linear tripeptide; AA represents an amino acid; the carboxyl group of R₀-COOH condenses with the α-amino group of AA₁ to form an amide bond; the carboxyl group of AA₁ condenses with the α-amino group of AA₂ to form an amide bond; the carboxyl group of AA₂ condenses with the α-amino group of AA₃ to form an amide bond; the carboxyl group of AA₃ condenses with the α-amino group of Dab₄ to form an amide bond; the carboxyl group of Dab₄ condenses with the α-amino group of AA₅ to form an amide bond; the carboxyl group of AA₅ condenses with the α-amino group of AA₆ to form an amide bond; the carboxyl group of AA₆ condenses with the α-amino group of AA₇ to form an amide bond; the carboxyl group of AA₇ condenses with the α-amino group of AA₈ to form an amide bond; the carboxyl group of AA₈ condenses with the α-amino group of AA₉ to form an amide bond; the carboxyl group of AA₉ condenses with the α-amino group of AA₁₀ to form an amide bond; and the carboxyl group of AA₁₀ condenses with the γ-amino group of Dab₄ to form an amide bond,

R₀-CO-AA₁-AA₂-D/L-AA₃-ring(4-10)[Dab₄-AA₅-D/L-AA₆-AA₇-AA₈-AA₉-AA₁₀] I

wherein:
R₀ is selected from the group consisting of (C₆-C₁₁) linear-chain alkyl and (C₇-C₁₂) branched-chain alkyl;
AA₁ and AA₃ are each independently selected from the group consisting of Ser (serine), Ser(CH₂SO₃H) (2-amino-3-sulfomethoxypropanoic acid), Dap (2,3-diaminopropanoic acid), Dap(CH₂SO₃H) (2-amino-3-sulfomethylaminopropanoic acid), Dab (2,4-diaminobutyric acid), Dab(CH₂SO₃H) (2-amino-4-sulfomethylaminobutyric acid), Orn (ornithine) and Lys (lysine); the amino acid at position 1 has an L configuration, and the amino acid at position 3 has a D or L configuration;
AA₂ and AA₁₀ are each independently selected from the group consisting of Thr (threonine) and Thr(CH₂SO₃H) (2-amino-3-sulfomethoxybutanoic acid); the amino acids at positions 2 and 10 have an L configuration;
Dab₄ has an L configuration;
AA₅, AA₈ and AA₉ are each independently selected from the group consisting of Dap (2,3-diaminopropanoic acid), Dap(CH₂SO₃H) (2-amino-3-(sulfomethylamino)propanoic acid, Dab (2,4-diaminobutyric acid), Dab(CH₂SO₃H) (2-amino-4-(sulfomethylamino)butyric acid) and Orn (ornithine); the amino acids at positions 5, 8 and 9 are in L-configuration;
AA₆ and AA₇ are each independently selected from the group consisting of Phe (phenylalanine), Phe(4-CH₂SO₃) (4-(sulfomethyl)phenylalanine), Leu (leucine), Thr (threonine) and Thr(CH₂SO₃H) (2-amino-3-(sulfomethoxy)butanoic acid); the amino acid at position 6 has a D or L configuration, and the amino acid at position 7 has an L configuration; the linear-chain alkyl may be hexyl, heptyl, octyl or nonyl; the branched-chain alkyl may be 5-methylhexyl, 5-methylheptyl, 6-methylheptyl or 6-methyloctyl, for example, (S)-5-methylheptyl.

Specifically, in the present disclosure, the antibacterial lipopeptide compound is selected from the following compounds 1 to 120:
(1) (S)-6-methyloctanoyl-Dab-Thr-D-Dap-ring(4-10)[Dab-Dab-D-Phe-Thr-Dab-Dab-Thr];
(2) 6-methylheptanoyl-Dab-Thr-D-Dap-ring(4-10)[Dab-Dab-D-Phe-Thr-Dab-Dab-Thr];
(3) Octanoyl-Dab-Thr-D-Dap-ring(4-10)[Dab-Dab-D-Phe-Thr-Dab-Dab-Thr];
(4) Heptanoyl-Dab-Thr-D-Dap-ring(4-10)[Dab-Dab-D-Phe-Thr-Dab-Dab-Thr];
(5) Nonanoyl-Dab-Thr-D-Dap-ring(4-10)[Dab-Dab-D-Phe-Thr-Dab-Dab-Thr];
(6) (S)-6-methyloctanoyl-Dab-Thr-Dap-ring(4-10)[Dab-Dab-D-Phe-Thr-Dab-Dab-Thr];
(7) 6-methylheptanoyl-Dab-Thr-Dap-ring(4-10)[Dab-Dab-D-Phe-Thr-Dab-Dab-Thr];
(8) Octanoyl-Dab-Thr-Dap-ring(4-10)[Dab-Dab-D-Phe-Thr-Dab-Dab-Thr];
(9) Heptanoyl-Dab-Thr-Dap-ring(4-10)[Dab-Dab-D-Phe-Thr-Dab-Dab-Thr];
(10) Nonanoyl-Dab-Thr-Dap-ring(4-10)[Dab-Dab-D-Phe-Thr-Dab-Dab-Thr];
(11) (S)-6-methyloctanoyl-Dab-Thr-D-Dab-ring(4-10)[Dab-Dab-D-Phe-Thr-Dab-Dab-Thr];
(12) 6-methylheptanoyl-Dab-Thr-D-Dab-ring(4-10)[Dab-Dab-D-Phe-Thr-Dab-Dab-Thr];
(13) Octanoyl-Dab-Thr-D-Dab-ring(4-10)[Dab-Dab-D-Phe-Thr-Dab-Dab-Thr];
(14) Heptanoyl-Dab-Thr-D-Dab-ring(4-10)[Dab-Dab-D-Phe-Thr-Dab-Dab-Thr];
(15) Nonanoyl-Dab-Thr-D-Dab-ring(4-10)[Dab-Dab-D-Phe-Thr-Dab-Dab-Thr];
(16) (S)-6-methyloctanoyl-Dab-Thr-Dab-ring(4-10)[Dab-Dab-D-Phe-Thr-Dab-Dab-Thr];
(17) 6-methylheptanoyl-Dab-Thr-Dab-ring(4-10)[Dab-Dab-D-Phe-Thr-Dab-Dab-Thr];
(18) Octanoyl-Dab-Thr-Dab-ring(4-10)[Dab-Dab-D-Phe-Thr-Dab-Dab-Thr];
(19) Heptanoyl-Dab-Thr-Dab-ring(4-10)[Dab-Dab-D-Phe-Thr-Dab-Dab-Thr];
(20) Nonanoyl-Dab-Thr-Dab-ring(4-10)[Dab-Dab-D-Phe-Thr-Dab-Dab-Thr];
(21) (S)-6-methyloctanoyl-Dab-Thr-D-Orn-ring(4-10)[Dab-Dab-D-Phe-Thr-Dab-Dab-Thr];
(22) 6-methylheptanoyl-Dab-Thr-D-Orn-ring(4-10)[Dab-Dab-D-Phe-Thr-Dab-Dab-Thr];
(23) Octanoyl-Dab-Thr-D-Orn-ring(4-10)[Dab-Dab-D-Phe-Thr-Dab-Dab-Thr];
(24) Heptanoyl-Dab-Thr-D-Orn-ring(4-10)[Dab-Dab-D-Phe-Thr-Dab-Dab-Thr];
(25) Nonanoyl-Dab-Thr-D-Orn-ring(4-10)[Dab-Dab-D-Phe-Thr-Dab-Dab-Thr];
(26) (S)-6-methyloctanoyl-Dab-Thr-Orn-ring(4-10)[Dab-Dab-D-Phe-Thr-Dab-Dab-Thr];
(27) 6-methylheptanoyl-Dab-Thr-Orn-ring(4-10)[Dab-Dab-D-Phe-Thr-Dab-Dab-Thr];
(28) Octanoyl-Dab-Thr-Orn-ring(4-10)[Dab-Dab-D-Phe-Thr-Dab-Dab-Thr];
(29) Heptanoyl-Dab-Thr-Orn-ring(4-10)[Dab-Dab-D-Phe-Thr-Dab-Dab-Thr];
(30) Nonanoyl-Dab-Thr-Orn-ring(4-10)[Dab-Dab-D-Phe-Thr-Dab-Dab-Thr];
(31) (S)-6-methyloctanoyl-Dab-Thr-D-Dap-ring(4-10)[Dab-Dab-D-Phe-Leu-Dab-Dab-Thr];
(32) 6-methylheptanoyl-Dab-Thr-D-Dap-ring(4-10)[Dab-Dab-D-Phe-Leu-Dab-Dab-Thr];
(33) Octanoyl-Dab-Thr-D-Dap-ring(4-10)[Dab-Dab-D-Phe-Leu-Dab-Dab-Thr];
(34) Heptanoyl-Dab-Thr-D-Dap-ring(4-10)[Dab-Dab-D-Phe-Leu-Dab-Dab-Thr];
(35) Nonanoyl-Dab-Thr-D-Dap-ring(4-10)[Dab-Dab-D-Phe-Leu-Dab-Dab-Thr];
(36) (S)-6-methyloctanoyl-Dab-Thr-Dap-ring(4-10)[Dab-Dab-D-Phe-Leu-Dab-Dab-Thr];
(37) 6-methylheptanoyl-Dab-Thr-Dap-ring(4-10)[Dab-Dab-D-Phe-Leu-Dab-Dab-Thr];
(38) Octanoyl-Dab-Thr-Dap-ring(4-10)[Dab-Dab-D-Phe-Leu-Dab-Dab-Thr];
(39) Heptanoyl-Dab-Thr-Dap-ring(4-10)[Dab-Dab-D-Phe-Leu-Dab-Dab-Thr];
(40) Nonanoyl-Dab-Thr-Dap-ring(4-10)[Dab-Dab-D-Phe-Leu-Dab-Dab-Thr];
(41) (S)-6-methyloctanoyl-Dab-Thr-D-Orn-ring(4-10)[Dab-Dab-D-Phe-Leu-Dab-Dab-Thr];
(42) 6-methylheptanoyl-Dab-Thr-D-Orn-ring(4-10)[Dab-Dab-D-Phe-Leu-Dab-Dab-Thr];
(43) Octanoyl-Dab-Thr-D-Orn-ring(4-10)[Dab-Dab-D-Phe-Leu-Dab-Dab-Thr];
(44) Heptanoyl-Dab-Thr-D-Orn-ring(4-10)[Dab-Dab-D-Phe-Leu-Dab-Dab-Thr];
(45) Nonanoyl-Dab-Thr-D-Orn-ring(4-10)[Dab-Dab-D-Phe-Leu-Dab-Dab-Thr];
(46) (S)-6-methyloctanoyl-Dab-Thr-Orn-ring(4-10)[Dab-Dab-D-Phe-Leu-Dab-Dab-Thr];
(47) 6-methylheptanoyl-Dab-Thr-Orn-ring(4-10)[Dab-Dab-D-Phe-Leu-Dab-Dab-Thr];
(48) Octanoyl-Dab-Thr-Orn-ring(4-10)[Dab-Dab-D-Phe-Leu-Dab-Dab-Thr];
(49) Heptanoyl-Dab-Thr-Orn-ring(4-10)[Dab-Dab-D-Phe-Leu-Dab-Dab-Thr];
(50) Nonanoyl-Dab-Thr-Orn-ring(4-10)[Dab-Dab-D-Phe-Leu-Dab-Dab-Thr];
(51) (S)-6-methyloctanoyl-Dap-Thr-D-Ser-ring(4-10)[Dab-Dab-D-Phe-Thr-Dab-Dab-Thr];
(52) 6-methylheptanoyl-Dap-Thr-D-Ser-ring(4-10)[Dab-Dab-D-Phe-Thr-Dab-Dab-Thr];
(53) Octanoyl-Dap-Thr-D-Ser-ring(4-10)[Dab-Dab-D-Phe-Thr-Dab-Dab-Thr];
(54) Heptanoyl-Dap-Thr-D-Ser-ring(4-10)[Dab-Dab-D-Phe-Thr-Dab-Dab-Thr];
(55) Nonanoyl-Dap-Thr-D-Ser-ring(4-10)[Dab-Dab-D-Phe-Thr-Dab-Dab-Thr];
(56) (S)-6-methyloctanoyl-Orn-Thr-D-Ser-ring(4-10)[Dab-Dab-D-Phe-Thr-Dab-Dab-Thr];
(57) 6-methylheptanoyl-Orn-Thr-D-Ser-ring(4-10)[Dab-Dab-D-Phe-Thr-Dab-Dab-Thr];
(58) Octanoyl-Orn-Thr-D-Ser-ring(4-10)[Dab-Dab-D-Phe-Thr-Dab-Dab-Thr];
(59) Heptanoyl-Orn-Thr-D-Ser-ring(4-10)[Dab-Dab-D-Phe-Thr-Dab-Dab-Thr];
(60) Nonanoyl-Orn-Thr-D-Ser-ring(4-10)[Dab-Dab-D-Phe-Thr-Dab-Dab-Thr];
(61) (S)-6-methyloctanoyl-Dab-Thr-D-Ser-ring(4-10)[Dab-Dap-D-Phe-Thr-Dab-Dab-Thr];
(62) 6-methylheptanoyl-Dab-Thr-D-Ser-ring(4-10)[Dab-Dap-D-Phe-Thr-Dab-Dab-Thr];
(63) Octanoyl-Dab-Thr-D-Ser-ring(4-10)[Dab-Dap-D-Phe-Thr-Dab-Dab-Thr];
(64) Heptanoyl-Dab-Thr-D-Ser-ring(4-10)[Dab-Dap-D-Phe-Thr-Dab-Dab-Thr];
(65) Nonanoyl-Dab-Thr-D-Ser-ring(4-10)[Dab-Dap-D-Phe-Thr-Dab-Dab-Thr];
(66) (S)-6-methyloctanoyl-Dab-Thr-D-Ser-ring(4-10)[Dab-Orn-D-Phe-Thr-Dab-Dab-Thr];
(67) 6-methylheptanoyl-Dab-Thr-D-Ser-ring(4-10)[Dab-Orn-D-Phe-Thr-Dab-Dab-Thr];
(68) Octanoyl-Dab-Thr-D-Ser-ring(4-10)[Dab-Orn-D-Phe-Thr-Dab-Dab-Thr];
(69) Heptanoyl-Dab-Thr-D-Ser-ring(4-10)[Dab-Orn-D-Phe-Thr-Dab-Dab-Thr];
(70) Nonanoyl-Dab-Thr-D-Ser-ring(4-10)[Dab-Orn-D-Phe-Thr-Dab-Dab-Thr];
(71) (S)-6-methyloctanoyl-Dab-Thr-D-Ser-ring(4-10)[Dab-Dab-D-Phe-Thr-Dap-Dab-Thr];
(72) 6-methylheptanoyl-Dab-Thr-D-Ser-ring(4-10)[Dab-Dab-D-Phe-Thr-Dap-Dab-Thr];
(73) Octanoyl-Dab-Thr-D-Ser-ring(4-10)[Dab-Dab-D-Phe-Thr-Dap-Dab-Thr];
(74) Heptanoyl-Dab-Thr-D-Ser-ring(4-10)[Dab-Dab-D-Phe-Thr-Dap-Dab-Thr];
(75) Nonanoyl-Dab-Thr-D-Ser-ring(4-10)[Dab-Dab-D-Phe-Thr-Dap-Dab-Thr];
(76) (S)-6-methyloctanoyl-Dab-Thr-D-Ser-ring(4-10)[Dab-Dab-D-Phe-Thr-Orn-Dab-Thr];
(77) 6-methylheptanoyl-Dab-Thr-D-Ser-ring(4-10)[Dab-Dab-D-Phe-Thr-Orn-Dab-Thr];
(78) Octanoyl-Dab-Thr-D-Ser-ring(4-10)[Dab-Dab-D-Phe-Thr-Orn-Dab- Thr];
(79) Heptanoyl-Dab-Thr-D-Ser-ring(4-10)[Dab-Dab-D-Phe-Thr-Orn-Dab-Thr];
(80) Nonanoyl-Dab-Thr-D-Ser-ring(4-10)[Dab-Dab-D-Phe-Thr-Orn-Dab-Thr];
(81) (S)-6-methyloctanoyl-Dab-Thr-D-Ser-ring(4-10)[Dab-Dab-D-Phe-Thr-Dab-Dap-Thr];
(82) 6-methylheptanoyl-Dab-Thr-D-Ser-ring(4-10)[Dab-Dab-D-Phe-Thr-Dab-Dap-Thr];
(83) Octanoyl-Dab-Thr-D-Ser-ring(4-10)[Dab-Dab-D-Phe-Thr-Dab-Dap-Thr];
(84) Heptanoyl-Dab-Thr-D-Ser-ring(4-10)[Dab-Dab-D-Phe-Thr-Dab-Dap-Thr];
(85) Nonanoyl-Dab-Thr-D-Ser-ring(4-10)[Dab-Dab-D-Phe-Thr-Dab-Dap-Thr];
(86) (S)-6-methyloctanoyl-Dab-Thr-D-Ser-ring(4-10)[Dab-Dab-D-Phe-Thr-Dab-Orn-Thr];
(87) 6-methylheptanoyl-Dab-Thr-D-Ser-ring(4-10)[Dab-Dab-D-Phe-Thr-Dab-Orn-Thr];
(88) Octanoyl-Dab-Thr-D-Ser-ring(4-10)[Dab-Dab-D-Phe-Thr-Dab-Orn-Thr];
(89) Heptanoyl-Dab-Thr-D-Ser-ring(4-10)[Dab-Dab-D-Phe-Thr-Dab-Orn-Thr];
(90) Nonanoyl-Dab-Thr-D-Ser-ring(4-10)[Dab-Dab-D-Phe-Thr-Dab-Orn-Thr];
(91) (S)-6-methyloctanoyl-Dap-Thr-D-Dab-ring(4-10)[Dab-Dab-D-Phe-Leu-Dab-Dab-Thr];
(92) 6-methylheptanoyl-Dap-Thr-D-Dab-ring(4-10)[Dab-Dab-D-Phe-Leu-Dab-Dab-Thr];
(93) Octanoyl-Dap-Thr-D-Dab-ring(4-10)[Dab-Dab-D-Phe-Leu-Dab-Dab-Thr];
(94) Heptanoyl-Dap-Thr-D-Dab-ring(4-10)[Dab-Dab-D-Phe-Leu-Dab-Dab-Thr];
(95) Nonanoyl-Dap-Thr-D-Dab-ring(4-10)[Dab-Dab-D-Phe-Leu-Dab-Dab-Thr];
(96) (S)-6-methyloctanoyl-Dap-Thr-D-Dab-ring(4-10)[Dab-Dab-D-Leu-Leu-Dab-Dab-Thr];
(97) 6-methylheptanoyl-Dap-Thr-D-Dab-ring(4-10)[Dab-Dab-D-Leu-Leu-Dab-Dab-Thr];
(98) Octanoyl-Dap-Thr-D-Dab-ring(4-10)[Dab-Dab-D-Leu-Leu-Dab-Dab-Thr];
(99) Heptanoyl-Dap-Thr-D-Dab-ring(4-10)[Dab-Dab-D-Leu-Leu-Dab-Dab-Thr];
(100) Nonanoyl-Dap-Thr-D-Dab-ring(4-10)[Dab-Dab-D-Leu-Leu-Dab-Dab-Thr];
(101) (S)-6-methyloctanoyl-Dab-Thr-D-Dap-ring(4-10)[Dab-Dab-Phe-Thr-Dab-Dab-Thr];
(102) 6-methylheptanoyl-Dab-Thr-D-Dap-ring(4-10)[Dab-Dab-Phe-Thr-Dab-Dab-Thr];
(103) 6-methylheptanoyl-Dab-Thr-D-Ser(CH₂SO₃H)-ring(4-10)[Dab-Dab-D-Phe-Thr-Dab-Dab-Thr];
(104) 6-methylheptanoyl-Dab-Thr(CH₂SO₃H)-D-Ser-ring(4-10)[Dab-Dab-D-Phe-Thr-Dab-Dab-Thr];
(105) 6-methylheptanoyl-Dab-Thr-D-Ser-ring(4-10)[Dab-Dab-D-Phe-Thr(CH₂SO₃H)-Dab-Dab-Thr];
(106) 6-methylheptanoyl-Dab-Thr-D-Ser-ring(4-10)[Dab-Dab-D-Phe-Thr-Dab-Dab-Thr(CH₂SO₃H)];
(107) 6-methylheptanoyl-Dab-Thr-D-Ser-ring(4-10)[Dab-Dab-D-Phe(4-CH₂SO₃H)-Thr-Dab-Dab-Thr];
(108) 6-methylheptanoyl-Dab(CH₂SO₃H)-Thr-D-Ser-ring(4-10)[Dab-Dab-D-Phe-Thr-Dab-Dab-Thr];
(109) 6-methylheptanoyl-Dab-Thr-D-Ser-ring(4-10)[Dab-Dab(CH₂SO₃H)-D-Phe-Thr-Dab-Dab-Thr];
(110) 6-methylheptanoyl-Dab-Thr-D-Ser-ring(4-10)[Dab-Dab-D-Phe-Thr-Dab(CH₂SO₃H)-Dab-Thr];
(111) 6-methylheptanoyl-Dab-Thr-D-Ser-ring(4-10)[Dab-Dab-D-Phe-Thr-Dab-Dab(CH₂SO₃H)-Thr];
(112) 6-methylheptanoyl-Dab-Thr-Dap(CH₂SO₃H)-ring(4-10)[Dab-Dab-D-Phe-Thr-Dab-Dab-Thr];
(113) 6-methylheptanoyl-Dab-Thr(CH₂SO₃H)-Dap-ring(4-10)[Dab-Dab-D-Phe-Thr-Dab-Dab-Thr];
(114) 6-methylheptanoyl-Dab-Thr-Dap-ring(4-10)[Dab-Dab-D-Phe-Thr(CH₂SO₃H)-Dab-Dab-Thr];
(115) 6-methylheptanoyl-Dab-Thr-Dap-ring(4-10) Dab-Dab-D-Phe-Thr-Dab-Dab-Thr(CH₂SO₃H)];
(116) 6-methylheptanoyl-Dab-Thr-Dap-ring(4-10)[Dab-Dab-D-Phe(4-CH₂SO₃H)-Thr-Dab-Dab-Thr];
(117) 6-methylheptanoyl-Dab(CH₂SO₃H)-Thr-Dap-ring(4-10)[Dab-Dab-D-Phe-Thr-Dab-Dab-Thr];
(118) 6-methylheptanoyl-Dab-Thr-Dap-ring(4-10)[Dab-Dab(CH₂SO₃H)-D-Phe-Thr-Dab-Dab-Thr];
(119) 6-methylheptanoyl-Dab-Thr-Dap-ring(4-10)[Dab-Dab-D-Phe-Thr-Dab(CH₂SO₃H)-Dab-Thr];
(120) 6-methylheptanoyl-Dab-Thr-Dap-ring(4-10)[Dab-Dab-D-Phe-Thr-Dab-Dab(CH₂SO₃H)-Thr].

Wherein, the term "ring(4-10)" refers to the heptapeptide ring formed by linking the carboxyl group of the amino acid at position 10 to the side-chain amino group of the alkaline amino acid at position 4 via an amide bond; its structure is shown in General Formula I.

The configuration of D-amino acids is denoted by "D". In the absence of specified configuration, the amino acid shall be deemed to have the L configuration. Dab stands for 2,4-diaminobutyric acid, Orn stands for 2,5-diaminopentanoic acid, and Dap stands for 2,3-diaminopropionic acid.

For the cyclic alkaline lipopeptide compound or the pharmaceutically acceptable salt thereof of the present disclosure, the pharmaceutically acceptable salt of the compound of general formula I comprises a salt formed by the compound of general formula I and an acid, and the acid is selected from the group consisting of inorganic acids and organic acids; the inorganic acids are preferably perchloric acid, hydroiodic acid, hydrobromic acid, hydrochloric acid, sulfuric acid, nitric acid or phosphoric acid; the organic acids are preferably acetic acid, trifluoroacetic acid, lactic acid, succinic acid, fumaric acid, maleic acid, citric acid, benzoic acid, methanesulfonic acid or p-toluenesulfonic acid.

The present disclosure further provides a method for solid-phase synthesis of a cyclic lipopeptide compound or a pharmaceutically acceptable salt thereof. The method is used to prepare a polymyxin compound or a pharmaceutically acceptable salt thereof via solid-phase condensation and solid-phase cyclization, which includes the following steps:
(1) reacting an Fmoc-AA-OP side chain free amino group of a protected alkaline amino acid with a halogenated resin to obtain Fmoc-AA-OP-resin; wherein P is a carboxyl-protecting group selected from allyl and benzyl (Bn); when Fmoc-AA-OP is Fmoc-Dab-OP, the structure thereof is represented by formula II; when Fmoc-AA-OP is Fmoc-Dap-OP, the structure thereof is represented by formula III; when Fmoc-AA-OP is Fmoc-Orn-OP, the structure thereof is represented by formula IV: 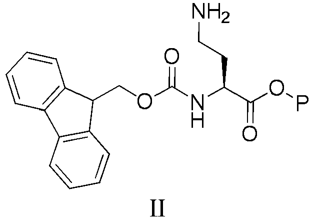 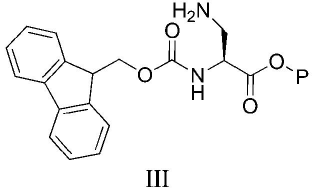 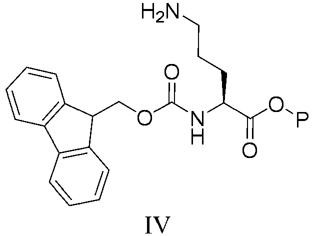
(2) coupling Fmoc-AA-OP-resin one by one to obtain a linear polypeptide-resin;
(3) selectively removing a protective group from the linear polypeptide-resin and carrying out solid-phase cyclization on the linear polypeptide-resin to obtain a cyclic polypeptide-resin;
(4) carrying out acidolysis on the cyclic polypeptide-resin to obtain a cyclic polypeptide crude product;
(5) carrying out purification and/or salt conversion and lyophilization on the cyclic polypeptide crude product to obtain a cyclic polypeptide pure product.

### Regarding step (1):

The halogenated resin described in the step (1) is selected from the group consisting of trityl chloride resin, 4-methyltrityl chloride resin, 4-methoxytrityl chloride resin, 2-chlorotrityl chloride resin, bromo-(4-methylphenyl)-methyl resin, or bromo-(4-methoxyphenyl)-methyl resin; for example, the resin is one or more of 2-chlorotrityl chloride resin.

The degree of substitution of the halogenated resin is 0.1-1.6 mmol/g, preferably 0.5-1.0 mmol/g.

The dosage of each Fmoc-protected amino acid is 1.2-6 times the total molar amount of the loaded resin, preferably 2.0-4.0 times.

The base is selected from at least one of N, N-diisopropylethylamine (DIEA), triethylamine (TEA) and pyridine, preferably DIEA; the molar dosage of the base is 1.5-3 times the molar amount of the Fmoc-protected amino acid, preferably, 2 times the molar amount of the Fmoc-protected amino acid.

The substitution reaction time is 1-12 h, preferably 2-3 h.

### Regarding step (2):

The reagent for removing the α-amino Fmoc protecting group in the step (2) includes, but is not limited to, a solution of piperidine (PIP) in N,N-dimethylformamide (DMF), with a concentration of PIP in DMF at 10-30%, preferably 20%. The dosage of the deprotection reagent is 5-15 mL per gram of the loaded resin, preferably 10 mL per gram of the loaded resin. The deprotection reaction time is 10-60 min, preferably 10-20 min. The reagent for removing the ivDde or Dde protecting group on the side-chain amino group of the amino acid at position 4 includes, but is not limited to, a solution of hydrazine hydrate in DMF, with a concentration of hydrazine hydrate in DMF at 1-10%, preferably 2%. The dosage of the deprotection reagent is 5-15 mL per gram of the loaded resin, preferably 10 mL per gram of the loaded resin. The deprotection reaction time is 30-100 min, preferably 30-60 min.

The coupling agent used in the coupling reaction is selected from at least one of N,N-diisopropylcarbodiimide (DIC), N,N-dicyclohexylcarbodiimide (DCC), 1-ethyl-3-(3-dimethylaminopropyl)carbodiimide hydrochloride (EDC), O-benzotriazole-N,N,N',N'-tetramethyluronium hexafluorophosphate (HBTU), 6-chloro-benzotriazole-1,1,3,3-tetramethyluronium hexafluorophosphate (HCTU), 2-(7-azabenzotriazol-1-yl)-N,N,N',N'-tetramethyluronium hexafluorophosphate (HATU), O-benzotriazole-N,N,N',N'-tetramethyluronium tetrafluoroborate (TBTU), and benzotriazol-1-yl-oxytripyrrolidinophosphonium hexafluorophosphate (PyBOP), preferably HCTU.

The molar dosage of the coupling agent is 1.2-6 times the total molar amount of the loaded resin, preferably 2.0-4.0 times.

The activator is selected from at least one of 1-hydroxybenzotriazole (HOBT), 6-chloro-1-hydroxybenzotriazole (Cl-HOBT), and 1-hydroxy-7-azabenzotriazole (HOAT), preferably HOBT.

The molar dosage of the activator is 1.2-6 times the total molar amount of the loaded resin, preferably 2.0-4.0 times.

The coupling reaction time is 60-300 min, preferably 60-120 min.

For some coupling agents used in the coupling reaction, a catalyst is required; the catalyst is an organic base selected from N, N-diisopropylethylamine (DIEA), triethylamine (TEA), and N-methylmorpholine (NMM), preferably DIEA.

The solvent is an aprotic polar solvent selected from N, N-dimethylformamide (DMF), N-methylpyrrolidone (NMP), or a mixture thereof, preferably DMF.

### Regarding step (3):

The reagent for removing the allyl protecting group on the carboxyl group in the step (3) is a solution of tetrakis(triphenylphosphine)palladium/phenylsilane in a mixed solvent of DCM and DMF (DCM:DMF = 5:5, v/v). The molar dosage of tetrakis(triphenylphosphine)palladium is 0.1-2 times the total molar amount of the loaded resin, preferably 0.1-0.3 times. The molar dosage of phenylsilane is 2-10 times the total molar amount of the loaded resin, preferably 3-7 times. The dosage of the deprotection reagent is 10-30 mL per gram of the loaded resin, preferably 20 mL per gram of the loaded resin. The deprotection reaction time is 60-300 min, preferably 60-120 min. The reagent for removing the carboxyl benzyl protecting group is H₂ and a 10% Pd/C ethanol solution. The molar dosage of 10% Pd/C is 0.1-2 times the total molar amount of the loaded resin, preferably 0.1-0.3 times. The deprotection reaction time is 30-100 min, preferably 30-60 min.

The solid-phase cyclization coupling agent is selected from the group consisting of (3H-1,2,3-triazolo[4,5-b]pyridin-3-yloxy)tripyrrolidinophosphonium hexafluorophosphate (PyAOP) and benzotriazol-1-yl-oxytripyrrolidinophosphonium hexafluorophosphate (PyBOP), preferably (3H-1,2,3-triazolo[4,5-b]pyridin-3-yloxy)tripyrrolidinophosphonium hexafluorophosphate (PyAOP) .

The molar dosage of the coupling agent is 1.2-6 times the total molar amount of the loaded resin, preferably 2.0-4.0 times.

The activator is selected from the group consisting of 1-hydroxybenzotriazole (HOBT) and 1-hydroxy-7-azabenzotriazole (HOAT), preferably 1-hydroxy-7-azabenzotriazole (HOAT).

The molar dosage of the activator is 1.2-6 times the total molar amount of the loaded resin, preferably 2.0-4.0 times.

The cyclization reaction time is 1-20 h, preferably 1-3 h.

The catalyst is an organic base selected from the group consisting of N, N-diisopropylethylamine (DIEA), triethylamine (TEA), and N-methylmorpholine (NMM), preferably N-methylmorpholine (NMM).

The solvent is an aprotic polar solvent selected from the group consisting of N, N-dimethylformamide (DMF), N-methylpyrrolidone (NMP), or a mixture thereof, preferably DMF.

### Regarding step (4):

The acidolysis solution used in the acidolysis step of step (4) contains hydrofluoric acid (HF) or trifluoroacetic acid (TFA), preferably acidolysis solution is TFA.

The dosage of the acidolysis solution is 5-30 mL per gram of the loaded resin, preferably 10 mL per gram of the loaded resin. The acidolysis solution is composed of TFA and a side-chain protecting group remover.

The concentration of TFA is 80-95%, and the remainder is the side-chain protecting group remover.

The side-chain protecting group remover is selected from the group consisting of thioanisole, triisopropylsilane, phenol, water, and 1, 2-ethanedithiol, preferably water.

The acidolysis time is 60-300 min, preferably 100-120 min.

The acidolysis solution containing the polypeptide after acidolysis is added to cold diethyl ether (volume ratio of acidolysis solution to cold diethyl ether = 1:20) to precipitate the peptide, followed by centrifugation and drying to obtain the crude peptide.

### Regarding step (5):

In the step (5), the crude peptide is dissolved in water and filtered through a filter membrane with a pore size of 0.22 µm, then purified by preparative high-performance liquid chromatography. Gradient elution is performed using mobile phase A (0.1% TFA/water solution) and mobile phase B (0.1% TFA/acetonitrile solution), with the detection wavelength at 214 nm. The product is dried by lyophilization. The final purity achieved by this method is greater than 95%, preferably greater than 99%.

Specifically, the preparation method includes the following steps:
In the step (1), for example, the preparation of Fmoc-AA-OP-resin is carried out as follows: adding a halogenated resin into a solid-phase polypeptide synthesis tube, adding DCM for swelling; after swelling, washing three times with DMF and three times with DCM; dissolving the protected starting amino acid Fmoc-AA-OP and DIEA in DCM, adding the solution into the peptide synthesis tube, reacting at room temperature for 2 h; vacuumizing to remove the reaction solution; washing the resin with DMF three times and DCM three times to obtain Fmoc-AA-OP-resin.

In the step (2), the coupling synthesis method includes the steps of: treating the Fmoc-AA-OP-resin obtained in the step (1) with 20% piperidine/DMF twice for 10 min each time to remove the α-amino Fmoc-protecting group; washing three times with DMF and three times with DCM; dissolving an amino acid or a side-chain carboxylic acid (R₀-COOH), HCTU and HOBT in DMF, adding the solution into the peptide synthesis tube, reacting at room temperature for 120 min; vacuumizing to remove the reaction solution; washing three times with DMF and three times with DCM; sequentially coupling amino acids from position 8 to position 1, and then coupling the side-chain carboxylic acid to the protected polypeptide-resin; removing the ivDde or Dde protecting group on the side-chain amino group of the amino acid at position 4 with 2% hydrazine hydrate/DMF solution for 30 min; washing three times with DMF and three times with DCM; coupling the carboxyl group of the amino acid at position 10 to the side-chain amino group of the amino acid at position 4 to obtain a fully protected linear polypeptide-resin; or sequentially coupling amino acids from position 7 to position 1, and then coupling the side-chain carboxylic acid to the protected polypeptide-resin; removing the ivDde or Dde protecting group on the side-chain amino group of the amino acid at position 4 with 2% hydrazine hydrate/DMF solution; washing three times with DMF and three times with DCM; coupling the carboxyl group of the amino acid at position 10 to the side-chain amino group of the amino acid at position 4, and then coupling the carboxyl group of the amino acid at position 9 to the α-amino group of the amino acid at position 10, to obtain a fully protected linear polypeptide-resin.

In the step (3), for example, the selective removal of protecting groups and solid-phase cyclization are specifically carried out as follows: treating the fully protected linear polypeptide-resin obtained in the step (2) with 20% piperidine/DMF (twice, 10 min each time) to remove the α-amino Fmoc-protecting group; washing three times with DMF and three times with DCM to form a free amino group; removing the carboxyl allyl-protecting group with tetrakis(triphenylphosphine)palladium/phenylsilane in a mixed solution of DCM and DMF( volume ratio of DCM and DMF is 5:5) to form a free carboxyl group (120min); dissolving PyAOP and HOAT in DMF, adding NMM, adding the mixture into a peptide synthesis tube, reacting at room temperature for 3 h; vacuumizing to remove the reaction solution; washing the mixture three times with DMF and three times with DCM to obtain a fully protected cyclic polypeptide-resin.

In the step (4), for example, the specific method for obtaining a cyclic alkaline lipopeptide crude product by acid hydrolysis is as follows: adding an acidolysis solution ( TFA/H₂O volume ratio of 95:5) into the peptide synthesis tube, reacting at room temperature for 120 min; adding the TFA cleavage solution into cold diethyl ether (volume ratio of TFA cleavage solution and cold diethyl ether ia 1:20 to precipitate the peptide; centrifuging and drying to obtain a crude peptide.

In the step (5), for example, the specific method for purification, salt conversion and lyophilization of the crude product is as follows: dissolving the crude product in water, filtering through a filter membrane with a pore size of 0.22 µm for later use; performing preparative high-performance liquid chromatography using a reversed-phase C18 packing material with a particle size of 10 µm, with mobile phase A of 0.1% TFA/water solution and mobile phase B of 0.1 % TFA/acetonitrile solution; performing gradient elution with a gradient system and cyclic sample injection for purification using a 22 mm×250 mm chromatographic column with a flow rate of 10 mL/min and a detection wavelength of 215 nm; loading the crude product solution onto the chromatographic column, starting mobile phase elution, collecting fractions corresponding to the main chromatographic peak; evaporating acetonitrile from the fractions to obtain an aqueous solution of the polymyxin compound; lyophilizing to obtain the product.

The final purity achievable by this method is greater than 95.0%, preferably greater than 99.0%. The yield rate is greater than 30.0%, calculated on the basis of the resin.

The present disclosure prepares novel compounds with modified alkaline amino acids or hydrophobicity in cyclic lipopeptide molecules. The compounds of the present disclosure can be readily prepared by the aforementioned chemical synthesis method, while clinically used polymyxin B and colistin (polymyxin E) are multi-component mixtures obtained by bacterial fermentation.

**Table 1 Structures of Some Compounds**

| S/N | Structural Formula | Molecular Formula | Precise Molecular Weight | [M+H]⁺ |
|---|---|---|---|---|
| 1 | (S)-6-methyloctanoyl-Dab-Thr-D-Dap-ring(4-10)[ Dab-Dab-D-Phe-Thr-Dab-Dab-Thr] | C₅₃H₉₂N₁₆ O₁₄ | 1176.6979 | 1177.7 |
| 2 | 6-methylheptanoyl-Dab-Thr-D-Dap-ring(4-10)[Da b-Dab-D-Phe-Thr-Dab-Dab-Thr] | C₅₂H₉₀N₁₆ O₁₄ | 1162.6822 | 1163.7 |
| 3 | Octanoyl-Dab-Thr-D-Dap-ring(4-10)[Dab-Dab-D-Phe-Thr-Dab-Dab-Thr] | C₅₂H₉₀N₁₆ O₁₄ | 1162.6822 | 1163.7 |
| 4 | Heptanoyl-Dab-Thr-D-Dap-ring(4-10)[Dab-Dab-D-Phe-Thr-Dab-Dab-Thr] | C₅₁H₈₈N₁₆ O₁₄ | 1148.6666 | 1149.7 |
| 5 | Nonanoyl-Dab-Thr-D-Dap-ring(4-10)[Dab-Dab-D -Phe-Thr-Dab-Dab-Thr] | C₅₃H₉₂N₁₆ O₁₄ | 1176.6979 | 1177.7 |
| 6 | (S)-6-methyloctanoyl-Dab-Thr-Dap-ring(4-10)[Da b-Dab-D-Phe-Thr-Dab-Dab-Thr] | C₅₃H₉₂N₁₆ O₁₄ | 1176.6979 | 1177.7 |
| 7 | 6-methylheptanoyl-Dab-Thr-Dap-ring(4-10)[Dab-Dab-D-Phe-Thr-Dab-Dab-Thr] | C₅₂H₉₀N₁₆ O₁₄ | 1162.6822 | 1163.7 |
| 8 | Octanoyl-Dab-Thr-Dap-ring(4-10)[Dab-Dab-D-Ph e-Thr-Dab-Dab-Thr] | C₅₂H₉₀N₁₆ O₁₄ | 1162.6822 | 1163.7 |
| 9 | Heptanoyl-Dab-Thr-Dap-ring(4-10)[Dab-Dab-D-P he-Thr-Dab-Dab-Thr] | C₅₁H₈₈N₁₆ O₁₄ | 1148.6666 | 1149.7 |
| 10 | Nonanoyl-Dab-Thr-Dap-ring(4-10)[Dab-Dab-D-P he-Thr-Dab-Dab-Thr] | C₅₃H₉₂N₁₆ O₁₄ | 1176.6979 | 1177.7 |
| 11 | (S)-6-methyloctanoyl-Dab-Thr-D-Dab-ring(4-10)[ Dab-Dab-D-Phe-Thr-Dab-Dab-Thr] | C₅₄H₉₄N₁₆ O₁₄ | 1190.7135 | 1191.7 |
| 12 | 6-methylheptanoyl-Dab-Thr-D-Dab-ring(4-10)[Da b-Dab-D-Phe-Thr-Dab-Dab-Thr] | C₅₃H₉₂N₁₆ O₁₄ | 1176.6979 | 1177.7 |
| 13 | Octanoyl-Dab-Thr-D-Dab-ring(4-10)[Dab-Dab-D-Phe-Thr-Dab-Dab-Thr] | C₅₃H₉₂N₁₆ O₁₄ | 1176.6979 | 1177.7 |
| 14 | Heptanoyl-Dab-Thr-D-Dab-ring(4-10)[Dab-Dab-D-Phe-Thr-Dab-Dab-Thr] | C₅₂H₉₀N₁₆ O₁₄ | 1162.6822 | 1163.7 |
| 15 | Nonanoyl-Dab-Thr-D-Dab-ring(4-10)[Dab-Dab-D -Phe-Thr-Dab-Dab-Thr] | C₅₄H₉₄N₁₆ O₁₄ | 1190.7135 | 1191.7 |
| 16 | (S)-6-methyloctanoyl-Dab-Thr-Dab-ring(4-10)[Da b-Dab-D-Phe-Thr-Dab-Dab-Thr] | C₅₄H₉₄N₁₆ O₁₄ | 1190.7135 | 1191.7 |
| 17 | 6-methylheptanoyl-Dab-Thr-Dab-ring(4-10)[Dab-Dab-D-Phe-Thr-Dab-Dab-Thr] | C₅₃H₉₂N₁₆ O₁₄ | 1176.6979 | 1177.7 |
| 18 | Octanoyl-Dab-Thr-Dab-ring(4-10)[Dab-Dab-D-Ph e-Thr-Dab-Dab-Thr] | C₅₃H₉₂N₁₆ O₁₄ | 1176.6979 | 1177.7 |
| 19 | Heptanoyl-Dab-Thr-Dab-ring(4-10)[Dab-Dab-D-P he-Thr-Dab-Dab-Thr] | C₅₂H₉₀N₁₆ O₁₄ | 1162.6822 | 1163.7 |
| 20 | Nonanoyl-Dab-Thr-Dab-ring(4-10)[Dab-Dab-D-P he-Thr-Dab-Dab-Thr] | C₅₄H₉₄N₁₆ O₁₄ | 1190.7135 | 1191.7 |
| 21 | (S)-6-methyloctanoyl-Dab-Thr-D-Orn-ring(4-10)[ Dab-Dab-D-Phe-Thr-Dab-Dab-Thr] | C₅₅H₉₆N₁₆ O₁₄ | 1204.7292 | 1205.7 |
| 22 | 6-methylheptanoyl-Dab-Thr-D-Orn-ring(4-10)[Da b-Dab-D-Phe-Thr-Dab-Dab-Thr] | C₅₄H₉₄N₁₆ O₁₄ | 1190.7135 | 1191.7 |
| 23 | Octanoyl-Dab-Thr-D-Orn-ring(4-10)[Dab-Dab-D-Phe-Thr-Dab-Dab-Thr] | C₅₄H₉₄N₁₆ O₁₄ | 1190.7135 | 1191.7 |
| 24 | Heptanoyl-Dab-Thr-D-Orn-ring(4-10)[Dab-Dab-D -Phe-Thr-Dab-Dab-Thr] | C₅₃H₉₂N₁₆ O₁₄ | 1176.6979 | 1177.7 |
| 25 | Nonanoyl-Dab-Thr-D-Orn-ring(4-10)[Dab-Dab-D -Phe-Thr-Dab-Dab-Thr] | C₅₅H₉₆N₁₆ O₁₄ | 1204.7292 | 1205.7 |
| 26 | (S)-6-methyloctanoyl-Dab-Thr-Orn-ring(4-10)[Da b-Dab-D-Phe-Thr-Dab-Dab-Thr] | C₅₅H₉₆N₁₆ O₁₄ | 1204.7292 | 1205.7 |
| 27 | 6-methylheptanoyl-Dab-Thr-Orn-ring(4-10)[Dab-Dab-D-Phe-Thr-Dab-Dab-Thr] | C₅₄H₉₄N₁₆ O₁₄ | 1190.7135 | 1191.7 |
| 28 | Octanoyl-Dab-Thr-Orn-ring(4-10)[Dab-Dab-D-Ph e-Thr-Dab-Dab-Thr] | C₅₄H₉₄N₁₆ O₁₄ | 1190.7135 | 1191.7 |
| 29 | Heptanoyl-Dab-Thr-Orn-ring(4-10)[Dab-Dab-D-P he-Thr-Dab-Dab-Thr] | C₅₃H₉₂N₁₆ O₁₄ | 1176.6979 | 1177.7 |
| 30 | Nonanoyl-Dab-Thr-Orn-ring(4-10)[Dab-Dab-D-P he-Thr-Dab-Dab-Thr] | C₅₅H₉₆N₁₆ O₁₄ | 1204.7292 | 1205.7 |
| 31 | (S)-6-methyloctanoyl-Dab-Thr-D-Dap-ring(4-10)[ Dab-Dab-D-Phe-Leu-Dab-Dab-Thr] | C₅₅H₉₆N₁₆ O₁₃ | 1188.7343 | 1189.7 |
| 32 | 6-methylheptanoyl-Dab-Thr-D-Dap-ring(4-10)[Da b-Dab-D-Phe-Leu-Dab-Dab-Thr] | C₅₄H₉₄N₁₆ O₁₃ | 1174.7186 | 1175.7 |
| 33 | Octanoyl-Dab-Thr-D-Dap-ring(4-10)[Dab-Dab-D-Phe-Leu-Dab-Dab-Thr] | C₅₄H₉₄N₁₆ O₁₃ | 1174.7186 | 1175.7 |
| 34 | Heptanoyl-Dab-Thr-D-Dap-ring(4-10)[Dab-Dab-D-Phe-Leu-Dab-Dab-Thr] | C₅₃H₉₂N₁₆ O₁₃ | 1160.7030 | 1161.7 |
| 35 | Nonanoyl-Dab-Thr-D-Dap-ring(4-10)[Dab-Dab-D -Phe-Leu-Dab-Dab-Thr] | C₅₅H₉₆N₁₆ O₁₃ | 1188.7343 | 1189.7 |
| 36 | (S)-6-methyloctanoyl-Dab-Thr-Dap-ring(4-10)[Da b-Dab-D-Phe-Leu-Dab-Dab-Thr] | C₅₅H₉₆N₁₆ O₁₃ | 1188.7343 | 1189.7 |
| 37 | 6-methylheptanoyl-Dab-Thr-Dap-ring(4-10)[Dab-Dab-D-Phe-Leu-Dab-Dab-Thr] | C₅₄H₉₄N₁₆ O₁₃ | 1174.7186 | 1175.7 |
| 38 | Octanoyl-Dab-Thr-Dap-ring(4-10)[Dab-Dab-D-Ph e-Leu-Dab-Dab-Thr] | C₅₄H₉₄N₁₆ O₁₃ | 1174.7186 | 1175.7 |
| 39 | Heptanoyl-Dab-Thr-Dap-ring(4-10)[Dab-Dab-D-P he-Leu-Dab-Dab-Thr] | C₅₃H₉₂N₁₆ O₁₃ | 1160.7030 | 1161.7 |
| 40 | Nonanoyl-Dab-Thr-Dap-ring(4-10)[Dab-Dab-D-P he-Leu-Dab-Dab-Thr] | C₅₅H₉₆N₁₆ O₁₃ | 1188.7343 | 1189.7 |
| 41 | (S)-6-methyloctanoyl-Dab-Thr-D-Orn-ring(4-10)[ Dab-Dab-D-Phe-Leu-Dab-Dab-Thr] | C₅₇H₁₀₀N_{1 6}O₁₃ | 1216.7656 | 1217.7 |
| 42 | 6-methylheptanoyl-Dab-Thr-D-Orn-ring(4-10)[Da b-Dab-D-Phe-Leu-Dab-Dab-Thr] | C₅₆H₉₈N₁₆ O₁₃ | 1202.7499 | 1203.7 |
| 43 | Octanoyl-Dab-Thr-D-Orn-ring(4-10)[Dab-Dab-D-Phe-Leu-Dab-Dab-Thr] | C₅₆H₉₈N₁₆ O₁₃ | 1202.7499 | 1203.7 |
| 44 | Heptanoyl-Dab-Thr-D-Orn-ring(4-10)[Dab-Dab-D -Phe-Leu-Dab-Dab-Thr] | C₅₅H₉₆N₁₆ O₁₃ | 1188.7343 | 1189.7 |
| 45 | Nonanoyl-Dab-Thr-D-Orn-ring(4-10)[Dab-Dab-D -Phe-Leu-Dab-Dab-Thr] | C₅₇H₁₀₀N_{1 6}O₁₃ | 1216.7656 | 1217.7 |
| 46 | (S)-6-methyloctanoyl-Dab-Thr-Orn-ring(4-10)[Da b-Dab-D-Phe-Leu-Dab-Dab-Thr] | C₅₇H₁₀₀N_{1 6}O₁₃ | 1216.7656 | 1217.7 |
| 47 | 6-methylheptanoyl-Dab-Thr-Orn-ring(4-10)[Dab-Dab-D-Phe-Leu-Dab-Dab-Thr] | C₅₆H₉₈N₁₆ O₁₃ | 1202.7499 | 1203.7 |
| 48 | Octanoyl-Dab-Thr-Orn-ring(4-10)[Dab-Dab-D-Ph e-Leu-Dab-Dab-Thr] | C₅₆H₉₈N₁₆ O₁₃ | 1202.7499 | 1203.7 |
| 49 | Heptanoyl-Dab-Thr-Orn-ring(4-10)[Dab-Dab-D-P he-Leu-Dab-Dab-Thr] | C₅₅H₉₆N₁₆ O₁₃ | 1188.7343 | 1189.7 |
| 50 | Nonanoyl-Dab-Thr-Orn-ring(4-10)[Dab-Dab-D-P he-Leu-Dab-Dab-Thr] | C₅₇H₁₀₀N_{1 6}O₁₃ | 1216.7656 | 1217.7 |
| 51 | (S)-6-methyloctanoyl-Dap-Thr-D-Ser-ring(4-10)[ Dab-Dab-D-Phe-Thr-Dab-Dab-Thr] | C₅₂H₈₉N₁₅ O₁₅ | 1163.6663 | 1164.7 |
| 52 | 6-methylheptanoyl-Dap-Thr-D-Ser-ring(4-10) [Da b-Dab-D-Phe-Thr-Dab-Dab-Thr] | C₅₁H₈₇N₁₅ O₁₅ | 1149.6506 | 1150.6 |
| 53 | Octanoyl-Dap-Thr-D-Ser-ring(4-10)[Dab-Dab-D-Phe-Thr-Dab-Dab-Thr] | C₅₁H₈₇N₁₅ O₁₅ | 1149.6506 | 1150.6 |
| 54 | Heptanoyl-Dap-Thr-D-Ser-ring(4-10)[Dab-Dab-D -Phe-Thr-Dab-Dab-Thr] | C₅₀H₈₅N₁₅ O₁₅ | 1135.6350 | 1136.6 |
| 55 | Nonanoyl-Dap-Thr-D-Ser-ring(4-10)[Dab-Dab-D-Phe-Thr-Dab-Dab-Thr] | C₅₂H₈₉N₁₅ O₁₅ | 1163.6663 | 1164.7 |
| 56 | (S)-6-methyloctanoyl-Orn-Thr-D-Ser-ring(4-10)[ Dab-Dab-D-Phe-Thr-Dab-Dab-Thr] | C₅₄H₉₃N₁₅ O₁₅ | 1191.6976 | 1192.7 |
| 57 | 6-methylheptanoyl-Orn-Thr-D-Ser-ring(4-10)[Dab -Dab-D-Phe-Thr-Dab-Dab-Thr] | C₅₃H₉₁N₁₅ O₁₅ | 1177.6819 | 1178.7 |
| 58 | Octanoyl-Orn-Thr-D-Ser-ring(4-10)[Dab-Dab-D-P he-Thr-Dab-Dab-Thr] | C₅₃H₉₁N₁₅ O₁₅ | 1177.6819 | 1178.7 |
| 59 | Heptanoyl-Orn-Thr-D-Ser-ring(4-10)[Dab-Dab-D-Phe-Thr-Dab-Dab-Thr] | C₅₂H₈₉N₁₅ O₁₅ | 1163.6663 | 1164.7 |
| 60 | Nonanoyl-Orn-Thr-D-Ser-ring(4-10)[Dab-Dab-D-Phe-Thr-Dab-Dab-Thr] | C₅₄H₉₃N₁₅ O₁₅ | 1191.6976 | 1192.7 |
| 61 | (S)-6-methyloctanoyl-Dab-Thr-D-Ser-ring(4-10)[ Dab-Dap-D-Phe-Thr-Dab-Dab-Thr] | C₅₂H₈₉N₁₅ O₁₅ | 1163.6663 | 1164.7 |
| 62 | 6-methylheptanoyl-Dab-Thr-D-Ser-ring(4-10) [Da b-Dap-D-Phe-Thr-Dab-Dab-Thr] | C₅₁H₈₇N₁₅ O₁₅ | 1149.6506 | 1150.6 |
| 63 | Octanoyl-Dab-Thr-D-Ser-ring(4-10)[Dab-Dap-D-Phe-Thr-Dab-Dab-Thr] | C₅₁H₈₇N₁₅ O₁₅ | 1149.6506 | 1150.6 |
| 64 | Heptanoyl-Dab-Thr-D-Ser-ring(4-10)[Dab-Dap-D -Phe-Thr-Dab-Dab-Thr] | C₅₀H₈₅N₁₅ O₁₅ | 1135.6350 | 1136.6 |
| 65 | Nonanoyl-Dab-Thr-D-Ser-ring(4-10)[Dab-Dap-D-Phe-Thr-Dab-Dab-Thr] | C₅₂H₈₉N₁₅ O₁₅ | 1163.6663 | 1164.7 |
| 66 | (S)-6-methyloctanoyl-Dab-Thr-D-Ser-ring(4-10)[ Dab-Orn-D-Phe-Thr-Dab-Dab-Thr] | C₅₄H₉₃N₁₅ O₁₅ | 1191.6976 | 1192.7 |
| 67 | 6-methylheptanoyl-Dab-Thr-D-Ser-ring(4-10) [Da b-Orn-D-Phe-Thr-Dab-Dab-Thr] | C₅₃H₉₁N₁₅ O₁₅ | 1177.6819 | 1178.7 |
| 68 | Octanoyl-Dab-Thr-D-Ser-ring(4-10)[Dab-Orn-D-P he-Thr-Dab-Dab-Thr] | C₅₃H₉₁N₁₅ O₁₅ | 1177.6819 | 1178.7 |
| 69 | Heptanoyl-Dab-Thr-D-Ser-ring(4-10)[Dab-Orn-D-Phe-Thr-Dab-Dab-Thr] | C₅₂H₈₉N₁₅ O₁₅ | 1163.6663 | 1164.7 |
| 70 | Nonanoyl-Dab-Thr-D-Ser-ring(4-10)[Dab-Orn-D-Phe-Thr-Dab-Dab-Thr] | C₅₄H₉₃N₁₅ O₁₅ | 1191.6976 | 1192.7 |
| 71 | (S)-6-methyloctanoyl-Dab-Thr-D-Ser-ring(4-10)[ Dab-Dab-D-Phe-Thr-Dap-Dab-Thr] | C₅₂H₈₉N₁₅ O₁₅ | 1163.6663 | 1164.7 |
| 72 | 6-methylheptanoyl-Dab-Thr-D-Ser-ring(4-10) [Da b-Dab-D-Phe-Thr-Dap-Dab-Thr] | C₅₁H₈₇N₁₅ O₁₅ | 1149.6506 | 1150.6 |
| 73 | Octanoyl-Dab-Thr-D-Ser-ring(4-10)[Dab-Dab-D-Phe-Thr-Dap-Dab-Thr] | C₅₁H₈₇N₁₅ O₁₅ | 1149.6506 | 1150.6 |
| 74 | Heptanoyl-Dab-Thr-D-Ser-ring(4-10)[Dab-Dab-D -Phe-Thr-Dap-Dab-Thr] | C₅₀H₈₅N₁₅ O₁₅ | 1135.6350 | 1136.6 |
| 75 | Nonanoyl-Dab-Thr-D-Ser-ring(4-10)[Dab-Dab-D-Phe-Thr-Dap-Dab-Thr] | C₅₂H₈₉N₁₅ O₁₅ | 1163.6663 | 1164.7 |
| 76 | (S)-6-methyloctanoyl-Dab-Thr-D-Ser-ring(4-10)[ Dab-Dab-D-Phe-Thr-Orn-Dab-Thr] | C₅₄H₉₃N₁₅ O₁₅ | 1191.6976 | 1192.7 |
| 77 | 6-methylheptanoyl-Dab-Thr-D-Ser-ring(4-10) [Da b-Dab-D-Phe-Thr-Orn-Dab-Thr] | C₅₃H₉₁N₁₅ O₁₅ | 1177.6819 | 1178.7 |
| 78 | Octanoyl-Dab-Thr-D-Ser-ring(4-10)[Dab-Dab-D-Phe-Thr-Orn-Dab-Thr] | C₅₃H₉₁N₁₅ O₁₅ | 1177.6819 | 1178.7 |
| 79 | Heptanoyl-Dab-Thr-D-Ser-ring(4-10)[Dab-Dab-D -Phe-Thr-Orn-Dab-Thr] | C₅₂H₈₉N₁₅ O₁₅ | 1163.6663 | 1164.7 |
| 80 | Nonanoyl-Dab-Thr-D-Ser-ring(4-10)[Dab-Dab-D-Phe-Thr-Orn-Dab-Thr] | C₅₄H₉₃N₁₅ O₁₅ | 1191.6976 | 1192.7 |
| 81 | (S)-6-methyloctanoyl-Dab-Thr-D-Ser-ring(4-10)[ Dab-Dab-D-Phe-Thr-Dab-Dap-Thr] | C₅₂H₈₉N₁₅ O₁₅ | 1163.6663 | 1164.7 |
| 82 | 6-methylheptanoyl-Dab-Thr-D-Ser-ring(4-10) [Da b-Dab-D-Phe-Thr-Dab-Dap-Thr] | C₅₁H₈₇N₁₅ O₁₅ | 1149.6506 | 1150.6 |
| 83 | Octanoyl-Dab-Thr-D-Ser-ring(4-10)[Dab-Dab-D-Phe-Thr-Dab-Dap-Thr] | C₅₁H₈₇N₁₅ O₁₅ | 1149.6506 | 1150.6 |
| 84 | Heptanoyl-Dab-Thr-D-Ser-ring(4-10)[Dab-Dab-D -Phe-Thr-Dab-Dap-Thr] | C₅₀H₈₅N₁₅ O₁₅ | 1135.6350 | 1136.6 |
| 85 | Nonanoyl-Dab-Thr-D-Ser-ring(4-10)[Dab-Dab-D-Phe-Thr-Dab-Dap-Thr] | C₅₂H₈₉N₁₅ O₁₅ | 1163.6663 | 1164.7 |
| 86 | (S)-6-methyloctanoyl-Dab-Thr-D-Ser-ring(4-10)[ Dab-Dab-D-Phe-Thr-Dab-Orn-Thr] | C₅₄H₉₃N₁₅ O₁₅ | 1191.6976 | 1192.7 |
| 87 | 6-methylheptanoyl-Dab-Thr-D-Ser-ring(4-10) [Da b-Dab-D-Phe- Thr-Dab-Orn- Thr] | C₅₃H₉₁N₁₅ O₁₅ | 1177.6819 | 1178.7 |
| 88 | Octanoyl-Dab-Thr-D-Ser-ring(4-10)[Dab-Dab-D-Phe-Thr-Dab-Orn-Thr] | C₅₃H₉₁N₁₅ O₁₅ | 1177.6819 | 1178.7 |
| 89 | Heptanoyl-Dab-Thr-D-Ser-ring(4-10)[Dab-Dab-D -Phe-Thr-Dab-Orn-Thr] | C₅₂H₈₉N₁₅ O₁₅ | 1163.6663 | 1164.7 |
| 90 | Nonanoyl-Dab-Thr-D-Ser-ring(4-10)[Dab-Dab-D-Phe-Thr-Dab-Orn-Thr] | C₅₄H₉₃N₁₅ O₁₅ | 1191.6976 | 1192.7 |
| 91 | (S)-6-methyloctanoyl-Dap-Thr-D-Dab-ring(4-10)[ Dab-Dab-D-Phe-Leu-Dab-Dab-Thr] | C₅₅H₉₆N₁₆ O₁₃ | 1188.7343 | 1189.7 |
| 92 | 6-methylheptanoyl-Dap-Thr-D-Dab-ring(4-10)[Da b-Dab-D-Phe-Leu-Dab-Dab-Thr] | C₅₄H₉₄N₁₆ O₁₃ | 1174.7186 | 1175.7 |
| 93 | Octanoyl-Dap-Thr-D-Dab-ring(4-10)[Dab-Dab-D-Phe-Leu-Dab-Dab-Thr] | C₅₄H₉₄N₁₆ O₁₃ | 1174.7186 | 1175.7 |
| 94 | Heptanoyl-Dap-Thr-D-Dab-ring(4-10)[Dab-Dab-D-Phe-Leu-Dab-Dab-Thr] | C₅₃H₉₂N₁₆ O₁₃ | 1160.7030 | 1161.7 |
| 95 | Nonanoyl-Dap-Thr-D-Dab-ring(4-10)[Dab-Dab-D -Phe-Leu-Dab-Dab-Thr] | C₅₅H₉₆N₁₆ O₁₃ | 1188.7343 | 1189.7 |
| 96 | (S)-6-methyloctanoyl-Dap-Thr-D-Dab-ring(4-10)[ Dab-Dab-D-Leu-Leu-Dab-Dab-Thr] | C₅₂H₉₈N₁₆ O₁₃ | 1154.7499 | 1155.7 |
| 97 | 6-methylheptanoyl-Dap-Thr-D-Dab-ring(4-10)[Da b-Dab-D-Leu-Leu-Dab-Dab-Thr] | C₅₁H₉₆N₁₆ O₁₃ | 1140.7343 | 1141.7 |
| 98 | Octanoyl-Dap-Thr-D-Dab-ring(4-10)[Dab-Dab-D-Leu-Leu-Dab-Dab-Thr] | C₅₁H₉₆N₁₆ O₁₃ | 1140.7343 | 1141.7 |
| 99 | Heptanoyl-Dap-Thr-D-Dab-ring(4-10)[Dab-Dab-D-Leu-Leu-Dab-Dab-Thr] | C₅₀H₉₄N₁₆ O₁₃ | 1126.7186 | 1127.7 |
| 100 | Nonanoyl-Dap-Thr-D-Dab-ring(4-10)[Dab-Dab-D -Leu-Leu-Dab-Dab-Thr] | C₅₂H₉₈N₁₆ O₁₃ | 1154.7499 | 1155.7 |
| 101 | (S)-6-methyloctanoyl-Dab-Thr-D-Dap-ring(4-10)[ Dab-Dab-Phe-Thr-Dab-Dab-Thr] | C₅₃H₉₂N₁₆ O₁₄ | 1176.6979 | 1177.7 |
| 102 | 6-methylheptanoyl-Dab-Thr-D-Dap-ring(4-10)[Da b-Dab-Phe-Thr-Dab-Dab-Thr] | C₅₂H₉₀N₁₆ O₁₄ | 1162.6822 | 1163.7 |
| 103 | 6-methylheptanoyl-Dab-Thr-D-Ser(CH₂SO₃H)-rin g(4-10)[Dab-Dab-D-Phe-Thr-Dab-Dab-Thr] | C₅₃H₉₁N₁₅ O₁₈S | 1257.6387 | 1258.6 |
| 104 | 6-methylheptanoyl-Dab-Thr(CH₂SO₃H)-D-Ser-rin g(4-10)[Dab-Dab-D-Phe-Thr-Dab-Dab-Thr] | C₅₃H₉₁N₁₅ O₁₈S | 1257.6387 | 1258.6 |
| 105 | 6-methylheptanoyl-Dab-Thr-D-Ser-ring(4-10) [Da b-Dab-D-Phe-Thr(CH₂SO₃H)-Dab-Dab-Thr] | C₅₃H₉₁N₁₅ O₁₈S | 1257.6387 | 1258.6 |
| 106 | 6-methylheptanoyl-Dab-Thr-D-Ser-ring(4-10) [Da b-Dab-D-Phe-Thr-Dab-Dab-Thr(CH₂SO₃H)] | C₅₃H₉₁N₁₅ O₁₈S | 1257.6387 | 1258.6 |
| 107 | 6-methylheptanoyl-Dab-Thr-D-Ser-ring(4-10) [Da b-Dab-D-Phe(4-CH₂SO₃H)-Thr-Dab-Dab-Thr] | C₅₃H₉₁N₁₅ O₁₈S | 1257.6387 | 1258.6 |
| 108 | 6-methylheptanoyl-Dab(CH₂SO₃H)-Thr-D-Ser-rin g(4-10)[Dab-Dab-D-Phe-Thr-Dab-Dab-Thr] | C₅₃H₉₁N₁₅ O₁₈S | 1257.6387 | 1258.6 |
| 109 | 6-methylheptanoyl-Dab-Thr-D-Ser-ring(4-10) [Da b-Dab(CH₂SO₃H)-D-Phe-Thr-Dab-Dab-Thr] | C₅₃H₉₁N₁₅ O₁₈S | 1257.6387 | 1258.6 |
| 110 | 6-methylheptanoyl-Dab-Thr-D-Ser-ring(4-10) [Da b-Dab-D-Phe-Thr-Dab(CH₂SO₃H)-Dab-Thr] | C₅₃H₉₁N₁₅ O₁₈S | 1257.6387 | 1258.6 |
| 111 | 6-methylheptanoyl-Dab-Thr-D-Ser-ring(4-10) [Da b-Dab-D-Phe-Thr-Dab-Dab(CH₂SO₃H)-Thr] | C₅₃H₉₁N₁₅ O₁₈S | 1257.6387 | 1258.6 |
| 112 | 6-methylheptanoyl-Dab-Thr-Dap(CH₂SO₃H)-ring( 4-10)[Dab-Dab-D-Phe-Thr-Dab-Dab-Thr] | C₅₃H₉₂N₁₆ O₁₇S | 1256.6547 | 1257.7 |
| 113 | 6-methylheptanoyl-Dab-Thr(CH₂SO₃H)-Dap-ring( 4-10)[Dab-Dab-D-Phe-Thr-Dab-Dab-Thr] | C₅₃H₉₂N₁₆ O₁₇S | 1256.6547 | 1257.7 |
| 114 | 6-methylheptanoyl-Dab-Thr-Dap-ring(4-10)[Dab-Dab-D-Phe-Thr(CH₂SO₃H)-Dab-Dab-Thr] | C₅₃H₉₂N₁₆ O₁₇S | 1256.6547 | 1257.7 |
| 115 | 6-methylheptanoyl-Dab-Thr-Dap-ring(4-10)[Dab-Dab-D-Phe-Thr-Dab-Dab-Thr(CH₂SO₃H)] | C₅₃H₉₂N₁₆ O₁₇S | 1256.6547 | 1257.7 |
| 116 | 6-methylheptanoyl-Dab-Thr-Dap-ring(4-10)[Dab-Dab-D-Phe(4-CH₂SO₃H)-Thr-Dab-Dab-Thr] | C₅₃H₉₂N₁₆ O₁₇S | 1256.6547 | 1257.7 |
| 117 | 6-methylheptanoyl-Dab(CH₂SO₃H)-Thr-Dap-ring( 4-10)[Dab-Dab-D-Phe-Thr-Dab-Dab-Thr] | C₅₃H₉₂N₁₆ O₁₇S | 1256.6547 | 1257.7 |
| 118 | 6-methylheptanoyl-Dab-Thr-Dap-ring(4-10)[Dab-Dab(CH₂SO₃H)-D-Phe-Thr-Dab-Dab-Thr] | C₅₃H₉₂N₁₆ O₁₇S | 1256.6547 | 1257.7 |
| 119 | 6-methylheptanoyl-Dab-Thr-Dap-ring(4-10)[Dab- | C₅₃H₉₂N₁₆ | 1256.6547 | 1257.7 |
| | Dab-D-Phe-Thr-Dab(CH₂SO₃H)-Dab-Thr] | O₁₇S | | |
| 120 | 6-methylheptanoyl-Dab-Thr-Dap-ring(4-10)[Dab-Dab-D-Phe-Thr-Dab-Dab(CH₂SO₃H)-Thr] | C₅₃H₉₂N₁₆ O₁₇S | 1256.6547 | 1257.7 |

Another object of the present disclosure is to provide use of the cyclic alkaline lipopeptide compound having the structure represented by General Formula I or a pharmaceutically acceptable salt thereof in the preparation of an antibacterial medicament.

Wherein, the antibacterial property refers to that of antibacterial agents against Gram-negative bacteria. The Gram-negative bacteria include *Escherichia coli, Klebsiella pneumoniae, Pseudomonas aeruginosa, Acinetobacter baumannii, Salmonella, Moraxella, Helicobacter, Legionella, Haemophilus influenzae, Enterobacter cloacae, Enterobacter aerogenes, Serratia marcescens, Morganella morganii, Providentia rettgeri, Proteus vulgaris, Proteus mirabilis, Stenotrophomonas maltophilia, Citrobacter freundii,* etc.

Gram-negative bacteria include, for example, *Escherichia coli, Klebsiella pneumoniae, Pseudomonas aeruginosa* and *Acinetobacter baumannii.*

The present disclosure further provides a cyclic lipopeptide compound or a pharmaceutically acceptable salt thereof, which has higher antibacterial activity and lower nephrotoxicity than clinically used polymyxin B and colistin (polymyxin E). Renal cells are selected from the group consisting of human renal tubular epithelial cells (HK-2 cells), human embryonic kidney epithelial cells (HEK293 cells), African green monkey kidney cells (Vero cells), canine kidney cells (MDCK cells), for example, human renal tubular epithelial cells (HK-2 cells).

The present disclosure further provides an antibacterial pharmaceutical composition, which includes a therapeutically effective amount of a cyclic alkaline lipopeptide compound having the structure represented by General Formula I or a pharmaceutically acceptable salt thereof as an active ingredient. The compound itself or a mixture of the compound with pharmaceutical excipients, diluents and the like can be administered orally in the form of tablets, capsules, granules, powders or syrups, or administered parenterally in the form of injections, sprays, aerosols, ointments or eye drops.

The aforementioned formulations can be prepared by conventional pharmaceutical methods. Examples of pharmaceutical excipients and diluents include excipients (e.g., carbohydrate derivatives such as lactose, sucrose, glucose, mannitol and sorbitol; starch derivatives such as corn starch, potato starch, dextrin and carboxymethyl starch; cellulose derivatives such as crystalline cellulose, hydroxypropyl cellulose, hydroxymethyl cellulose, calcium hydroxymethyl cellulose, sodium hydroxymethyl cellulose; gum arabic; dextran; silicate derivatives such as magnesium aluminum metasilicate; phosphate derivatives such as calcium phosphate; carbonate derivatives such as calcium carbonate; sulfate derivatives such as calcium sulfate, etc.); binders (e.g., gelatin, polyvinylpyrrolidone and polyethylene glycol); disintegrants (e.g., cellulose derivatives such as sodium carboxymethyl cellulose, polyvinylpyrrolidone); lubricants (e.g., talc, calcium stearate, magnesium stearate, cetyl alcohol, boric acid, sodium benzoate, leucine); stabilizers (methylparaben, propylparaben, etc.); flavoring agents (e.g., commonly used sweeteners, sour agents and fragrances, etc.); diluents and injection solvents (e.g., water, ethanol, glycerol, etc.).

Synthetic route: Taking the synthetic route of compound 1 as an example, the other compounds are synthesized using a similar route under the identical reaction conditions as those for compound 1, with the only differences in the types and sequences of the starting amino acid Fmoc-AA-OP, and the amino acids and fatty acids added during the coupling reaction. 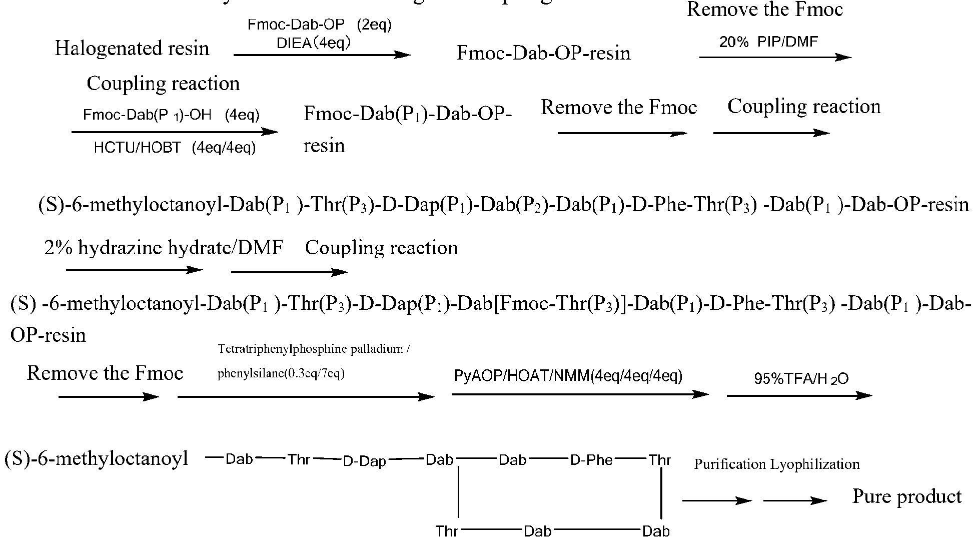
P: Allyl
P₁: tert-Butoxycarbonyl (Boc)
P₂: 1-(4,4-Dimethyl-2,6-dioxocyclohex-1-ylidene)ethyl (Dde), 1-(4,4-dimethyl-2,6-dioxocyclohex-1-ylidene)-3-methylbutyl (ivDde)
P₃: tert-Butyl (tBu)
Fmoc: 9-Fluorenylmethyloxycarbonyl

Compared with the existing compounds or synthetic methods, the method of the present disclosure is simple and efficient, environmentally friendly; the crude polypeptides produced have high purity, are easy to separate and purify, with a total yield of up to 40%.

### DETAILED DESCRIPTION

The present disclosure will be further described below in conjunction with specific embodiments, and the advantages and features of the present disclosure will become more apparent with the description. However, these embodiments are merely exemplary and do not constitute any limitation on the scope of the present disclosure. Those skilled in the art should understand that modifications or substitutions can be made to the details and forms of the technical solutions of the present disclosure without departing from the spirit and scope thereof, and all such modifications and substitutions shall fall within the protection scope of the present disclosure.

### Example 1: Preparation of (S)-6-methyloctanoyl-Dab-Thr-D-Dap-ring(4-10)[Dab-Dab-D-Phe-Thr-Dab-Dab-Thr] (compound 1)

The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Dap(Boc)-OH, Fmoc-Thr(tBu)-OH, Fmoc-Dab(Boc)-OH, (S)-6-methyloctanoic acid, and Fmoc-Thr(tBu)-OH.

2-Cl-Trt resin (0.5 mmol, substitution degree = 0.5 mmol/g) was added to a solid-phase polypeptide synthesis tube, and (S)-6-methyloctanoyl-Dab-Thr-D-Dap-ring(4-10)[Dab-Dab-D-Phe-Thr-Dab-Dab-Thr] was prepared according to the method illustrated in the synthetic route.

Crude peptide (530 mg) was obtained with a yield of 90.0%. The crude peptide was dissolved in water, filtered through a filter membrane at a pore size of 0.22 µm for later use. Purification was performed by preparative high-performance liquid chromatography (preparative HPLC) using a reversed-phase C18 packing material with a particle size of 10 µm, with mobile phase A of 0.1% TFA/water solution and mobile phase B of 0.1% TFA/acetonitrile solution; gradient elution with a gradient system and cyclic sample injection for purification were performed using a 22 mm×250 mm chromatographic column with a flow rate of 8 mL/min and a detection wavelength of 214 nm; the crude product solution was loaded onto the chromatographic column, and the mobile phase elution was started, fractions corresponding to the main chromatographic peak were collected and the acetonitrile was removed by evaporation to obtain an aqueous solution of compound 1, which was further freeze-dried to obtain the product.

The yield was 238 mg, corresponding to a yield rate of 40.8% based on 0.5 mmol of 2-Cl-Trt resin. Characterization of the purified peptide: purity (calculated by area integration of the HPLC chromatogram) > 99.0%; ESI: m/z = 1177.7([M+H]⁺).

### Example 2: Preparation of 6-methylheptanoyl-Dab-Thr-D-Dap-ring(4-10)[Dab-Dab-D-Phe-Thr-Dab-Dab-Thr] (compound 2)

Compound 2 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Dap(Boc)-OH, Fmoc-Thr(tBu)-OH, Fmoc-Dab(Boc)-OH, 6-methylheptanoic acid, Fmoc-Thr(tBu)-OH.

The yield was 240 mg, corresponding to a yield rate of 41.2% based on 0.5 mmol of 2-Cl-Trt resin. Characterization of the purified peptide: purity (calculated by area integration of the HPLC chromatogram)>99.0%; ESI: m/z = 1163.7([M+H]⁺).

### Example 3: Preparation of Octanoyl-Dab-Thr-D-Dap-ring(4-10)[Dab-Dab-D-Phe-Thr-Dab-Dab-Thr] (compound 3)

Compound 3 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Dap(Boc)-OH, Fmoc-Thr(tBu)-OH, Fmoc-Dab(Boc)-OH, octanoic acid, Fmoc-Thr(tBu)-OH.

The yield was 235 mg, corresponding to a yield rate of 40.4% based on 0.5 mmol of 2-Cl-Trt resin. Characterization of the purified peptide: purity (calculated by area integration of the HPLC chromatogram)>99.0%; ESI: m/z = 1163.7([M+H]⁺).

### Example 4: Preparation of Heptanoyl-Dab-Thr-D-Dap-ring(4-10)[Dab-Dab-D-Phe-Thr-Dab-Dab-Thr] (compound 4)

Compound 4 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Dap(Boc)-OH, Fmoc-Thr(tBu)-OH, Fmoc-Dab(Boc)-OH, heptanoic acid, Fmoc-Thr(tBu)-OH.

The yield was 230 mg, corresponding to a yield rate of 40.0% based on 0.5 mmol of 2-Cl-Trt resin. Characterization of the purified peptide: purity (calculated by area integration of the HPLC chromatogram)>99.0%; ESI: m/z = 1149.7([M+H]⁺).

### Example 5: Preparation of Nonanoyl-Dab-Thr-D-Dap-ring(4-10)[Dab-Dab-D-Phe-Thr-Dab-Dab-Thr] (compound 5)

Compound 5 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Dap(Boc)-OH, Fmoc-Thr(tBu)-OH, Fmoc-Dab(Boc)-OH, nonanoic acid, Fmoc-Thr(tBu)-OH.

The yield was 250 mg, corresponding to a yield rate of 42.4% based on 0.5 mmol of 2-Cl-Trt resin. Characterization of the purified peptide: purity (calculated by area integration of the HPLC chromatogram)>99.0%; ESI: m/z = 1177.7([M+H]⁺).

### Example 6: Preparation of (S)-6-methyloctanoyl-Dab-Thr-Dap-ring(4-10)[Dab-Dab-D-Phe-Thr-Dab-Dab-Thr] (compound 6)

Compound 6 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Dap(Boc)-OH, Fmoc-Thr(tBu)-OH, Fmoc-Dab(Boc)-OH, (S)-6-methyloctanoic acid, Fmoc-Thr(tBu)-OH.

The yield was 240 mg, corresponding to a yield rate of 40.8% based on 0.5 mmol of 2-Cl-Trt resin. Characterization of the purified peptide: purity (calculated by area integration of the HPLC chromatogram)>99.0%; ESI: m/z = 1177.7([M+H]⁺).

### Example 7: Preparation of 6-methylheptanoyl-Dab-Thr-Dap-ring(4-10)[Dab-Dab-D-Phe-Thr-Dab-Dab-Thr] (compound 7)

Compound 7 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Dap(Boc)-OH, Fmoc-Thr(tBu)-OH, Fmoc-Dab(Boc)-OH, 6-methylheptanoic acid, Fmoc-Thr(tBu)-OH.

The yield was 242 mg, corresponding to a yield rate of 41.6% based on 0.5 mmol of 2-Cl-Trt resin. Characterization of the purified peptide: purity (calculated by area integration of the HPLC chromatogram)>99.0%; ESI: m/z = 1163.7([M+H]⁺).

### Example 8: Preparation of Octanoyl-Dab-Thr-Dap-ring(4-10)[Dab-Dab-D-Phe-Thr-Dab-Dab-Thr] (compound 8)

Compound 8 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Dap(Boc)-OH, Fmoc-Thr(tBu)-OH, Fmoc-Dab(Boc)-OH, octanoic acid, Fmoc-Thr(tBu)-OH.

The yield was 238 mg, corresponding to a yield rate of 40.9% based on 0.5 mmol of 2-Cl-Trt resin. Characterization of the purified peptide: purity (calculated by area integration of the HPLC chromatogram)>99.0%; ESI: m/z = 1163.7([M+H]⁺).

### Example 9: Preparation of Heptanoyl-Dab-Thr-Dap-ring(4-10)[Dab-Dab-D-Phe-Thr-Dab-Dab-Thr] (compound 9)

Compound 9 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Dap(Boc)-OH, Fmoc-Thr(tBu)-OH, Fmoc-Dab(Boc)-OH, heptanoic acid, Fmoc-Thr(tBu)-OH.

The yield was 235 mg, corresponding to a yield rate of 40.9% based on 0.5 mmol of 2-Cl-Trt resin. Characterization of the purified peptide: purity (calculated by area integration of the HPLC chromatogram)>99.0%; ESI: m/z = 1149.7([M+H]⁺).

### Example 10: Preparation of Nonanoyl-Dab-Thr-Dap-ring(4-10)[Dab-Dab-D-Phe-Thr-Dab-Dab-Thr] (compound 10)

Compound 10 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Dap(Boc)-OH, Fmoc-Thr(tBu)-OH, Fmoc-Dab(Boc)-OH, nonanoic acid, Fmoc-Thr(tBu)-OH.

The yield was 251 mg, corresponding to a yield rate of 42.6% based on 0.5 mmol of 2-Cl-Trt resin. Characterization of the purified peptide: purity (calculated by area integration of the HPLC chromatogram)>99.0%; ESI: m/z = 1177.7([M+H]⁺).

### Example 11: Preparation of (S)-6-methyloctanoyl-Dab-Thr-D-Dab-ring(4-10)[Dab-Dab-D-Phe-Thr-Dab-Dab-Thr] (compound 11)

Compound 11 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Dab(Boc)-OH, Fmoc-Thr(tBu)-OH, Fmoc-Dab(Boc)-OH, (S)-6-methyloctanoic acid, Fmoc-Thr(tBu)-OH.

The yield was 248 mg, corresponding to a yield rate of 41.6% based on 0.5 mmol of 2-Cl-Trt resin. Characterization of the purified peptide: purity (calculated by area integration of the HPLC chromatogram)>99.0%; ESI: m/z = 1191.7([M+H]⁺).

### Example 12: Preparation of 6-methylheptanoyl-Dab-Thr-D-Dab-ring(4-10)[Dab-Dab-D-Phe-Thr-Dab-Dab-Thr] (compound 12)

Compound 12 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Dab(Boc)-OH, Fmoc-Thr(tBu)-OH, Fmoc-Dab(Boc)-OH, 6-methylheptanoic acid, Fmoc-Thr(tBu)-OH.

The yield was 240 mg, corresponding to a yield rate of 40.8% based on 0.5 mmol of 2-Cl-Trt resin. Characterization of the purified peptide: purity (calculated by area integration of the HPLC chromatogram)>99.0%; ESI: m/z = 1177.7([M+H]⁺).

### Example 13: Preparation of Octanoyl-Dab-Thr-D-Dab-ring(4-10)[Dab-Dab-D-Phe-Thr-Dab-Dab-Thr] (compound 13)

Compound 13 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Dab(Boc)-OH, Fmoc-Thr(tBu)-OH, Fmoc-Dab(Boc)-OH, octanoic acid, Fmoc-Thr(tBu)-OH.

The yield was 235 mg, corresponding to a yield rate of 39.9% based on 0.5 mmol of 2-Cl-Trt resin. Characterization of the purified peptide: purity (calculated by area integration of the HPLC chromatogram)>99.0%; ESI: m/z = 1177.7([M+H]⁺).

### Example 14: Preparation of Heptanoyl-Dab-Thr-D-Dab-ring(4-10)[Dab-Dab-D-Phe-Thr-Dab-Dab-Thr] (compound 14)

Compound 14 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Dab(Boc)-OH, Fmoc-Thr(tBu)-OH, Fmoc-Dab(Boc)-OH, heptanoic acid, Fmoc-Thr(tBu)-OH.

The yield was 235 mg, corresponding to a yield rate of 40.4% based on 0.5 mmol of 2-Cl-Trt resin. Characterization of the purified peptide: purity (calculated by area integration of the HPLC chromatogram)>99.0%; ESI: m/z = 1163.7([M+H]⁺).

### Example 15: Preparation of Nonanoyl-Dab-Thr-D-Dab-ring(4-10)[Dab-Dab-D-Phe-Thr-Dab-Dab-Thr] (compound 15)

Compound 15 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Dab(Boc)-OH, Fmoc-Thr(tBu)-OH, Fmoc-Dab(Boc)-OH, nonanoic acid, Fmoc-Thr(tBu)-OH.

The yield was 250 mg, corresponding to a yield rate of 42.0% based on 0.5 mmol of 2-Cl-Trt resin. Characterization of the purified peptide: purity (calculated by area integration of the HPLC chromatogram)>99.0%; ESI: m/z = 1191.7([M+H]⁺).

### Example 16: Preparation of (S)-6-methyloctanoyl-Dab-Thr-Dab-ring(4-10)[Dab-Dab-D-Phe-Thr-Dab-Dab-Thr] (compound 16)

Compound 16 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Dab(Boc)-OH, Fmoc-Thr(tBu)-OH, Fmoc-Dab(Boc)-OH, (S)-6-methyloctanoic acid, Fmoc-Thr(tBu)-OH.

The yield was 245 mg, corresponding to a yield rate of 41.1% based on 0.5 mmol of 2-Cl-Trt resin. Characterization of the purified peptide: purity (calculated by area integration of the HPLC chromatogram)>99.0%; ESI: m/z = 1191.7([M+H]⁺).

### Example 17: Preparation of 6-methylheptanoyl-Dab-Thr-Dab-ring(4-10)[Dab-Dab-D-Phe-Thr-Dab-Dab-Thr] (compound 17)

Compound 17 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Dab(Boc)-OH, Fmoc-Thr(tBu)-OH, Fmoc-Dab(Boc)-OH, 6-methylheptanoic acid, Fmoc-Thr(tBu)-OH.

The yield was 245 mg, corresponding to a yield rate of 41.6% based on 0.5 mmol of 2-Cl-Trt resin. Characterization of the purified peptide: purity (calculated by area integration of the HPLC chromatogram)>99.0%; ESI: m/z = 1177.7([M+H]⁺).

### Example 18: Preparation of Octanoyl-Dab-Thr-Dab-ring(4-10)[Dab-Dab-D-Phe-Thr-Dab-Dab-Thr] (compound 18)

Compound 18 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Dab(Boc)-OH, Fmoc-Thr(tBu)-OH, Fmoc-Dab(Boc)-OH, octanoic acid, Fmoc-Thr(tBu)-OH.

The yield was 241 mg, corresponding to a yield rate of 40.9% based on 0.5 mmol of 2-Cl-Trt resin. Characterization of the purified peptide: purity (calculated by area integration of the HPLC chromatogram)>99.0%; ESI: m/z = 1177.7([M+H]⁺).

### Example 19: Preparation of Heptanoyl-Dab-Thr-Dab-ring(4-10)[Dab-Dab-D-Phe-Thr-Dab-Dab-Thr] (compound 19)

Compound 19 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Dab(Boc)-OH, Fmoc-Thr(tBu)-OH, Fmoc-Dab(Boc)-OH, heptanoic acid, Fmoc-Thr(tBu)-OH.

The yield was 238 mg, corresponding to a yield rate of 40.9% based on 0.5 mmol of 2-Cl-Trt resin. Characterization of the purified peptide: purity (calculated by area integration of the HPLC chromatogram)>99.0%; ESI: m/z = 1163.7([M+H]⁺).

### Example 20: Preparation of Nonanoyl-Dab-Thr-Dab-ring(4-10)[Dab-Dab-D-Phe-Thr-Dab-Dab-Thr] (compound 20)

Compound 20 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Dab(Boc)-OH, Fmoc-Thr(tBu)-OH, Fmoc-Dab(Boc)-OH, nonanoic acid, Fmoc-Thr(tBu)-OH.

The yield was 254 mg, corresponding to a yield rate of 42.6% based on 0.5 mmol of 2-Cl-Trt resin. Characterization of the purified peptide: purity (calculated by area integration of the HPLC chromatogram)>99.0%; ESI: m/z = 1191.7([M+H]⁺).

### Example 21: Preparation of (S)-6-methyloctanoyl-Dab-Thr-D-Orn-ring(4-10)[Dab-Dab-D-Phe-Thr-Dab-Dab-Thr] (compound 21)

Compound 21 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Orn(Boc)-OH, Fmoc-Thr(tBu)-OH, Fmoc-Dab(Boc)-OH, (S)-6-methyloctanoic acid, Fmoc-Thr(tBu)-OH.

The yield was 251 mg, corresponding to a yield rate of 41.6% based on 0.5 mmol of 2-Cl-Trt resin. Characterization of the purified peptide: purity (calculated by area integration of the HPLC chromatogram)>99.0%; ESI: m/z = 1205.7([M+H]⁺).

### Example 22: Preparation of 6-methylheptanoyl-Dab-Thr-D-Orn-ring(4-10)[Dab-Dab-D-Phe-Thr-Dab-Dab-Thr] (compound 22)

Compound 22 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Orn(Boc)-OH, Fmoc-Thr(tBu)-OH, Fmoc-Dab(Boc)-OH, 6-methylheptanoic acid, Fmoc-Thr(tBu)-OH.

The yield was 243 mg, corresponding to a yield rate of 40.8% based on 0.5 mmol of 2-Cl-Trt resin. Characterization of the purified peptide: purity (calculated by area integration of the HPLC chromatogram)>99.0%; ESI: m/z = 1191.7([M+H]⁺).

### Example 23: Preparation of Octanoyl-Dab-Thr-D-Orn-ring(4-10)[Dab-Dab-D-Phe-Thr-Dab-Dab-Thr] (compound 23)

Compound 23 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Orn(Boc)-OH, Fmoc-Thr(tBu)-OH, Fmoc-Dab(Boc)-OH, octanoic acid, Fmoc-Thr(tBu)-OH.

The yield was 239 mg, corresponding to a yield rate of 40.1% based on 0.5 mmol of 2-Cl-Trt resin. Characterization of the purified peptide: purity (calculated by area integration of the HPLC chromatogram)>99.0%; ESI: m/z = 1191.7([M+H]⁺).

### Example 24: Preparation of Heptanoyl-Dab-Thr-D-Orn-ring(4-10)[Dab-Dab-D-Phe-Thr-Dab-Dab-Thr] (compound 24)

Compound 24 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Orn(Boc)-OH, Fmoc-Thr(tBu)-OH, Fmoc-Dab(Boc)-OH, heptanoic acid, Fmoc-Thr(tBu)-OH.

The yield was 238 mg, corresponding to a yield rate of 40.4% based on 0.5 mmol of 2-Cl-Trt resin. Characterization of the purified peptide: purity (calculated by area integration of the HPLC chromatogram)>99.0%; ESI: m/z = 1177.7([M+H]⁺).

### Example 25: Preparation of Nonanoyl-Dab-Thr-D-Orn-ring(4-10)[Dab-Dab-D-Phe-Thr-Dab-Dab-Thr] (compound 25)

Compound 25 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Orn(Boc)-OH, Fmoc-Thr(tBu)-OH, Fmoc-Dab(Boc)-OH, nonanoic acid, Fmoc-Thr(tBu)-OH.

The yield was 253 mg, corresponding to a yield rate of 42.0% based on 0.5 mmol of 2-Cl-Trt resin. Characterization of the purified peptide: purity (calculated by area integration of the HPLC chromatogram)>99.0%; ESI: m/z = 1205.7([M+H]⁺).

### Example 26: Preparation of (S)-6-methyloctanoyl-Dab-Thr-Orn-ring(4-10)[Dab-Dab-D-Phe-Thr-Dab-Dab-Thr] (compound 26)

Compound 26 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Orn(Boc)-OH, Fmoc-Thr(tBu)-OH, Fmoc-Dab(Boc)-OH, (S)-6-methyloctanoic acid, Fmoc-Thr(tBu)-OH.

The yield was 248 mg, corresponding to a yield rate of 41.1% based on 0.5 mmol of 2-Cl-Trt resin. Characterization of the purified peptide: purity (calculated by area integration of the HPLC chromatogram)>99.0%; ESI: m/z = 1205.7([M+H]⁺).

### Example 27: Preparation of 6-methylheptanoyl-Dab-Thr-Orn-ring(4-10)[Dab-Dab-D-Phe-Thr-Dab-Dab-Thr] (compound 27)

Compound 27 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Orn(Boc)-OH, Fmoc-Thr(tBu)-OH, Fmoc-Dab(Boc)-OH, 6-methylheptanoic acid, Fmoc-Thr(tBu)-OH.

The yield was 245 mg, corresponding to a yield rate of 41.1% based on 0.5 mmol of 2-Cl-Trt resin. Characterization of the purified peptide: purity (calculated by area integration of the HPLC chromatogram)>99.0%; ESI: m/z = 1191.7([M+H]⁺).

### Example 28: Preparation of Octanoyl-Dab-Thr-Orn-ring(4-10)[Dab-Dab-D-Phe-Thr-Dab-Dab-Thr] (compound 28)

Compound 28 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Orn(Boc)-OH, Fmoc-Thr(tBu)-OH, Fmoc-Dab(Boc)-OH, octanoic acid, Fmoc-Thr(tBu)-OH.

The yield was 244 mg, corresponding to a yield rate of 40.9% based on 0.5 mmol of 2-Cl-Trt resin. Characterization of the purified peptide: purity (calculated by area integration of the HPLC chromatogram)>99.0%; ESI: m/z = 1191.7([M+H]⁺).

### Example 29: Preparation of Heptanoyl-Dab-Thr-Orn-ring(4-10)[Dab-Dab-D-Phe-Thr-Dab-Dab-Thr] (compound 29)

Compound 29 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Orn(Boc)-OH, Fmoc-Thr(tBu)-OH, Fmoc-Dab(Boc)-OH, heptanoic acid, Fmoc-Thr(tBu)-OH.

The yield was 240 mg, corresponding to a yield rate of 40.8% based on 0.5 mmol of 2-Cl-Trt resin. Characterization of the purified peptide: purity (calculated by area integration of the HPLC chromatogram)>99.0%; ESI: m/z = 1177.7([M+H]⁺).

### Example 30: Preparation of Nonanoyl-Dab-Thr-Orn-ring(4-10)[Dab-Dab-D-Phe-Thr-Dab-Dab-Thr] (compound 30)

Compound 30 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Orn(Boc)-OH, Fmoc-Thr(tBu)-OH, Fmoc-Dab(Boc)-OH, nonanoic acid, Fmoc-Thr(tBu)-OH.

The yield was 258 mg, corresponding to a yield rate of 42.8% based on 0.5 mmol of 2-Cl-Trt resin. Characterization of the purified peptide: purity (calculated by area integration of the HPLC chromatogram)>99.0%; ESI: m/z = 1205.7([M+H]⁺).

### Example 31: Preparation of (S)-6-methyloctanoyl-Dab-Thr-D-Dap-ring(4-10)[Dab-Dab-D-Phe-Leu-Dab-Dab-Thr] (compound 31)

Compound 31 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-D-Dap(Boc)-OH, Fmoc-Thr(tBu)-OH, Fmoc-Dab(Boc)-OH, (S)-6-methyloctanoic acid, Fmoc-Thr(tBu)-OH.

The yield was 241 mg, corresponding to a yield rate of 40.5% based on 0.5 mmol of 2-Cl-Trt resin. Characterization of the purified peptide: purity (calculated by area integration of the HPLC chromatogram)>99.0%; ESI: m/z = 1189.7([M+H]⁺).

### Example 32: Preparation of 6-methylheptanoyl-Dab-Thr-D-Dap-ring(4-10)[Dab-Dab-D-Phe-Leu-Dab-Dab-Thr] (compound 32)

Compound 32 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-D-Dap(Boc)-OH, Fmoc-Thr(tBu)-OH, Fmoc-Dab(Boc)-OH, 6-methylheptanoic acid, Fmoc-Thr(tBu)-OH.

The yield was 245 mg, corresponding to a yield rate of 41.7% based on 0.5 mmol of 2-Cl-Trt resin. Characterization of the purified peptide: purity (calculated by area integration of the HPLC chromatogram)>99.0%; ESI: m/z = 1175.7([M+H]⁺).

### Example 33: Preparation of Octanoyl-Dab-Thr-D-Dap-ring(4-10)[Dab-Dab-D-Phe-Leu-Dab-Dab-Thr] (compound 33)

Compound 33 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-D-Dap(Boc)-OH, Fmoc-Thr(tBu)-OH, Fmoc-Dab(Boc)-OH, octanoic acid, Fmoc-Thr(tBu)-OH.

The yield was 238 mg, corresponding to a yield rate of 40.5% based on 0.5 mmol of 2-Cl-Trt resin. Characterization of the purified peptide: purity (calculated by area integration of the HPLC chromatogram)>99.0%; ESI: m/z = 1175.7([M+H]⁺).

### Example 34: Preparation of Heptanoyl-Dab-Thr-D-Dap-ring(4-10)[Dab-Dab-D-Phe-Leu-Dab-Dab-Thr] (compound 34)

Compound 34 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-D-Dap(Boc)-OH, Fmoc-Thr(tBu)-OH, Fmoc-Dab(Boc)-OH, heptanoic acid, Fmoc-Thr(tBu)-OH.

The yield was 235 mg, corresponding to a yield rate of 40.4% based on 0.5 mmol of 2-Cl-Trt resin. Characterization of the purified peptide: purity (calculated by area integration of the HPLC chromatogram)>99.0%; ESI: m/z = 1161.7([M+H]⁺).

### Example 35: Preparation of Nonanoyl-Dab-Thr-D-Dap-ring(4-10)[Dab-Dab-D-Phe-Leu-Dab-Dab-Thr] (compound 35)

Compound 35 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-D-Dap(Boc)-OH, Fmoc-Thr(tBu)-OH, Fmoc-Dab(Boc)-OH, nonanoic acid, Fmoc-Thr(tBu)-OH.

The yield was 254 mg, corresponding to a yield rate of 42.7% based on 0.5 mmol of 2-Cl-Trt resin. Characterization of the purified peptide: purity (calculated by area integration of the HPLC chromatogram)>99.0%; ESI: m/z = 1189.7([M+H]⁺).

### Example 36: Preparation of (S)-6-methyloctanoyl-Dab-Thr-Dap-ring(4-10)[Dab-Dab-D-Phe-Leu-Dab-Dab-Thr] (compound 36)

Compound 36 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-Dap(Boc)-OH, Fmoc-Thr(tBu)-OH, Fmoc-Dab(Boc)-OH, (S)-6-methyloctanoic acid, Fmoc-Thr(tBu)-OH.

The yield was 245 mg, corresponding to a yield rate of 41.2% based on 0.5 mmol of 2-Cl-Trt resin. Characterization of the purified peptide: purity (calculated by area integration of the HPLC chromatogram)>99.0%; ESI: m/z = 1189.7([M+H]⁺).

### Example 37: Preparation of 6-methylheptanoyl-Dab-Thr-Dap-ring(4-10)[Dab-Dab-D-Phe-Leu-Dab-Dab-Thr] (compound 37)

Compound 37 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-Dap(Boc)-OH, Fmoc-Thr(tBu)-OH, Fmoc-Dab(Boc)-OH, 6-methylheptanoic acid, Fmoc-Thr(tBu)-OH.

The yield was 245 mg, corresponding to a yield rate of 41.6% based on 0.5 mmol of 2-Cl-Trt resin. Characterization of the purified peptide: purity (calculated by area integration of the HPLC chromatogram)>99.0%; ESI: m/z = 1175.7([M+H]⁺).

### Example 38: Preparation of Octanoyl-Dab-Thr-Dap-ring(4-10)[Dab-Dab-D-Phe-Leu-Dab-Dab-Thr] (compound 38)

Compound 38 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-Dap(Boc)-OH, Fmoc-Thr(tBu)-OH, Fmoc-Dab(Boc)-OH, octanoic acid, Fmoc-Thr(tBu)-OH.

The yield was 235 mg, corresponding to a yield rate of 40.0% based on 0.5 mmol of 2-Cl-Trt resin. Characterization of the purified peptide: purity (calculated by area integration of the HPLC chromatogram)>99.0%; ESI: m/z = 1175.7([M+H]⁺).

### Example 39: Preparation of Heptanoyl-Dab-Thr-Dap-ring(4-10)[Dab-Dab-D-Phe-Leu-Dab-Dab-Thr] (compound 39)

Compound 39 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-Dap(Boc)-OH, Fmoc-Thr(tBu)-OH, Fmoc-Dab(Boc)-OH, heptanoic acid, Fmoc-Thr(tBu)-OH.

The yield was 235 mg, corresponding to a yield rate of 40.5% based on 0.5 mmol of 2-Cl-Trt resin. Characterization of the purified peptide: purity (calculated by area integration of the HPLC chromatogram)>99.0%; ESI: m/z = 1161.7([M+H]⁺).

### Example 40: Preparation of Nonanoyl-Dab-Thr-Dap-ring(4-10)[Dab-Dab-D-Phe-Leu-Dab-Dab-Thr] (compound 40)

Compound 40 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-Dap(Boc)-OH, Fmoc-Thr(tBu)-OH, Fmoc-Dab(Boc)-OH, nonanoic acid, Fmoc-Thr(tBu)-OH.

The yield was 250 mg, corresponding to a yield rate of 42.0% based on 0.5 mmol of 2-Cl-Trt resin. Characterization of the purified peptide: purity (calculated by area integration of the HPLC chromatogram)>99.0%; ESI: m/z = 1189.7([M+H]⁺).

### Example 41: Preparation of (S)-6-methyloctanoyl-Dab-Thr-D-Orn-ring(4-10)[Dab-Dab-D-Phe-Leu-Dab-Dab-Thr] (compound 41)

Compound 41 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-D-Orn(Boc)-OH, Fmoc-Thr(tBu)-OH, Fmoc-Dab(Boc)-OH, (S)-6-methyloctanoic acid, Fmoc-Thr(tBu)-OH.

The yield was 254 mg, corresponding to a yield rate of 41.7% based on 0.5 mmol of 2-Cl-Trt resin. Characterization of the purified peptide: purity (calculated by area integration of the HPLC chromatogram)>99.0%; ESI: m/z = 1217.7([M+H]⁺).

### Example 42: Preparation of 6-methylheptanoyl-Dab-Thr-D-Orn-ring(4-10)[Dab-Dab-D-Phe-Leu-Dab-Dab-Thr] (compound 42)

Compound 42 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-D-Orn(Boc)-OH, Fmoc-Thr(tBu)-OH, Fmoc-Dab(Boc)-OH, 6-methylheptanoic acid, Fmoc-Thr(tBu)-OH.

The yield was 245 mg, corresponding to a yield rate of 40.7% based on 0.5 mmol of 2-Cl-Trt resin. Characterization of the purified peptide: purity (calculated by area integration of the HPLC chromatogram)>99.0%; ESI: m/z = 1203.7([M+H]⁺).

### Example 43: Preparation of Octanoyl-Dab-Thr-D-Orn-ring(4-10)[Dab-Dab-D-Phe-Leu-Dab-Dab-Thr] (compound 43)

Compound 43 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-D-Orn(Boc)-OH, Fmoc-Thr(tBu)-OH, Fmoc-Dab(Boc)-OH, octanoic acid, Fmoc-Thr(tBu)-OH.

The yield was 244 mg, corresponding to a yield rate of 40.5% based on 0.5 mmol of 2-Cl-Trt resin. Characterization of the purified peptide: purity (calculated by area integration of the HPLC chromatogram)>99.0%; ESI: m/z = 1203.7([M+H]⁺).

### Example 44: Preparation of Heptanoyl-Dab-Thr-D-Orn-ring(4-10)[Dab-Dab-D-Phe-Leu-Dab-Dab-Thr] (compound 44)

Compound 44 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-D-Orn(Boc)-OH, Fmoc-Thr(tBu)-OH, Fmoc-Dab(Boc)-OH, heptanoic acid, Fmoc-Thr(tBu)-OH.

The yield was 239 mg, corresponding to a yield rate of 40.2% based on 0.5 mmol of 2-Cl-Trt resin. Characterization of the purified peptide: purity (calculated by area integration of the HPLC chromatogram)>99.0%; ESI: m/z = 1189.7([M+H]⁺).

### Example 45: Preparation of Nonanoyl-Dab-Thr-D-Orn-ring(4-10)[Dab-Dab-D-Phe-Leu-Dab-Dab-Thr] (compound 45)

Compound 45 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-D-Orn(Boc)-OH, Fmoc-Thr(tBu)-OH, Fmoc-Dab(Boc)-OH, nonanoic acid, Fmoc-Thr(tBu)-OH.

The yield was 255 mg, corresponding to a yield rate of 41.9% based on 0.5 mmol of 2-Cl-Trt resin. Characterization of the purified peptide: purity (calculated by area integration of the HPLC chromatogram)>99.0%; ESI: m/z = 1205.7([M+H]⁺).

### Example 46: Preparation of (S)-6-methyloctanoyl-Dab-Thr-Orn-ring(4-10)[Dab-Dab-D-Phe-Leu-Dab-Dab-Thr] (compound 46)

Compound 46 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-Orn(Boc)-OH, Fmoc-Thr(tBu)-OH, Fmoc-Dab(Boc)-OH, (S)-6-methyloctanoic acid, Fmoc-Thr(tBu)-OH.

The yield was 248 mg, corresponding to a yield rate of 40.7% based on 0.5 mmol of 2-Cl-Trt resin. Characterization of the purified peptide: purity (calculated by area integration of the HPLC chromatogram)>99.0%; ESI: m/z = 1217.7([M+H]⁺).

### Example 47: Preparation of 6-methylheptanoyl-Dab-Thr-Orn-ring(4-10)[Dab-Dab-D-Phe-Leu-Dab-Dab-Thr] (compound 47)

Compound 47 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-Orn(Boc)-OH, Fmoc-Thr(tBu)-OH, Fmoc-Dab(Boc)-OH, 6-methylheptanoic acid, Fmoc-Thr(tBu)-OH.

The yield was 241 mg, corresponding to a yield rate of 40.0% based on 0.5 mmol of 2-Cl-Trt resin. Characterization of the purified peptide: purity (calculated by area integration of the HPLC chromatogram)>99.0%; ESI: m/z = 1203.7([M+H]⁺).

### Example 48: Preparation of Octanoyl-Dab-Thr-Orn-ring(4-10)[Dab-Dab-D-Phe-Leu-Dab-Dab-Thr] (compound 48)

Compound 48 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-Orn(Boc)-OH, Fmoc-Thr(tBu)-OH, Fmoc-Dab(Boc)-OH, octanoic acid, Fmoc-Thr(tBu)-OH.

The yield was 245 mg, corresponding to a yield rate of 40.7% based on 0.5 mmol of 2-Cl-Trt resin. Characterization of the purified peptide: purity (calculated by area integration of the HPLC chromatogram)>99.0%; ESI: m/z = 1203.7([M+H]⁺).

### Example 49: Preparation of Heptanoyl-Dab-Thr-Orn-ring(4-10)[Dab-Dab-D-Phe-Leu-Dab-Dab-Thr] (compound 49)

Compound 49 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-Orn(Boc)-OH, Fmoc-Thr(tBu)-OH, Fmoc-Dab(Boc)-OH, heptanoic acid, Fmoc-Thr(tBu)-OH.

The yield was 245 mg, corresponding to a yield rate of 41.2% based on 0.5 mmol of 2-Cl-Trt resin. Characterization of the purified peptide: purity (calculated by area integration of the HPLC chromatogram)>99.0%; ESI: m/z = 1189.7([M+H]⁺).

### Example 50: Preparation of Nonanoyl-Dab-Thr-Orn-ring(4-10)[Dab-Dab-D-Phe-Leu-Dab-Dab-Thr] (compound 50)

Compound 50 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-Orn(Boc)-OH, Fmoc-Thr(tBu)-OH, Fmoc-Dab(Boc)-OH, nonanoic acid, Fmoc-Thr(tBu)-OH.

The yield was 250 mg, corresponding to a yield rate of 41.0% based on 0.5 mmol of 2-Cl-Trt resin. Characterization of the purified peptide: purity (calculated by area integration of the HPLC chromatogram)>99.0%; ESI: m/z = 1217.7([M+H]⁺).

### Example 51: Preparation of (S)-6-methyloctanoyl-Dap-Thr-D-Ser-ring(4-10)[Dab-Dab-D-Phe-Thr-Dab-Dab-Thr] (compound 51)

Compound 51 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dap(Boc)-OH, (S)-6-methyloctanoic acid, Fmoc-Thr(tBu)-OH.

The yield was 241 mg, corresponding to a yield rate of 41.4% based on 0.5 mmol of 2-Cl-Trt resin. Characterization of the purified peptide: purity (calculated by area integration of the HPLC chromatogram)>99.0%; ESI: m/z = 1164.7([M+H]⁺).

### Example 52: Preparation of 6-methylheptanoyl-Dap-Thr-D-Ser-ring(4-10)[Dab-Dab-D-Phe-Thr-Dab-Dab-Thr] (compound 52)

Compound 52 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dap(Boc)-OH, 6-methylheptanoic acid, Fmoc-Thr(tBu)-OH.

The yield was 241 mg, corresponding to a yield rate of 41.9% based on 0.5 mmol of 2-Cl-Trt resin. Characterization of the purified peptide: purity (calculated by area integration of the HPLC chromatogram)>99.0%; ESI: m/z = 1150.6([M+H]⁺).

### Example 53: Preparation of Octanoyl-Dap-Thr-D-Ser-ring(4-10)[Dab-Dab-D-Phe-Thr-Dab-Dab-Thr] (compound 53)

Compound 53 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dap(Boc)-OH, octanoic acid, Fmoc-Thr(tBu)-OH.

The yield was 235 mg, corresponding to a yield rate of 40.8% based on 0.5 mmol of 2-Cl-Trt resin. Characterization of the purified peptide: purity (calculated by area integration of the HPLC chromatogram)>99.0%; ESI: m/z = 1150.6([M+H]⁺).

### Example 54: Preparation of Heptanoyl-Dap-Thr-D-Ser-ring(4-10)[Dab-Dab-D-Phe-Thr-Dab-Dab-Thr] (compound 54)

Compound 54 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dap(Boc)-OH, heptanoic acid, Fmoc-Thr(tBu)-OH.

The yield was 235 mg, corresponding to a yield rate of 41.3% based on 0.5 mmol of 2-Cl-Trt resin. Characterization of the purified peptide: purity (calculated by area integration of the HPLC chromatogram)>99.0%; ESI: m/z = 1136.6([M+H]⁺).

### Example 55: Preparation of Nonanoyl-Dap-Thr-D-Ser-ring(4-10)[Dab-Dab-D-Phe-Thr-Dab-Dab-Thr] (compound 55)

Compound 55 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dap(Boc)-OH, nonanoic acid, Fmoc-Thr(tBu)-OH.

The yield was 250 mg, corresponding to a yield rate of 42.9% based on 0.5 mmol of 2-Cl-Trt resin. Characterization of the purified peptide: purity (calculated by area integration of the HPLC chromatogram)>99.0%; ESI: m/z = 1164.7([M+H]⁺).

### Example 56: Preparation of (S)-6-methyloctanoyl-Orn-Thr-D-Ser-ring(4-10)[Dab-Dab-D-Phe-Thr-Dab-Dab-Thr] (compound 56)

Compound 56 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Orn(Boc)-OH, (S)-6-methyloctanoic acid, Fmoc-Thr(tBu)-OH.

The yield was 240 mg, corresponding to a yield rate of 40.2% based on 0.5 mmol of 2-Cl-Trt resin. Characterization of the purified peptide: purity (calculated by area integration of the HPLC chromatogram)>99.0%; ESI: m/z = 1192.7([M+H]⁺).

### Example 57: Preparation of 6-methylheptanoyl-Orn-Thr-D-Ser-ring(4-10)[Dab-Dab-D-Phe-Thr-Dab-Dab-Thr] (compound 57)

Compound 57 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Orn(Boc)-OH, 6-methylheptanoic acid, Fmoc-Thr(tBu)-OH.

The yield was 240 mg, corresponding to a yield rate of 40.7% based on 0.5 mmol of 2-Cl-Trt resin. Characterization of the purified peptide: purity (calculated by area integration of the HPLC chromatogram)>99.0%; ESI: m/z = 1178.7([M+H]⁺).

### Example 58: Preparation of Octanoyl-Orn-Thr-D-Ser-ring(4-10)[Dab-Dab-D-Phe-Thr-Dab-Dab-Thr] (compound 58)

Compound 58 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Orn(Boc)-OH, octanoic acid, Fmoc-Thr(tBu)-OH.

The yield was 240 mg, corresponding to a yield rate of 40.7% based on 0.5 mmol of 2-Cl-Trt resin. Characterization of the purified peptide: purity (calculated by area integration of the HPLC chromatogram)>99.0%; ESI: m/z = 1178.7([M+H]⁺).

### Example 59: Preparation of Heptanoyl-Orn-Thr-D-Ser-ring(4-10)[Dab-Dab-D-Phe-Thr-Dab-Dab-Thr] (compound 59)

Compound 59 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Orn(Boc)-OH, heptanoic acid, Fmoc-Thr(tBu)-OH.

The yield was 238 mg, corresponding to a yield rate of 40.9% based on 0.5 mmol of 2-Cl-Trt resin. Characterization of the purified peptide: purity (calculated by area integration of the HPLC chromatogram)>99.0%; ESI: m/z = 1164.7([M+H]⁺).

### Example 60: Preparation of Nonanoyl-Orn-Thr-D-Ser-ring(4-10)[Dab-Dab-D-Phe-Thr-Dab-Dab-Thr] (compound 60)

Compound 60 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Orn(Boc)-OH, nonanoic acid, Fmoc-Thr(tBu)-OH.

The yield was 252 mg, corresponding to a yield rate of 42.3% based on 0.5 mmol of 2-Cl-Trt resin. Characterization of the purified peptide: purity (calculated by area integration of the HPLC chromatogram)>99.0%; ESI: m/z = 1192.7([M+H]⁺).

### Example 61: Preparation of (S)-6-methyloctanoyl-Dab-Thr-D-Ser-ring(4-10)[Dab-Dap-D-Phe-Thr-Dab-Dab-Thr] (compound 61)

Compound 61 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dap(Boc)-OH, Fmoc-Dab(Dde)-OH, Fmoc-D-Ser(tBu)-OH, Fmoc-Thr(tBu)-OH, Fmoc-Dab(Boc)-OH, (S)-6-methyloctanoic acid, Fmoc-Thr(tBu)-OH.

The yield was 241 mg, corresponding to a yield rate of 41.4% based on 0.5 mmol of 2-Cl-Trt resin. Characterization of the purified peptide: purity (calculated by area integration of the HPLC chromatogram)>99.0%; ESI: m/z = 1164.7([M+H]⁺).

### Example 62: Preparation of 6-methylheptanoyl-Dab-Thr-D-Ser-ring(4-10)[Dab-Dap-D-Phe-Thr-Dab-Dab-Thr] (compound 62)

Compound 62 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dap(Boc)-OH, Fmoc-Dab(Dde)-OH, Fmoc-D-Ser(tBu)-OH, Fmoc-Thr(tBu)-OH, Fmoc-Dab(Boc)-OH, 6-methylheptanoic acid, Fmoc-Thr(tBu)-OH.

The yield was 240 mg, corresponding to a yield rate of 41.7% based on 0.5 mmol of 2-Cl-Trt resin. Characterization of the purified peptide: purity (calculated by area integration of the HPLC chromatogram)>99.0%; ESI: m/z = 1150.6([M+H]⁺).

### Example 63: Preparation of Octanoyl-Dab-Thr-D-Ser-ring(4-10)[Dab-Dap-D-Phe-Thr-Dab-Dab-Thr] (compound 63)

Compound 63 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dap(Boc)-OH, Fmoc-Dab(Dde)-OH, Fmoc-D-Ser(tBu)-OH, Fmoc-Thr(tBu)-OH, Fmoc-Dab(Boc)-OH, octanoic acid, Fmoc-Thr(tBu)-OH.

The yield was 230 mg, corresponding to a yield rate of 40.0% based on 0.5 mmol of 2-Cl-Trt resin. Characterization of the purified peptide: purity (calculated by area integration of the HPLC chromatogram)>99.0%; ESI: m/z = 1150.6([M+H]⁺).

### Example 64: Preparation of Heptanoyl-Dab-Thr-D-Ser-ring(4-10)[Dab-Dap-D-Phe-Thr-Dab-Dab-Thr] (compound 64)

Compound 64 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dap(Boc)-OH, Fmoc-Dab(Dde)-OH, Fmoc-D-Ser(tBu)-OH, Fmoc-Thr(tBu)-OH, Fmoc-Dab(Boc)-OH, heptanoic acid, Fmoc-Thr(tBu)-OH.

The yield was 230 mg, corresponding to a yield rate of 40.5% based on 0.5 mmol of 2-Cl-Trt resin. Characterization of the purified peptide: purity (calculated by area integration of the HPLC chromatogram)>99.0%; ESI: m/z = 1136.6([M+H]⁺).

### Example 65: Preparation of Nonanoyl-Dab-Thr-D-Ser-ring(4-10)[Dab-Dap-D-Phe-Thr-Dab-Dab-Thr] (compound 65)

Compound 65 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dap(Boc)-OH, Fmoc-Dab(Dde)-OH, Fmoc-D-Ser(tBu)-OH, Fmoc-Thr(tBu)-OH, Fmoc-Dab(Boc)-OH, nonanoic acid, Fmoc-Thr(tBu)-OH.

The yield was 250 mg, corresponding to a yield rate of 42.9% based on 0.5 mmol of 2-Cl-Trt resin. Characterization of the purified peptide: purity (calculated by area integration of the HPLC chromatogram)>99.0%; ESI: m/z = 1164.7([M+H]⁺).

### Example 66: Preparation of (S)-6-methyloctanoyl-Dab-Thr-D-Ser-ring(4-10)[Dab-Orn-D-Phe-Thr-Dab-Dab-Thr] (compound 66)

Compound 66 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Orn(Boc)-OH, Fmoc-Dab(Dde)-OH, Fmoc-D-Ser(tBu)-OH, Fmoc-Thr(tBu)-OH, Fmoc-Dab(Boc)-OH, (S)-6-methyloctanoic acid, Fmoc-Thr(tBu)-OH.

The yield was 240 mg, corresponding to a yield rate of 40.2% based on 0.5 mmol of 2-Cl-Trt resin. Characterization of the purified peptide: purity (calculated by area integration of the HPLC chromatogram)>99.0%; ESI: m/z = 1192.7([M+H]⁺).

### Example 67: Preparation of 6-methylheptanoyl-Dab-Thr-D-Ser-ring(4-10)[Dab-Orn-D-Phe-Thr-Dab-Dab-Thr] (compound 67)

Compound 67 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Orn(Boc)-OH, Fmoc-Dab(Dde)-OH, Fmoc-D-Ser(tBu)-OH, Fmoc-Thr(tBu)-OH, Fmoc-Dab(Boc)-OH, 6-methylheptanoic acid, Fmoc-Thr(tBu)-OH.

The yield was 245 mg, corresponding to a yield rate of 41.5% based on 0.5 mmol of 2-Cl-Trt resin. Characterization of the purified peptide: purity (calculated by area integration of the HPLC chromatogram)>99.0%; ESI: m/z = 1178.7([M+H]⁺).

### Example 68: Preparation of Octanoyl-Dab-Thr-D-Ser-ring(4-10)[Dab-Orn-D-Phe-Thr-Dab-Dab-Thr] (compound 68)

Compound 68 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Orn(Boc)-OH, Fmoc-Dab(Dde)-OH, Fmoc-D-Ser(tBu)-OH, Fmoc-Thr(tBu)-OH, Fmoc-Dab(Boc)-OH, octanoic acid, Fmoc-Thr(tBu)-OH.

The yield was 236 mg, corresponding to a yield rate of 40.0% based on 0.5 mmol of 2-Cl-Trt resin. Characterization of the purified peptide: purity (calculated by area integration of the HPLC chromatogram)>99.0%; ESI: m/z = 1178.7([M+H]⁺).

### Example 69: Preparation of Heptanoyl-Dab-Thr-D-Ser-ring(4-10)[Dab-Orn-D-Phe-Thr-Dab-Dab-Thr] (compound 69)

Compound 69 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Orn(Boc)-OH, Fmoc-Dab(Dde)-OH, Fmoc-D-Ser(tBu)-OH, Fmoc-Thr(tBu)-OH, Fmoc-Dab(Boc)-OH, heptanoic acid, Fmoc-Thr(tBu)-OH.

The yield was 235 mg, corresponding to a yield rate of 40.3% based on 0.5 mmol of 2-Cl-Trt resin. Characterization of the purified peptide: purity (calculated by area integration of the HPLC chromatogram)>99.0%; ESI: m/z = 1164.7([M+H]⁺).

### Example 70: Preparation of Nonanoyl-Dab-Thr-D-Ser-ring(4-10)[Dab-Orn-D-Phe-Thr-Dab-Dab-Thr] (compound 70)

Compound 70 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Orn(Boc)-OH, Fmoc-Dab(Dde)-OH, Fmoc-D-Ser(tBu)-OH, Fmoc-Thr(tBu)-OH, Fmoc-Dab(Boc)-OH, nonanoic acid, Fmoc-Thr(tBu)-OH.

The yield was 250 mg, corresponding to a yield rate of 41.9% based on 0.5 mmol of 2-Cl-Trt resin. Characterization of the purified peptide: purity (calculated by area integration of the HPLC chromatogram)>99.0%; ESI: m/z = 1192.7([M+H]⁺).

### Example 71: Preparation of (S)-6-methyloctanoyl-Dab-Thr-D-Ser-ring(4-10)[Dab-Dab-D-Phe-Thr-Dap-Dab-Thr] (compound 71)

Compound 71 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dap(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, (S)-6-methyloctanoic acid, Fmoc-Thr(tBu)-OH.

The yield was 240 mg, corresponding to a yield rate of 41.2% based on 0.5 mmol of 2-Cl-Trt resin. Characterization of the purified peptide: purity (calculated by area integration of the HPLC chromatogram)>99.0%; ESI: m/z = 1164.7([M+H]⁺).

### Example 72: Preparation of 6-methylheptanoyl-Dab-Thr-D-Ser-ring(4-10)[Dab-Dab-D-Phe-Thr-Dap-Dab-Thr] (compound 72)

Compound 72 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dap(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, 6-methylheptanoic acid, Fmoc-Thr(tBu)-OH.

The yield was 240 mg, corresponding to a yield rate of 41.9% based on 0.5 mmol of 2-Cl-Trt resin. Characterization of the purified peptide: purity (calculated by area integration of the HPLC chromatogram)>99.0%; ESI: m/z = 1150.6([M+H]⁺).

### Example 73: Preparation of Octanoyl-Dab-Thr-D-Ser-ring(4-10)[Dab-Dab-D-Phe-Thr-Dap-Dab-Thr] (compound 73)

Compound 73 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dap(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, octanoic acid, Fmoc-Thr(tBu)-OH.

The yield was 231 mg, corresponding to a yield rate of 40.1% based on 0.5 mmol of 2-Cl-Trt resin. Characterization of the purified peptide: purity (calculated by area integration of the HPLC chromatogram)>99.0%; ESI: m/z = 1150.6([M+H]⁺).

### Example 74: Preparation of Heptanoyl-Dab-Thr-D-Ser-ring(4-10)[Dab-Dab-D-Phe-Thr-Dap-Dab-Thr] (compound 74)

Compound 74 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dap(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, heptanoic acid, Fmoc-Thr(tBu)-OH.

The yield was 231 mg, corresponding to a yield rate of 40.6% based on 0.5 mmol of 2-Cl-Trt resin. Characterization of the purified peptide: purity (calculated by area integration of the HPLC chromatogram)>99.0%; ESI: m/z = 1136.6([M+H]⁺).

### Example 75: Preparation of Nonanoyl-Dab-Thr-D-Ser-ring(4-10)[Dab-Dab-D-Phe-Thr-Dap-Dab-Thr] (compound 75)

Compound 75 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dap(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, nonanoic acid, Fmoc-Thr(tBu)-OH.

The yield was 250 mg, corresponding to a yield rate of 42.9% based on 0.5 mmol of 2-Cl-Trt resin. Characterization of the purified peptide: purity (calculated by area integration of the HPLC chromatogram)>99.0%; ESI: m/z = 1164.7([M+H]⁺).

### Example 76: Preparation of (S)-6-methyloctanoyl-Dab-Thr-D-Ser-ring(4-10)[Dab-Dab-D-Phe-Thr-Orn-Dab-Thr] (compound 76)

Compound 76 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Om(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, (S)-6-methyloctanoic acid, Fmoc-Thr(tBu)-OH.

The yield was 241 mg, corresponding to a yield rate of 40.3% based on 0.5 mmol of 2-Cl-Trt resin. Characterization of the purified peptide: purity (calculated by area integration of the HPLC chromatogram)>99.0%; ESI: m/z = 1192.7([M+H]⁺).

### Example 77: Preparation of 6-methylheptanoyl-Dab-Thr-D-Ser-ring(4-10)[Dab-Dab-D-Phe-Thr-Orn-Dab-Thr] (compound 77)

Compound 77 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Om(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, 6-methylheptanoic acid, Fmoc-Thr(tBu)-OH.

The yield was 246 mg, corresponding to a yield rate of 41.7% based on 0.5 mmol of 2-Cl-Trt resin. Characterization of the purified peptide: purity (calculated by area integration of the HPLC chromatogram)>99.0%; ESI: m/z = 1178.7([M+H]⁺).

### Example 78: Preparation of Octanoyl-Dab-Thr-D-Ser-ring(4-10)[Dab-Dab-D-Phe-Thr-Orn-Dab-Thr] (compound 78)

Compound 78 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Om(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, octanoic acid, Fmoc-Thr(tBu)-OH.

The yield was 238 mg, corresponding to a yield rate of 40.2% based on 0.5 mmol of 2-Cl-Trt resin. Characterization of the purified peptide: purity (calculated by area integration of the HPLC chromatogram)>99.0%; ESI: m/z = 1178.7([M+H]⁺).

### Example 79: Preparation of Heptanoyl-Dab-Thr-D-Ser-ring(4-10)[Dab-Dab-D-Phe-Thr-Orn-Dab-Thr] (compound 79)

Compound 79 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Om(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, heptanoic acid, Fmoc-Thr(tBu)-OH.

The yield was 236 mg, corresponding to a yield rate of 40.5% based on 0.5 mmol of 2-Cl-Trt resin. Characterization of the purified peptide: purity (calculated by area integration of the HPLC chromatogram)>99.0%; ESI: m/z = 1164.7([M+H]⁺).

### Example 80: Preparation of Nonanoyl-Dab-Thr-D-Ser-ring(4-10)[Dab-Dab-D-Phe-Thr-Orn-Dab-Thr] (compound 80)

Compound 80 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Om(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, nonanoic acid, Fmoc-Thr(tBu)-OH.

The yield was 251 mg, corresponding to a yield rate of 42.2% based on 0.5 mmol of 2-Cl-Trt resin. Characterization of the purified peptide: purity (calculated by area integration of the HPLC chromatogram)>99.0%; ESI: m/z = 1192.7([M+H]⁺).

### Example 81: Preparation of (S)-6-methyloctanoyl-Dab-Thr-D-Ser-ring(4-10)[Dab-Dab-D-Phe-Thr-Dab-Dap-Thr] (compound 81)

Compound 81 was prepared under the identical reaction conditions and purification procedure as those adopted for Compound 1. The addition sequence of the protected amino acids and side-chain carboxylic acids involved in the synthetic route was as follows: Fmoc-Dap-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, (S)-6-methyloctanoic acid, Fmoc-Thr(tBu)-OH.

The yield was 241 mg, corresponding to a yield rate of 41.3% based on 0.5 mmol of 2-Cl-Trt resin. Characterization of the purified peptide: purity (calculated by area integration of the HPLC chromatogram)>99.0%; ESI: m/z = 1164.7([M+H]⁺).

### Example 82: Preparation of 6-methylheptanoyl-Dab-Thr-D-Ser-ring(4-10)[Dab-Dab-D-Phe-Thr-Dab-Dap-Thr] (compound 82)

Compound 82 was prepared under the identical reaction conditions and purification procedure as those adopted for Compound 1. The addition sequence of the protected amino acids and side-chain carboxylic acids involved in the synthetic route was as follows: Fmoc-Dap-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, 6-methylheptanoic acid, Fmoc-Thr(tBu)-OH.

The yield was 240 mg, corresponding to a yield rate of 41.9% based on 0.5 mmol of 2-Cl-Trt resin. Characterization of the purified peptide: purity (calculated by area integration of the HPLC chromatogram)>99.0%; ESI: m/z = 1150.6([M+H]⁺).

### Example 83: Preparation of Octanoyl-Dab-Thr-D-Ser-ring(4-10)[Dab-Dab-D-Phe-Thr-Dab-Dap-Thr] (compound 83)

Compound 83 was prepared under the identical reaction conditions and purification procedure as those adopted for Compound 1. The addition sequence of the protected amino acids and side-chain carboxylic acids involved in the synthetic route was as follows: Fmoc-Dap-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, octanoic acid, Fmoc-Thr(tBu)-OH.

The yield was 232 mg, corresponding to a yield rate of 40.3% based on 0.5 mmol of 2-Cl-Trt resin. Characterization of the purified peptide: purity (calculated by area integration of the HPLC chromatogram)>99.0%; ESI: m/z = 1150.6([M+H]⁺).

### Example 84: Preparation of Heptanoyl-Dab-Thr-D-Ser-ring(4-10)[Dab-Dab-D-Phe-Thr-Dab-Dap-Thr] (compound 84)

Compound 84 was prepared under the identical reaction conditions and purification procedure as those adopted for Compound 1. The addition sequence of the protected amino acids and side-chain carboxylic acids involved in the synthetic route was as follows: Fmoc-Dap-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, heptanoic acid, Fmoc-Thr(tBu)-OH.

The yield was 231 mg, corresponding to a yield rate of 40.6% based on 0.5 mmol of 2-Cl-Trt resin. Characterization of the purified peptide: purity (calculated by area integration of the HPLC chromatogram)>99.0%; ESI: m/z = 1136.6([M+H]⁺).

### Example 85: Preparation of Nonanoyl-Dab-Thr-D-Ser-ring(4-10)[Dab-Dab-D-Phe-Thr-Dab-Dap-Thr] (compound 85)

Compound 85 was prepared under the identical reaction conditions and purification procedure as those adopted for Compound 1. The addition sequence of the protected amino acids and side-chain carboxylic acids involved in the synthetic route was as follows: Fmoc-Dap-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, nonanoic acid, Fmoc-Thr(tBu)-OH.

The yield was 249 mg, corresponding to a yield rate of 42.7% based on 0.5 mmol of 2-Cl-Trt resin. Characterization of the purified peptide: purity (calculated by area integration of the HPLC chromatogram)>99.0%; ESI: m/z = 1164.7([M+H]⁺).

### Example 86: Preparation of (S)-6-methyloctanoyl-Dab-Thr-D-Ser-ring(4-10)[Dab-Dab-D-Phe-Thr-Dab-Orn-Thr] (compound 86)

Compound 86 was prepared under the identical reaction conditions and purification procedure as those adopted for Compound 1. The addition sequence of the protected amino acids and side-chain carboxylic acids involved in the synthetic route was as follows: Fmoc-Om-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, (S)-6-methyloctanoic acid, Fmoc-Thr(tBu)-OH.

The yield was 240 mg, corresponding to a yield rate of 40.1% based on 0.5 mmol of 2-Cl-Trt resin. Characterization of the purified peptide: purity (calculated by area integration of the HPLC chromatogram)>99.0%; ESI: m/z = 1192.7([M+H]⁺).

### Example 87: Preparation of 6-methylheptanoyl-Dab-Thr-D-Ser-ring(4-10)[Dab-Dab-D-Phe-Thr-Dab-Orn-Thr] (compound 87)

Compound 87 was prepared under the identical reaction conditions and purification procedure as those adopted for Compound 1. The addition sequence of the protected amino acids and side-chain carboxylic acids involved in the synthetic route was as follows: Fmoc-Om-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, 6-methylheptanoic acid, Fmoc-Thr(tBu)-OH.

The yield was 245 mg, corresponding to a yield rate of 41.5% based on 0.5 mmol of 2-Cl-Trt resin. Characterization of the purified peptide: purity (calculated by area integration of the HPLC chromatogram)>99.0%; ESI: m/z = 1178.7([M+H]⁺).

### Example 88: Preparation of Octanoyl-Dab-Thr-D-Ser-ring(4-10)[Dab-Dab-D-Phe-Thr-Dab-Orn-Thr] (compound 88)

Compound 88 was prepared under the identical reaction conditions and purification procedure as those adopted for Compound 1. The addition sequence of the protected amino acids and side-chain carboxylic acids involved in the synthetic route was as follows: Fmoc-Om-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, octanoic acid, Fmoc-Thr(tBu)-OH.

The yield was 237 mg, corresponding to a yield rate of 40.0% based on 0.5 mmol of 2-Cl-Trt resin. Characterization of the purified peptide: purity (calculated by area integration of the HPLC chromatogram)>99.0%; ESI: m/z = 1178.7([M+H]⁺).

### Example 89: Preparation of Heptanoyl-Dab-Thr-D-Ser-ring(4-10)[Dab-Dab-D-Phe-Thr-Dab-Orn-Thr] (compound 89)

Compound 89 was prepared under the identical reaction conditions and purification procedure as those adopted for Compound 1. The addition sequence of the protected amino acids and side-chain carboxylic acids involved in the synthetic route was as follows: Fmoc-Om-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, heptanoic acid, Fmoc-Thr(tBu)-OH.

The yield was 235 mg, corresponding to a yield rate of 40.3% based on 0.5 mmol of 2-Cl-Trt resin. Characterization of the purified peptide: purity (calculated by area integration of the HPLC chromatogram)>99.0%; ESI: m/z = 1164.7([M+H]⁺).

### Example 90: Preparation of Nonanoyl-Dab-Thr-D-Ser-ring(4-10)[Dab-Dab-D-Phe-Thr-Dab-Orn-Thr] (compound 90)

Compound 90 was prepared under the identical reaction conditions and purification procedure as those adopted for Compound 1. The addition sequence of the protected amino acids and side-chain carboxylic acids involved in the synthetic route was as follows: Fmoc-Om-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, nonanoic acid, Fmoc-Thr(tBu)-OH.

The yield was 251 mg, corresponding to a yield rate of 42.2% based on 0.5 mmol of 2-Cl-Trt resin. Characterization of the purified peptide: purity (calculated by area integration of the HPLC chromatogram)>99.0%; ESI: m/z = 1192.7([M+H]⁺).

### Example 91: Preparation of (S)-6-methyloctanoyl-Dap-Thr-D-Dab-ring(4-10)[Dab-Dab-D-Phe-Leu-Dab-Dab-Thr] (compound 91)

Compound 91 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-D-Dab(Boc)-OH, Fmoc-Thr(tBu)-OH, Fmoc-Dap(Boc)-OH, (S)-6-methyloctanoic acid, Fmoc-Thr(tBu)-OH.

The yield was 241 mg, corresponding to a yield rate of 40.5% based on 0.5 mmol of 2-Cl-Trt resin. Characterization of the purified peptide: purity (calculated by area integration of the HPLC chromatogram)>99.0%; ESI: m/z = 1189.7([M+H]⁺).

### Example 92: Preparation of 6-methylheptanoyl-Dap-Thr-D-Dab-ring(4-10)[Dab-Dab-D-Phe-Leu-Dab-Dab-Thr] (compound 92)

Compound 92 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-D-Dab(Boc)-OH, Fmoc-Thr(tBu)-OH, Fmoc-Dap(Boc)-OH, 6-methylheptanoic acid, Fmoc-Thr(tBu)-OH.

The yield was 241 mg, corresponding to a yield rate of 41.0% based on 0.5 mmol of 2-Cl-Trt resin. Characterization of the purified peptide: purity (calculated by area integration of the HPLC chromatogram)>99.0%; ESI: m/z = 1175.7([M+H]⁺).

### Example 93: Preparation of Octanoyl-Dap-Thr-D-Dab-ring(4-10)[Dab-Dab-D-Phe-Leu-Dab-Dab-Thr] (compound 93)

Compound 93 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-D-Dab(Boc)-OH, Fmoc-Thr(tBu)-OH, Fmoc-Dap(Boc)-OH, octanoic acid, Fmoc-Thr(tBu)-OH.

The yield was 240 mg, corresponding to a yield rate of 40.8% based on 0.5 mmol of 2-Cl-Trt resin. Characterization of the purified peptide: purity (calculated by area integration of the HPLC chromatogram)>99.0%; ESI: m/z = 1175.7([M+H]⁺).

### Example 94: Preparation of Heptanoyl-Dap-Thr-D-Dab-ring(4-10)[Dab-Dab-D-Phe-Leu-Dab-Dab-Thr] (compound 94)

Compound 94 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-D-Dab(Boc)-OH, Fmoc-Thr(tBu)-OH, Fmoc-Dap(Boc)-OH, heptanoic acid, Fmoc-Thr(tBu)-OH.

The yield was 240 mg, corresponding to a yield rate of 41.3% based on 0.5 mmol of 2-Cl-Trt resin. Characterization of the purified peptide: purity (calculated by area integration of the HPLC chromatogram)>99.0%; ESI: m/z = 1161.7([M+H]⁺).

### Example 95: Preparation of Nonanoyl-Dap-Thr-D-Dab-ring(4-10)[Dab-Dab-D-Phe-Leu-Dab-Dab-Thr] (compound 95)

Compound 95 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Phe-OH, Fmoc-Dab(Boc)-OH, Fmoc-Dab(Dde)-OH, Fmoc-D-Dab(Boc)-OH, Fmoc-Thr(tBu)-OH, Fmoc-Dap(Boc)-OH, nonanoic acid, Fmoc-Thr(tBu)-OH.

The yield was 240 mg, corresponding to a yield rate of 40.3% based on 0.5 mmol of 2-Cl-Trt resin. Characterization of the purified peptide: purity (calculated by area integration of the HPLC chromatogram)>99.0%; ESI: m/z = 1189.7([M+H]⁺).

### Example 96: Preparation of (S)-6-methyloctanoyl-Dap-Thr-D-Dab-ring(4-10)[Dab-Dab-D-Leu-Leu-Dab-Dab-Thr] (compound 96)

Compound 96 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Leu-OH, Fmoc-Dab(Boc)-OH, Fmoc-Dab(Dde)-OH, Fmoc-D-Dab(Boc)-OH, Fmoc-Thr(tBu)-OH, Fmoc-Dap(Boc)-OH, (S)-6-methyloctanoic acid, Fmoc-Thr(tBu)-OH.

The yield was 240 mg, corresponding to a yield rate of 41.5% based on 0.5 mmol of 2-Cl-Trt resin. Characterization of the purified peptide: purity (calculated by area integration of the HPLC chromatogram)>99.0%; ESI: m/z = 1155.7([M+H]⁺).

### Example 97: Preparation of 6-methylheptanoyl-Dap-Thr-D-Dab-ring(4-10)[Dab-Dab-D-Leu-Leu-Dab-Dab-Thr] (compound 97)

Compound 97 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Leu-OH, Fmoc-Dab(Boc)-OH, Fmoc-Dab(Dde)-OH, Fmoc-D-Dab(Boc)-OH, Fmoc-Thr(tBu)-OH, Fmoc-Dap(Boc)-OH, 6-methylheptanoic acid, Fmoc-Thr(tBu)-OH.

The yield was 240 mg, corresponding to a yield rate of 42.0% based on 0.5 mmol of 2-Cl-Trt resin. Characterization of the purified peptide: purity (calculated by area integration of the HPLC chromatogram)>99.0%; ESI: m/z = 1141.7([M+H]⁺).

### Example 98: Preparation of Octanoyl-Dap-Thr-D-Dab-ring(4-10)[Dab-Dab-D-Leu-Leu-Dab-Dab-Thr] (compound 98)

Compound 98 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Leu-OH, Fmoc-Dab(Boc)-OH, Fmoc-Dab(Dde)-OH, Fmoc-D-Dab(Boc)-OH, Fmoc-Thr(tBu)-OH, Fmoc-Dap(Boc)-OH, octanoic acid, Fmoc-Thr(tBu)-OH.

The yield was 241 mg, corresponding to a yield rate of 42.2% based on 0.5 mmol of 2-Cl-Trt resin. Characterization of the purified peptide: purity (calculated by area integration of the HPLC chromatogram)>99.0%; ESI: m/z = 1141.7([M+H]⁺).

### Example 99: Preparation of Heptanoyl-Dap-Thr-D-Dab-ring(4-10)[Dab-Dab-D-Leu-Leu-Dab-Dab-Thr] (compound 99)

Compound 99 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Leu-OH, Fmoc-Dab(Boc)-OH, Fmoc-Dab(Dde)-OH, Fmoc-D-Dab(Boc)-OH, Fmoc-Thr(tBu)-OH, Fmoc-Dap(Boc)-OH, heptanoic acid, Fmoc-Thr(tBu)-OH.

The yield was 230 mg, corresponding to a yield rate of 40.8% based on 0.5 mmol of 2-Cl-Trt resin. Characterization of the purified peptide: purity (calculated by area integration of the HPLC chromatogram)>99.0%; ESI: m/z = 1127.7([M+H]⁺).

### Example 100: Preparation of Nonanoyl-Dap-Thr-D-Dab-ring(4-10)[Dab-Dab-D-Leu-Leu-Dab-Dab-Thr] (compound 100)

Compound 100 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Leu-OH, Fmoc-D-Leu-OH, Fmoc-Dab(Boc)-OH, Fmoc-Dab(Dde)-OH, Fmoc-D-Dab(Boc)-OH, Fmoc-Thr(tBu)-OH, Fmoc-Dap(Boc)-OH, nonanoic acid, Fmoc-Thr(tBu)-OH.

The yield was 235 mg, corresponding to a yield rate of 40.7% based on 0.5 mmol of 2-Cl-Trt resin. Characterization of the purified peptide: purity (calculated by area integration of the HPLC chromatogram)>99.0%; ESI: m/z = 1155.7([M+H]⁺).

### Example 101: Preparation of (S)-6-methyloctanoyl-Dab-Thr-D-Dap-ring(4-10)[Dab-Dab-Phe-Thr-Dab-Dab-Thr] (compound 101)

Compound 101 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-Phe-OH, Fmoc-Dab(Boc)-OH, Fmoc-Dab(Dde)-OH, Fmoc-D-Dap(Boc)-OH, Fmoc-Thr(tBu)-OH, Fmoc-Dab(Boc)-OH, (S)-6-methyloctanoic acid, Fmoc-Thr(tBu)-OH.

The yield was 240 mg, corresponding to a yield rate of 40.7% based on 0.5 mmol of 2-Cl-Trt resin. Characterization of the purified peptide: purity (calculated by area integration of the HPLC chromatogram)>99.0%; ESI: m/z = 1177.7([M+H]⁺).

### Example 102: Preparation of 6-methylheptanoyl-Dab-Thr-D-Dap-ring(4-10)[Dab-Dab-Phe-Thr-Dab-Dab-Thr] (compound 102)

Compound 102 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-Phe-OH, Fmoc-Dab(Boc)-OH, Fmoc-Dab(Dde)-OH, Fmoc-D-Dap(Boc)-OH, Fmoc-Thr(tBu)-OH, Fmoc-Dab(Boc)-OH, 6-methylheptanoic acid, Fmoc-Thr(tBu)-OH.

The yield was 240 mg, corresponding to a yield rate of 41.2% based on 0.5 mmol of 2-Cl-Trt resin. Characterization of the purified peptide: purity (calculated by area integration of the HPLC chromatogram)>99.0%; ESI: m/z = 1163.7([M+H]⁺).

### Example 103: Preparation of 6-methylheptanoyl-Dab-Thr-D-Ser(CH₂SO₃H)-ring(4-10)[Dab-Dab-D-Phe-Thr-Dab-Dab-Thr] (compound 103)

Compound 103 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(CH₂SO₃H)-OH, Fmoc-Thr(tBu)-OH, Fmoc-Dab(Boc)-OH, 6-methylheptanoic acid, Fmoc-Thr(tBu)-OH.

The yield was 240 mg, corresponding to a yield rate of 38.1% based on 0.5 mmol of 2-Cl-Trt resin. Characterization of the purified peptide: purity (calculated by area integration of the HPLC chromatogram)>99.0%; ESI: m/z = 1258.6([M+H]⁺).

### Example 104: Preparation of 6-methylheptanoyl-Dab-Thr(CH₂SO₃H)-D-Ser-ring(4-10)[Dab-Dab-D-Phe-Thr-Dab-Dab-Thr] (compound 104)

Compound 104 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(CH₂SO₃H)-OH, Fmoc-Dab(Boc)-OH, 6-methylheptanoic acid, Fmoc-Thr(tBu)-OH.

The yield was 245 mg, corresponding to a yield rate of 38.9% based on 0.5 mmol of 2-Cl-Trt resin. Characterization of the purified peptide: purity (calculated by area integration of the HPLC chromatogram)>99.0%; ESI: m/z = 1258.6([M+H]⁺).

### Example 105: Preparation of 6-methylheptanoyl-Dab-Thr-D-Ser-ring(4-10)[Dab-Dab-D-Phe-Thr(CH₂SO₃H)-Dab-Dab-Thr] (compound 105)

Compound 105 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(CH₂SO₃H)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, 6-methylheptanoic acid, Fmoc-Thr(tBu)-OH.

The yield was 248 mg, corresponding to a yield rate of 39.4% based on 0.5 mmol of 2-Cl-Trt resin. Characterization of the purified peptide: purity (calculated by area integration of the HPLC chromatogram)>99.0%; ESI: m/z = 1258.6([M+H]⁺).

### Example 106: Preparation of 6-methylheptanoyl-Dab-Thr-D-Ser-ring(4-10)[Dab-Dab-D-Phe-Thr-Dab-Dab-Thr(CH₂SO₃H)] (compound 106)

Compound 106 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, 6-methylheptanoic acid, Fmoc-Thr(CH₂SO₃H)-OH.

The yield was 250 mg, corresponding to a yield rate of 39.7% based on 0.5 mmol of 2-Cl-Trt resin. Characterization of the purified peptide: purity (calculated by area integration of the HPLC chromatogram)>99.0%; ESI: m/z = 1258.6([M+H]⁺).

### Example 107: Preparation of 6-methylheptanoyl-Dab-Thr-D-Ser-ring(4-10)[Dab-Dab-D-Phe(4-CH₂SO₃H)-Thr-Dab-Dab-Thr] (compound 107)

Compound 107 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe(4-CH₂SO₃H)-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, 6-methylheptanoic acid, Fmoc-Thr(tBu)-OH.

The yield was 260 mg, corresponding to a yield rate of 41.3% based on 0.5 mmol of 2-Cl-Trt resin. Characterization of the purified peptide: purity (calculated by area integration of the HPLC chromatogram)>99.0%; ESI: m/z = 1258.6([M+H]⁺).

### Example 108: Preparation of 6-methylheptanoyl-Dab(CH₂SO₃H)-Thr-D-Ser-ring(4-10)[Dab-Dab-D-Phe-Thr-Dab-Dab-Thr] (compound 108)

Compound 108 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(CH₂SO₃H)-OH, 6-methylheptanoic acid, Fmoc-Thr(tBu)-OH.

The yield was 245 mg, corresponding to a yield rate of 38.9% based on 0.5 mmol of 2-Cl-Trt resin. Characterization of the purified peptide: purity (calculated by area integration of the HPLC chromatogram)>99.0%; ESI: m/z = 1258.6([M+H]⁺).

### Example 109: Preparation of 6-methylheptanoyl-Dab-Thr-D-Ser-ring(4-10)[Dab-Dab(CH₂SO₃H)-D-Phe-Thr-Dab-Dab-Thr] (compound 109)

Compound 109 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(CH₂SO₃H)-OH, Fmoc-Dab(Dde)-OH, Fmoc-D-Ser(tBu)-OH, Fmoc-Thr(tBu)-OH, Fmoc-Dab(Boc)-OH, 6-methylheptanoic acid, Fmoc-Thr(tBu)-OH.

The yield was 248 mg, corresponding to a yield rate of 39.4% based on 0.5 mmol of 2-Cl-Trt resin. Characterization of the purified peptide: purity (calculated by area integration of the HPLC chromatogram)>99.0%; ESI: m/z = 1258.6([M+H]⁺).

### Example 110: Preparation of 6-methylheptanoyl-Dab-Thr-D-Ser-ring(4-10)[Dab-Dab-D-Phe-Thr-Dab(CH₂SO₃H)-Dab-Thr] (compound 110)

Compound 110 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(CH₂SO₃)-OH, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, 6-methylheptanoic acid, Fmoc-Thr(tBu)-OH.

The yield was 250 mg, corresponding to a yield rate of 39.7% based on 0.5 mmol of 2-Cl-Trt resin. Characterization of the purified peptide: purity (calculated by area integration of the HPLC chromatogram)>99.0%; ESI: m/z = 1258.6([M+H]⁺).

### Example 111: Preparation of 6-methylheptanoyl-Dab-Thr-D-Ser-ring(4-10)[Dab-Dab-D-Phe-Thr-Dab-Dab(CH₂SO₃H)-Thr] (compound 111)

Compound 111 was prepared under the reaction conditions and purification procedure similar to those adopted for Compound 1. The addition sequence of the protected amino acids and side-chain carboxylic acids involved in the synthetic route was as follows: Fmoc-Dab-OAllyl, Fmoc-Thr(tBu)-OH, Fmoc-D-Phe-OH, Fmoc-Dab(Boc)-OH, Fmoc-Dab(Dde)-OH, Fmoc-D-Ser(tBu)-OH, Fmoc-Thr(tBu)-OH, Fmoc-Dab(Boc)-OH, 6-methylheptanoic acid, Fmoc-Thr(tBu)-OH, Fmoc-Dab(CH₂SO₃H)-OH.

The yield was 260 mg, corresponding to a yield rate of 41.3% based on 0.5 mmol of 2-Cl-Trt resin. Characterization of the purified peptide: purity (calculated by area integration of the HPLC chromatogram)>99.0%; ESI: m/z = 1258.6([M+H]⁺).

### Example 112: Preparation of 6-methylheptanoyl-Dab-Thr-Dap(CH₂SO₃H)-ring(4-10)[Dab-Dab-D-Phe-Thr-Dab-Dab-Thr] (compound 112)

Compound 112 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Dap(CH₂SO₃H)-OH, Fmoc-Thr(tBu)-OH, Fmoc-Dab(Boc)-OH, 6-methylheptanoic acid, Fmoc-Thr(tBu)-OH.

The yield was 240 mg, corresponding to a yield rate of 38.2% based on 0.5 mmol of 2-Cl-Trt resin. Characterization of the purified peptide: purity (calculated by area integration of the HPLC chromatogram)>99.0%; ESI: m/z = 1257.7([M+H]⁺).

### Example 113: Preparation of 6-methylheptanoyl-Dab-Thr(CH₂SO₃H)-Dap-ring(4-10)[Dab-Dab-D-Phe-Thr-Dab-Dab-Thr] (compound 113)

Compound 113 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Dap(Boc)-OH, Fmoc-Thr(CH₂SO₃H)-OH, Fmoc-Dab(Boc)-OH, 6-methylheptanoic acid, Fmoc-Thr(tBu)-OH.

The yield was 245 mg, corresponding to a yield rate of 38.9% based on 0.5 mmol of 2-Cl-Trt resin. Characterization of the purified peptide: purity (calculated by area integration of the HPLC chromatogram)>99.0%; ESI: m/z = 1257.7([M+H]⁺).

### Example 114: Preparation of 6-methylheptanoyl-Dab-Thr-Dap-ring(4-10)[Dab-Dab-D-Phe-Thr(CH₂SO₃H)-Dab-Dab-Thr] (compound 114)

Compound 114 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(CH₂SO₃H)-OH, Fmoc-D-Phe-OH, Fmoc-Dab(Boc)-OH, Fmoc-Dab(Dde)-OH, Fmoc-Dap(Boc)-OH, Fmoc-Thr(tBu)-OH, Fmoc-Dab(Boc)-OH, 6-methylheptanoic acid, Fmoc-Thr(tBu)-OH.

The yield was 248 mg, corresponding to a yield rate of 39.4% based on 0.5 mmol of 2-Cl-Trt resin. Characterization of the purified peptide: purity (calculated by area integration of the HPLC chromatogram)>99.0%; ESI: m/z = 1257.7([M+H]⁺).

### Example 115: Preparation of 6-methylheptanoyl-Dab-Thr-Dap-ring(4-10)[Dab-Dab-D-Phe-Thr-Dab-Dab-Thr(CH₂SO₃H)] (compound 115)

Compound 115 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Dap(Boc)-OH, Fmoc-Thr(tBu)-OH, Fmoc-Dab(Boc)-OH, 6-methylheptanoic acid, Fmoc-Thr(CH₂SO₃H)-OH.

The yield was 250 mg, corresponding to a yield rate of 39.7% based on 0.5 mmol of 2-Cl-Trt resin. Characterization of the purified peptide: purity (calculated by area integration of the HPLC chromatogram)>99.0%; ESI: m/z = 1257.7([M+H]⁺).

### Example 116: Preparation of 6-methylheptanoyl-Dab-Thr-Dap-ring(4-10)[Dab-Dab-D-Phe(4-CH₂SO₃H)-Thr-Dab-Dab-Thr] (compound 116)

Compound 116 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe(4-CH₂SO₃H)-OH, Fmoc-Dab(Boc)-OH, Fmoc-Dab(Dde)-OH, Fmoc-Dap(Boc)-OH, Fmoc-Thr(tBu)-OH, Fmoc-Dab(Boc)-OH, 6-methylheptanoic acid, Fmoc-Thr(tBu)-OH.

The yield was 260 mg, corresponding to a yield rate of 41.3% based on 0.5 mmol of 2-Cl-Trt resin. Characterization of the purified peptide: purity (calculated by area integration of the HPLC chromatogram)>99.0%; ESI: m/z = 1257.7([M+H]⁺).

### Example 117: Preparation of 6-methylheptanoyl-Dab(CH₂SO₃H)-Thr-Dap-ring(4-10)[Dab-Dab-D-Phe-Thr-Dab-Dab-Thr] (compound 117)

Compound 117 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(Boc)-OH, Fmoc-Dab(Dde)-OH, Fmoc-Dap(Boc)-OH, Fmoc-Thr(tBu)-OH, Fmoc-Dab(CH₂SO₃H)-OH, 6-methylheptanoic acid, Fmoc-Thr(tBu)-OH.

The yield was 245 mg, corresponding to a yield rate of 38.9% based on 0.5 mmol of 2-Cl-Trt resin. Characterization of the purified peptide: purity (calculated by area integration of the HPLC chromatogram)>99.0%; ESI: m/z = 1257.7([M+H]⁺).

### Example 118: Preparation of 6-methylheptanoyl-Dab-Thr-Dap-ring(4-10)[Dab-Dab(CH₂SO₃H)-D-Phe-Thr-Dab-Dab-Thr] (compound 118)

Compound 118 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(Boc)-OH, Fmoc-Thr(tBu)-OH, Fmoc-D-Phe-OH, Fmoc-Dab(CH₂SO₃H)-OH, Fmoc-Dab(Dde)-OH, Fmoc-Dap(Boc)-OH, Fmoc-Thr(tBu)-OH, Fmoc-Dab(Boc)-OH, 6-methylheptanoic acid, Fmoc-Thr(tBu)-OH.

The yield was 248 mg, corresponding to a yield rate of 39.4% based on 0.5 mmol of 2-Cl-Trt resin. Characterization of the purified peptide: purity (calculated by area integration of the HPLC chromatogram)>99.0%; ESI: m/z = 1257.7([M+H]⁺).

### Example 119: Preparation of 6-methylheptanoyl-Dab-Thr-Dap-ring(4-10)[Dab-Dab-D-Phe-Thr-Dab(CH₂SO₃H)-Dab-Thr] (compound 119)

Compound 119 was prepared under the identical reaction conditions and purification procedure as those adopted for Compound 1. The addition sequence of the protected amino acids and side-chain carboxylic acids involved in the synthetic route was as follows: Fmoc-Dab-OAllyl, Fmoc-Dab(CH₂SO₃)-OH, Fmoc-Thr(tBu)-OH, Fmoc-D-Phe-OH, Fmoc-Dab(Boc)-OH, Fmoc-Dab(Dde)-OH, Fmoc-Dap(Boc)-OH, Fmoc-Thr(tBu)-OH, Fmoc-Dab(Boc)-OH, 6-methylheptanoic acid, Fmoc-Thr(tBu)-OH.

The yield was 250 mg, corresponding to a yield rate of 39.7% based on 0.5 mmol of 2-Cl-Trt resin. Characterization of the purified peptide: purity (calculated by area integration of the HPLC chromatogram)>99.0%; ESI: m/z = 1257.7([M+H]⁺).

### Example 120: Preparation of 6-methylheptanoyl-Dab-Thr-Dap-ring(4-10)[Dab-Dab-D-Phe-Thr-Dab-Dab(CH₂SO₃H)-Thr] (compound 120)

Compound 120 was prepared under the reaction conditions and purification procedure similar to those adopted for Compound 1. The addition sequence of the protected amino acids and side-chain carboxylic acids involved in the synthetic route was as follows: Fmoc-Dab-OAllyl, Fmoc-Thr(tBu)-OH, Fmoc-D-Phe-OH, Fmoc-Dab(Boc)-OH, Fmoc-Dab(Dde)-OH, Fmoc-Dap(Boc)-OH, Fmoc-Thr(tBu)-OH, Fmoc-Dab(Boc)-OH, 6-methylheptanoic acid, Fmoc-Thr(tBu)-OH, Fmoc-Dab(CH₂SO₃H)-OH.

The yield was 260 mg, corresponding to a yield rate of 41.3% based on 0.5 mmol of 2-Cl-Trt resin. Characterization of the purified peptide: purity (calculated by area integration of the HPLC chromatogram)>99.0%; ESI: m/z = 1257.7([M+H]⁺).

### Experimental Example 1: Antibacterial activity assay

The minimal inhibitory concentration (MIC) was determined by the broth microdilution method according to the recommended procedures of the Clinical and Laboratory Standards Institute (CLSI).

An appropriate amount of the cyclic lipopeptide compounds prepared in Examples 1-120 and the reference standards were weighed and dissolved in sterile pure water. An appropriate volume of the mother solution was subjected to ten-fold dilution with sterile Mueller-Hinton (MH) broth to a concentration of 0.256 mg/mL. Half of the drug solution was aliquoted into 96-well reagent reservoirs, while the other half was subjected to two-fold serial dilution with sterile MH broth before being transferred into deep-well reagent reservoirs. The above steps were repeated, so that the drug concentrations in the reagent reservoirs were adjusted to 128, 64, 32, 16, 8, 4, 2, 1, 0.5, 0.25, 0.125 and 0.06 mg/L in sequence, and all solutions were prepared and used immediately. Several colonies were picked from agar plates cultured for 18-24 h and directly suspended in sterile normal saline, and the concentration of the bacterial suspension was adjusted to 0.5 McFarland units. The calibrated bacterial suspension was diluted with MH broth to a concentration of (4-8) × 10⁵ colony-forming units per milliliter (CFU/mL) and prepared and used immediately. A volume of 100 µL of the aforementioned compounds of the present disclosure (e.g., the compounds prepared in the examples) and reference solutions at different concentrations was separately pipetted into wells 1 to 12 of sterile 96-well polystyrene plates, and an additional 100 µL of the aforementioned inoculum was added to each well. The final concentrations of the samples in the wells were adjusted to 64, 32, 16, 8, 4, 2, 1, 0.5, 0.25, 0.125, 0.06 and 0.03 mg/L, respectively, and the final concentration of the inoculum in each well was (2-4) × 10⁵ CFU/mL. Additional growth control wells were set up, each containing 100 µL of the inoculum and 100 µL of sterile MH broth. The test substances and inocula in all wells were mixed thoroughly and then sealed. The inoculated 96-well plates were incubated in an incubator at 35-37°C for 16-20 h. After the incubation period, the bacterial growth in each well was observed, and the lowest drug concentration that completely inhibited bacterial growth in the wells was defined as the minimal inhibitory concentration (MIC).

The strains used for the antibacterial activity assay were obtained from the American Type Culture Collection (ATCC) and clinical isolates.

The strains employed for the antibacterial activity assay included *Escherichia coli* ATCC BAA-2523, ATCC BAA-2340 (KPC), *Klebsiella pneumoniae* KPN+19-15, ATCC BAA-1705 (KPC), *Pseudomonas aeruginosa* PAE19-3, ATCC 27853, *Acinetobacter baumannii* ABA19-1, and NCTC 13304 (OXA-27).

Test samples: Cyclic lipopeptide compounds prepared according to the technical solution of the present disclosure.

Reference standard: Polymyxin B.

### Results:

The activity of the cyclic lipopeptide compounds of the present disclosure against Gram-negative bacteria was presented in Table 2.

**Table 2 Antibacterial Activity of the Compounds against Gram-Negative Bacteria (MIC, Unit: µg/mL)**

| Compound (µg/mL) | *Escherichia coli* ATCC BAA-2 523 | *Escherichia coli* ATCC BAA-2 340 (KPC) | *Klebsiella pneumon ice* KPN 19-15 | *Klebsiella pneumon iae* ATCC BAA-17 05 (KPC) | *Pseudo monas aerugin osa* PAE19-3 | *Pseudo monas aerugin osa* ATCC2 7853 | *Acineto bacter bauman nii* ABA19 -1 | *Acinetob acter bauman nii* NCTC 13304 (OXA-2 7) |
|---|---|---|---|---|---|---|---|---|
| 1 | ≤0.03 | ≤0.03 | ≤0.03 | ≤0.03 | 0.125 | 0.03 | ≤0.03 | ≤0.03 |
| 2 | ≤0.03 | ≤0.03 | ≤0.03 | ≤0.03 | 0.125 | ≤0.03 | ≤0.03 | ≤0.03 |
| 3 | ≤0.03 | ≤0.03 | ≤0.03 | ≤0.03 | 0.25 | 0.03 | ≤0.03 | ≤0.03 |
| 4 | 0.03 | 0.03 | 0.03 | 0.03 | 0.25 | 0.03 | 0.03 | 0.03 |
| 5 | 0.06 | 0.06 | 0.06 | 0.06 | 0.5 | 0.06 | 0.06 | 0.06 |
| 6 | ≤0.03 | ≤0.03 | ≤0.03 | ≤0.03 | 0.125 | 0.03 | ≤0.03 | ≤0.03 |
| 7 | ≤0.03 | ≤0.03 | ≤0.03 | ≤0.03 | 0.125 | ≤0.03 | ≤0.03 | ≤0.03 |
| 8 | ≤0.03 | ≤0.03 | ≤0.03 | ≤0.03 | 0.25 | 0.125 | ≤0.03 | ≤0.03 |
| 9 | 0.03 | 0.03 | 0.03 | 0.03 | 0.25 | 0.125 | 0.03 | 0.03 |
| 10 | 0.06 | 0.06 | 0.06 | 0.06 | 0.5 | 0.25 | 0.06 | 0.06 |
| 11 | ≤0.03 | ≤0.03 | ≤0.03 | ≤0.03 | 1 | 1 | ≤0.03 | ≤0.03 |
| 12 | ≤0.03 | ≤0.03 | ≤0.03 | 0.125 | 0.5 | 0.5 | 0.06 | ≤0.03 |
| 13 | ≤0.03 | ≤0.03 | ≤0.03 | 0.06 | 1 | 1 | ≤0.03 | ≤0.03 |
| 14 | 0.03 | 0.03 | 0.03 | 0.06 | 2 | 2 | 0.03 | 0.03 |
| 15 | 0.06 | 0.06 | 0.06 | 0.06 | 2 | 2 | 0.06 | 0.06 |
| 16 | ≤0.03 | ≤0.03 | ≤0.03 | ≤0.03 | 0.5 | 0.5 | ≤0.03 | ≤0.03 |
| 17 | 0.06 | ≤0.03 | ≤0.03 | 0.5 | 0.5 | 0.5 | 0.5 | 0.25 |
| 18 | ≤0.03 | 0.06 | ≤0.03 | 0.5 | 0.5 | 0.5 | 0.5 | 0.25 |
| 19 | 0.03 | 0.03 | 0.03 | 0.06 | 2 | 2 | 0.5 | 0.25 |
| 20 | 0.06 | 0.06 | 0.06 | 0.06 | 2 | 2 | 0.5 | 0.25 |
| 21 | ≤0.03 | ≤0.03 | 0.125 | 0.06 | 1 | 0.25 | 0.125 | 0.06 |
| 22 | ≤0.03 | ≤0.03 | 0.125 | 0.06 | 1 | 0.25 | 0.125 | 0.06 |
| 23 | ≤0.03 | ≤0.03 | 0.125 | 0.06 | 1 | 0.25 | 0.125 | 0.06 |
| 24 | ≤0.03 | ≤0.03 | 0.125 | 0.06 | 2 | 0.5 | 0.125 | 0.06 |
| 25 | ≤0.03 | ≤0.03 | 0.125 | 0.06 | 2 | 0.5 | 0.125 | 0.06 |
| 26 | ≤0.03 | ≤0.03 | ≤0.03 | ≤0.03 | 0.25 | ≤0.03 | 0.125 | 0.06 |
| 27 | ≤0.03 | ≤0.03 | ≤0.03 | ≤0.03 | 0.25 | ≤0.03 | 0.125 | 0.06 |
| 28 | ≤0.03 | ≤0.03 | ≤0.03 | ≤0.03 | 0.25 | ≤0.03 | 0.125 | 0.06 |
| 29 | 0.03 | 0.03 | 0.03 | 0.03 | 0.5 | 0.03 | 0.125 | 0.06 |
| 30 | 0.03 | 0.03 | 0.03 | 0.03 | 0.5 | 0.03 | 0.125 | 0.06 |
| 31 | 0.03 | 0.03 | 0.03 | 0.03 | 0.25 | 0.03 | 0.03 | 0.03 |
| 32 | 0.03 | 0.03 | 0.03 | 0.03 | 0.25 | 0.03 | 0.03 | 0.03 |
| 33 | 0.03 | 0.03 | 0.03 | 0.03 | 0.25 | 0.03 | 0.03 | 0.03 |
| 34 | 0.06 | 0.06 | 0.06 | 0.06 | 0.5 | 0.06 | 0.06 | 0.06 |
| 35 | 0.06 | 0.06 | 0.06 | 0.06 | 0.5 | 0.06 | 0.06 | 0.06 |
| 36 | 0.03 | 0.03 | 0.03 | 0.03 | 0.25 | 0.03 | 0.03 | 0.03 |
| 37 | 0.03 | 0.03 | 0.03 | 0.03 | 0.25 | 0.03 | 0.03 | 0.03 |
| 38 | 0.03 | 0.03 | 0.03 | 0.03 | 0.25 | 0.03 | 0.03 | 0.03 |
| 39 | 0.03 | 0.03 | 0.03 | 0.03 | 0.5 | 0.06 | 0.03 | 0.03 |
| 40 | 0.06 | 0.06 | 0.06 | 0.06 | 0.5 | 0.06 | 0.06 | 0.06 |
| 41 | 0.03 | 0.03 | 0.125 | 0.06 | 1 | 0.25 | 0.125 | 0.06 |
| 42 | 0.03 | 0.03 | 0.125 | 0.06 | 1 | 0.25 | 0.125 | 0.06 |
| 43 | 0.03 | 0.03 | 0.125 | 0.06 | 1 | 0.25 | 0.125 | 0.06 |
| 44 | 0.06 | 0.06 | 0.125 | 0.06 | 2 | 0.5 | 0.125 | 0.06 |
| 45 | 0.06 | 0.06 | 0.125 | 0.06 | 2 | 0.5 | 0.125 | 0.06 |
| 46 | 0.03 | 0.03 | ≤0.03 | ≤0.03 | 0.25 | ≤0.03 | 0.125 | 0.06 |
| 47 | 0.03 | 0.03 | ≤0.03 | ≤0.03 | 0.25 | ≤0.03 | 0.125 | 0.06 |
| 48 | 0.03 | 0.03 | ≤0.03 | ≤0.03 | 0.25 | ≤0.03 | 0.125 | 0.06 |
| 49 | 0.03 | 0.03 | 0.03 | 0.03 | 0.5 | 0.03 | 0.125 | 0.06 |
| 50 | 0.06 | 0.06 | 0.03 | 0.03 | 0.5 | 0.03 | 0.125 | 0.06 |
| 51 | ≤0.03 | ≤0.03 | 0.5 | 0.25 | 4 | 64 | 0.25 | 0.25 |
| 52 | ≤0.03 | ≤0.03 | 0.5 | 0.25 | 4 | 64 | 0.25 | 0.25 |
| 53 | ≤0.03 | ≤0.03 | 0.5 | 0.25 | 4 | 64 | 0.25 | 0.25 |
| 54 | 0.03 | 0.03 | 0.5 | 0.25 | 8 | 64 | 0.25 | 0.25 |
| 55 | 0.03 | 0.03 | 0.5 | 0.25 | 8 | 64 | 0.25 | 0.25 |
| 56 | 0.125 | 0.125 | 0.5 | 0.25 | 4 | 64 | 0.25 | 0.25 |
| 57 | 0.125 | 0.125 | 0.5 | 0.25 | 4 | 64 | 0.25 | 0.25 |
| 58 | 0.125 | 0.125 | 0.5 | 0.25 | 4 | 64 | 0.25 | 0.25 |
| 59 | 0.25 | 0.25 | 0.5 | 0.25 | 8 | 64 | 0.25 | 0.25 |
| 60 | 0.25 | 0.25 | 0.5 | 0.25 | 8 | 64 | 0.25 | 0.25 |
| 61 | 0.06 | 0.06 | 0.5 | 0.25 | 4 | 64 | 0.25 | 0.25 |
| 62 | 0.06 | 0.06 | 0.5 | 0.25 | 4 | 64 | 0.25 | 0.25 |
| 63 | 0.125 | 0.125 | 0.5 | 0.25 | 4 | 64 | 0.25 | 0.25 |
| 64 | 0.25 | 0.25 | 0.5 | 0.25 | 8 | 64 | 0.25 | 0.25 |
| 65 | 0.25 | 0.25 | 0.5 | 0.25 | 8 | 64 | 0.25 | 0.25 |
| 66 | 0.125 | 0.125 | 0.5 | 0.25 | 4 | 64 | 0.25 | 0.25 |
| 67 | 0.25 | 0.25 | 0.5 | 0.25 | 4 | 64 | 0.25 | 0.25 |
| 68 | 0.25 | 0.25 | 0.5 | 0.25 | 4 | 64 | 0.25 | 0.25 |
| 69 | 0.5 | 0.5 | 0.5 | 0.25 | 8 | 64 | 0.25 | 0.25 |
| 70 | 0.5 | 0.5 | 0.5 | 0.25 | 8 | 64 | 0.25 | 0.25 |
| 71 | ≤0.03 | ≤0.03 | 0.5 | 0.25 | 64 | >64 | 0.06 | ≤0.03 |
| 72 | ≤0.03 | ≤0.03 | 0.5 | 0.25 | 64 | >64 | 0.06 | ≤0.03 |
| 73 | ≤0.03 | ≤0.03 | 0.5 | 0.25 | 64 | >64 | 0.06 | ≤0.03 |
| 74 | 0.06 | 0.06 | 0.5 | 0.25 | 64 | >64 | 0.06 | 0.03 |
| 75 | 0.06 | 0.06 | 0.5 | 0.25 | 64 | >64 | 0.06 | 0.03 |
| 76 | ≤0.03 | ≤0.03 | 0.5 | 0.25 | 64 | >64 | 0.06 | ≤0.03 |
| 77 | ≤0.03 | ≤0.03 | 0.5 | 0.25 | 64 | >64 | 0.06 | ≤0.03 |
| 78 | ≤0.03 | ≤0.03 | 0.5 | 0.25 | 64 | >64 | 0.06 | ≤0.03 |
| 79 | 0.03 | 0.03 | 0.5 | 0.25 | 64 | >64 | 0.06 | 0.03 |
| 80 | 0.06 | 0.06 | 0.5 | 0.25 | 64 | >64 | 0.06 | 0.03 |
| 81 | 0.03 | 0.03 | 0.5 | 0.25 | 64 | >64 | 0.06 | ≤0.03 |
| 82 | 0.03 | 0.03 | 0.5 | 0.25 | 64 | >64 | 0.06 | ≤0.03 |
| 83 | 0.06 | 0.06 | 0.5 | 0.25 | 64 | >64 | 0.06 | ≤0.03 |
| 84 | 0.25 | 0.25 | 0.5 | 0.25 | 64 | >64 | 0.06 | 0.03 |
| 85 | 0.25 | 0.25 | 0.5 | 0.25 | 64 | >64 | 0.06 | 0.03 |
| 86 | 0.03 | 0.03 | 0.5 | 0.25 | 64 | >64 | 0.06 | ≤0.03 |
| 87 | 0.03 | 0.03 | 0.5 | 0.25 | 64 | >64 | 0.06 | ≤0.03 |
| 88 | 0.03 | 0.03 | 0.5 | 0.25 | 64 | >64 | 0.06 | ≤0.03 |
| 89 | 0.125 | 0.125 | 0.5 | 0.25 | 64 | >64 | 0.06 | 0.03 |
| 90 | 0.25 | 0.25 | 0.5 | 0.25 | 64 | >64 | 0.06 | 0.03 |
| 91 | 0.03 | 0.03 | 0.03 | 0.03 | 0.5 | 0.5 | 0.03 | 0.03 |
| 92 | 0.03 | 0.03 | 0.03 | 0.03 | 0.5 | 0.5 | 0.03 | 0.03 |
| 93 | 0.03 | 0.03 | 0.03 | 0.03 | 1 | 1 | 0.03 | 0.03 |
| 94 | 0.06 | 0.06 | 0.03 | 0.03 | 1 | 1 | 0.03 | 0.03 |
| 95 | 0.125 | 0.125 | 0.06 | 0.06 | 1 | 1 | 0.06 | 0.06 |
| 96 | 0.03 | 0.03 | 0.03 | 0.03 | 0.5 | 0.5 | 0.03 | 0.03 |
| 97 | 0.03 | 0.03 | 0.03 | 0.03 | 0.5 | 0.5 | 0.03 | 0.03 |
| 98 | 0.03 | 0.03 | 0.03 | 0.03 | 0.5 | 0.5 | 0.03 | 0.03 |
| 99 | 0.06 | 0.06 | 0.03 | 0.03 | 1 | 1 | 0.03 | 0.03 |
| 100 | 0.125 | 0.125 | 0.06 | 0.06 | 1 | 1 | 0.06 | 0.06 |
| 101 | >64 | >64 | >64 | >64 | >64 | >64 | >64 | >64 |
| 102 | >64 | >64 | >64 | >64 | >64 | >64 | >64 | >64 |
| 103 | 0.03 | 0.03 | 0.125 | 0.125 | 0.25 | 0.25 | 0.06 | 0.06 |
| 104 | 0.03 | 0.03 | 0.125 | 0.125 | 0.25 | 0.25 | 0.06 | 0.06 |
| 105 | 0.03 | 0.03 | 0.125 | 0.125 | 0.25 | 0.25 | 0.06 | 0.06 |
| 106 | 0.03 | 0.03 | 0.125 | 0.125 | 0.25 | 0.25 | 0.06 | 0.06 |
| 107 | 0.03 | 0.03 | 0.125 | 0.125 | 0.25 | 0.25 | 0.06 | 0.06 |
| 108 | 0.06 | 0.06 | 0.125 | 0.125 | 0.5 | 0.25 | 0.25 | 0.06 |
| 109 | 0.06 | 0.06 | 0.125 | 0.125 | 0.5 | 0.25 | 0.25 | 0.06 |
| 110 | 0.06 | 0.06 | 0.125 | 0.125 | 0.5 | 0.25 | 0.25 | 0.06 |
| 111 | 0.06 | 0.06 | 0.125 | 0.125 | 0.5 | 0.25 | 0.25 | 0.06 |
| 112 | 0.03 | 0.03 | 0.125 | 0.125 | 0.25 | 0.25 | 0.06 | 0.06 |
| 113 | 0.03 | 0.03 | 0.125 | 0.125 | 0.25 | 0.25 | 0.06 | 0.06 |
| 114 | 0.03 | 0.03 | 0.125 | 0.125 | 0.25 | 0.25 | 0.06 | 0.06 |
| 115 | 0.03 | 0.03 | 0.125 | 0.125 | 0.25 | 0.25 | 0.06 | 0.06 |
| 116 | 0.03 | 0.03 | 0.125 | 0.125 | 0.25 | 0.25 | 0.06 | 0.06 |
| 117 | 0.06 | 0.06 | 0.125 | 0.125 | 0.5 | 0.25 | 0.25 | 0.06 |
| 118 | 0.06 | 0.06 | 0.125 | 0.125 | 0.5 | 0.25 | 0.25 | 0.06 |
| 119 | 0.06 | 0.06 | 0.125 | 0.125 | 0.5 | 0.25 | 0.25 | 0.06 |
| 120 | 0.06 | 0.06 | 0.125 | 0.125 | 0.5 | 0.25 | 0.25 | 0.06 |
| Polymyxin B | 0.03 | 0.03 | 0.125 | 0.125 | 0.25 | 0.25 | 0.25 | 0.06 |

### Experimental Example 2: Nephrotoxicity assay

*In vitro* cytotoxicity detection of HK-2 cells was performed by the CCK-8 assay.

The culture medium for human renal HK-2 cells was a complete RPMI-1640 medium (Invitrogen) supplemented with 10% fetal bovine serum (FBS, Gibco). The cryopreservation solution was composed of 90% FBS and 10% dimethyl sulfoxide (DMSO), and the cells were preserved long-term in liquid nitrogen. Cells were cultured in a sterile incubator with a humidified atmosphere containing 5% CO₂ at 37°C. When the cell density reached 70%-80%, cell digestion and subculture were conducted. The cyclic lipopeptide compounds prepared in Examples 1-120 were weighed and dissolved in sterile water to prepare a mother solution with a concentration of 1 mg/mL. Drug-containing culture media were then formulated, with the final concentrations set as 500, 250, 200, 100, 50, 25, and 12.5 µg/mL, respectively. Cells were adjusted to a density of 5 × 10⁴ cells/mL and seeded into 96-well plates, with 100 µL of cell suspension added to each well. After stable incubation for 24 h, the original medium was replaced with the prepared drug-containing medium, and the plates were incubated in the incubator for an additional 72 h. The medium was gently aspirated, and 100 µL of blank medium containing 10% CCK-8 was added to each well. After incubation in the incubator for 3 h, the optical density (OD) values at 450 nm were measured using a BIOTEK microplate reader. The experiment was repeated three times. For each replicate, polymyxin B was set as the positive drug, and a drug-free blank group was established as the control. The cell viability (%) was calculated by the formula: Cell viability (%) = [(OD of drug-treated group - OD of zero-setting) / (OD of drug-free group - OD of zero-setting)] × 100%. The half-maximal inhibitory concentration (IC₅₀) values were calculated through logarithmic regression analysis.

The cells used for the nephrotoxicity assay were obtained from the ATCC ( American Type Culture Collection). The Cell Counting Kit-8 (CCK-8) used in the assay was supplied by Beyotime Biotechnology Co., Ltd.

Test sample: Antibacterial lipopeptide compounds prepared according to the technical solutions of the present disclosure.

Reference standard: Polymyxin B.

### Results:

The nephrotoxicity results of partial cyclic lipopeptide compounds of the present disclosure were presented in Table 3.

**Table 3 Human Renal Cytotoxicity of Partial Compounds of the Present disclosure (IC₅₀, Unit: µg/mL)**

| Compound (µg/mL) | HK-2 cells |
|---|---|
| 1 | >500 |
| 2 | >500 |
| 3 | >500 |
| 4 | >500 |
| 5 | 380.5±30.5 |
| 6 | >500 |
| 7 | >500 |
| 8 | >500 |
| 9 | >500 |
| 10 | 350.1±30.7 |
| 11 | 370.7±54.6 |
| 12 | >500 |
| 13 | >500 |
| 14 | >500 |
| 15 | 320.5±31.5 |
| 16 | >500 |
| 17 | >500 |
| 18 | >500 |
| 19 | >500 |
| 20 | 280.5±10.3 |
| 21 | >500 |
| 22 | >500 |
| 23 | >500 |
| 24 | >500 |
| 25 | 337.7±15.1 |
| 26 | >500 |
| 27 | >500 |
| 28 | >500 |
| 29 | >500 |
| 30 | 270.1±13.1 |
| 31 | 221.7±10.2 |
| 32 | 325.1±11.7 |
| 33 | 339.2±9.1 |
| 34 | 297.5±3.7 |
| 35 | 137.8±8.3 |
| 36 | 237.1±5.3 |
| 37 | 317.8±10.2 |
| 38 | 218.9±9.4 |
| 39 | 357.1±8.1 |
| 40 | 157.8±6.4 |
| 41 | 258.7±7.8 |
| 42 | 315.6±6.7 |
| 43 | 328.2±8.6 |
| 44 | 307.5±9.7 |
| 45 | 138.8±6.7 |
| 46 | 287.1±10.7 |
| 47 | 357.8±11.2 |
| 48 | 238.9±7.4 |
| 49 | 367.1±8.7 |
| 50 | 149.8±5.2 |
| 51 | >500 |
| 52 | >500 |
| 53 | >500 |
| 54 | >500 |
| 55 | 300.5±10.8 |
| 56 | >500 |
| 57 | >500 |
| 58 | >500 |
| 59 | >500 |
| 60 | 280.8±9.5 |
| 61 | >500 |
| 62 | >500 |
| 63 | >500 |
| 64 | >500 |
| 65 | 370.4±31.8 |
| 66 | >500 |
| 67 | >500 |
| 68 | >500 |
| 69 | >500 |
| 70 | 383.8±29.5 |
| 71 | >500 |
| 72 | >500 |
| 73 | >500 |
| 74 | >500 |
| 75 | 309.5±19.8 |
| 76 | >500 |
| 77 | >500 |
| 78 | >500 |
| 79 | >500 |
| 80 | 287.8±19.5 |
| 81 | >500 |
| 82 | >500 |
| 83 | >500 |
| 84 | >500 |
| 85 | 357.3±28.8 |
| 86 | >500 |
| 87 | >500 |
| 88 | >500 |
| 89 | >500 |
| 90 | 319.8±23.6 |
| 91 | 125.7±19.2 |
| 92 | 228.1±18.7 |
| 93 | 239.3±19.1 |
| 94 | 298.1±30.7 |
| 95 | 112.8±10.3 |
| 96 | 207.1±13.2 |
| 97 | 217.2±11.2 |
| 98 | 238.3±9.9 |
| 99 | 317.2±18.1 |
| 100 | 117.1±15.6 |
| 101 | >500 |
| 102 | >500 |
| 103 | >500 |
| 104 | >500 |
| 105 | >500 |
| 106 | >500 |
| 107 | >500 |
| 108 | >500 |
| 109 | >500 |
| 110 | >500 |
| 111 | >500 |
| 112 | >500 |
| 113 | >500 |
| 114 | >500 |
| 115 | >500 |
| 116 | >500 |
| 117 | >500 |
| 118 | >500 |
| 119 | >500 |
| 120 | >500 |
| Polymyxin B | 66.3±8.3 |

### Experimental Example 3: Acute toxicity test of compounds 2 and 7 in healthy mice

Healthy ICR mice aged 5-6 weeks were selected, with 5-10 mice in each group, and administered via a single tail vein injection at an administration volume of 20 mL/kg. In the preliminary test, the drug dose concentrations that caused 100% mortality and no mortality in mice after a single intravenous injection were determined. Based on this dose range, 4-5 dose groups were set up, with a dose ratio of 1:0.5 to 1:0.8 between adjacent groups. The mortality of animals within 7 days after administration was recorded. According to the mortality rate of experimental animals in each group, the median lethal dose (LD₅₀) of the test samples, i.e., the dose causing 50% mortality of the tested animals, was calculated by the Bliss method.

No abnormalities were observed in mice in the vehicle control group after a single tail vein injection of the vehicle.

For Compound 2, the mortality rates at single tail vein injection doses of 30, 24, 19.2, and 15.36 mg/kg were 100% (3/3), 62.5% (5/8), 25% (2/8), and 0% (0/8), respectively, with an LD₅₀ of 22.03 mg/kg (95% confidence interval: 19.61-24.79). For Compound 7, the mortality rates at single tail vein injection doses of 30, 24, 19.2, and 15.36 mg/kg were 100% (3/3), 25% (2/8), 12.5% (1/8), and 0% (0/8), respectively, with an LD₅₀ of 24.43 mg/kg (95% confidence interval: 21.78-27.59).

### Experimental Example 4: In vivo antibacterial test of compound 7

*Pseudomonas aeruginosa* ATCC27853 was selected for the determination of *in vivo* antibacterial activity.

One day before infection, the test bacteria were inoculated onto the corresponding nutrient agar plates and cultured overnight. On the day of infection, single colonies grown on the plates were picked and suspended in sterile normal saline, and the suspension was mixed thoroughly to obtain the original bacterial solution. The bacterial solution was then adjusted to the required concentration with 5% dried yeast powder and prepared freshly prior to use.

Healthy ICR mice were randomly divided into groups, with 5 mice per group, half male and half female. Bacterial solutions of different dilutions were aspirated and injected intraperitoneally into mice at a volume of 0.5 mL per 20 g of body weight. The mortality of mice was recorded within 7 days after infection. The minimum bacterial dose causing 100% mortality in mice was defined as the minimum lethal dose (MLD), which was used as the infection dose for the *in vivo* protection test.

Drug administration was performed via tail vein injection at 2 h, 8 h, 24 h, and 30 h after infection, with a total of 4 administrations, and the administration volume was 20 mL/kg. For the preparation of drug solutions, Compound 7, together with the control drugs polymyxin B and meropenem, were dissolved in normal saline to the required concentrations for tail vein injection, and all solutions were prepared freshly prior to use.

In the preliminary test, the drug dose concentrations that caused 100% mortality and no mortality in mice after infection with the tested bacteria were determined. Based on this dose range, 4-5 dose groups were established, with a dose ratio of 1:0.5 to 1:0.8 between adjacent groups.

Mice were randomly grouped according to body weight, with 8 mice per group (half male and half female). Each mouse was intraperitoneally infected with the test bacterial solution at a volume of 0.5 mL per 20 g of body weight, followed by tail vein injection at 2 h, 8 h, 24 h, and 30 h after infection. Mice in the blank control group were not infected with bacterial solution but were only given an equal volume of vehicle at an administration volume of 20 mL/kg. The mortality of mice was observed and recorded for 7 consecutive days. According to the mortality data, the median effective dose (ED₅₀) and its 95% confidence interval were calculated by the Bliss method, and statistical analysis was performed.

The minimum lethal bacterial concentration of *Pseudomonas aeruginosa* ATCC27853 for intraperitoneal infection in mice was 4.2 × 10² CFU/mL, which was used as the infection dose in the formal test.

Mice in the infection control group were intraperitoneally inoculated with *Pseudomonas aeruginosa* ATCC27853 bacterial solution at the MLD concentration (4.0 × 10² CFU/mL). Infection symptoms began to appear 3-4 hours after inoculation, and all 8 animals died within 48 hours (8/8), with a mortality rate of 100%.

Polymyxin B was administered twice daily for 2 days. At the end of the observation period, the mortality rates of the 2 mg/kg and 1.4 mg/kg dose groups were 62.5% and 87.5%, respectively, while the mortality rates of both the 1.0 mg/kg and 0.7 mg/kg dose groups reached 100%.

Meropenem was administered twice daily for 2 days. At the end of the observation period, the mortality rate of the 20 mg/kg dose group was 0% (0/8); the mortality rates of the 10 mg/kg, 5 mg/kg, and 2.5 mg/kg dose groups were 37.5%, 62.5%, and 100%, respectively, with ED₅, ED₅₀, and ED₉₅ values of 2.91 mg/kg, 7.07 mg/kg, and 17.19 mg/kg, respectively.

Compound 7 was administered twice daily for 2 days. At the end of the observation period, the mortality rate of the 10 mg/kg dose group was 0% (0/8); the mortality rates of the 7 mg/kg, 4.9 mg/kg, and 3.4 mg/kg dose groups were 37.5%, 87.5%, and 100%, respectively, with ED₅, ED₅₀, and ED₉₅ values of 4.54 mg/kg, 6.38 mg/kg, and 8.96 mg/kg, respectively.

Due to space limitations, only part of the experiments was described in the specification. Other compounds of the present disclosure exhibited the same or similar therapeutic effects.

In summary, some of the polymyxin compounds prepared in the present disclosure exhibited low nephrotoxicity, low acute toxicity, and high *in vitro* and *in vivo* antibacterial activity, thus having great potential to be developed into a new class of clinical antibiotics.

## Claims

1. A cyclic alkaline lipopeptide compound having the structure represented by General Formula I or a pharmaceutically acceptable salt thereof, wherein the compound consists of three moieties: a cyclic heptapeptide, a linear tripeptide, and a side-chain acyl chain (i.e., R₀-CO-) linked to the linear tripeptide; AA represents an amino acid; the carboxyl group of R₀-COOH condenses with the α-amino group of AA₁ to form an amide bond; the carboxyl group of AA₁ condenses with the α-amino group of AA₂ to form an amide bond; the carboxyl group of AA₂ condenses with the α-amino group of AA₃ to form an amide bond; the carboxyl group of AA₃ condenses with the α-amino group of Dab₄ to form an amide bond; the carboxyl group of Dab₄ condenses with the α-amino group of AA₅ to form an amide bond; the carboxyl group of AA₅ condenses with the α-amino group of AA₆ to form an amide bond; the carboxyl group of AA₆ condenses with the α-amino group of AA₇ to form an amide bond; the carboxyl group of AA₇ condenses with the α-amino group of AA₈ to form an amide bond; the carboxyl group of AA₈ condenses with the α-amino group of AA₉ to form an amide bond; the carboxyl group of AA₉ condenses with the α-amino group of AA₁₀ to form an amide bond; and the carboxyl group of AA₁₀ condenses with the γ-amino group of Dab₄ to form an amide bond,
R₀-CO-AA₁-AA₂-D/L-AA₃-cyclo(4-10)[Dab₄-AA₅-D/L-AA₆-AA₇-AA₈-AA₉-AA₁₀] I
wherein:
R₀ is selected from the group consisting of (C₆-C₁₁) straight-chain alkyl and (C₇-C₁₂) branched-chain alkyl;
AA₁ and AA₃ are each independently selected from the group consisting of Ser (serine), Ser(CH₂SO₃H) (2-amino-3-sulfomethoxypropanoic acid), Dap (2,3-diaminopropanoic acid), Dap(CH₂SO₃H) (2-amino-3-sulfomethylaminopropanoic acid), Dab (2,4-diaminobutyric acid), Dab(CH₂SO₃H) (2-amino-4-sulfomethylaminobutyric acid), Orn (ornithine) and Lys (lysine); the amino acid at position 1 has an L configuration, and the amino acid at position 3 has a D or L configuration;
AA₂ and AA₁₀ are each independently selected from the group consisting of Thr (threonine) and Thr(CH₂SO₃H) (2-amino-3-sulfomethoxybutanoic acid); the amino acids at positions 2 and 10 have an L configuration;
Dab₄ has an L configuration;
AA₅, AA₈ and AA₉ are each independently selected from the group consisting of Dap (2,3-diaminopropanoic acid), Dap(CH₂SO₃H) (2-amino-3-(sulfomethylamino)propanoic acid), Dab (2,4-diaminobutyric acid), Dab(CH₂SO₃H) (2-amino-4-(sulfomethylamino)butyric acid) and Orn (omithine); the amino acids at positions 5, 8 and 9 are in L-configuration;
AA₆ and AA₇ are each independently selected from the group consisting of Phe (phenylalanine), Phe(4-CH₂SO₃) (4-(sulfomethyl)phenylalanine), Leu (leucine), Thr (threonine) and Thr(CH₂SO₃H) (2-amino-3-(sulfomethoxy)butanoic acid); the amino acid at position 6 has a D or L configuration, and the amino acid at position 7 has an L configuration;
The straight-chain alkyl may be hexyl, heptyl, octyl or nonyl; the branched-chain alkyl may be 5-methylhexyl, 5-methylheptyl, 6-methylheptyl or 6-methyloctyl, preferably (S)-5-methylheptyl.

2. The cyclic alkaline lipopeptide compound having the structure represented by general formula I or the pharmaceutically acceptable salt thereof of claim 1, wherein the cyclic lipopeptide compound is selected from the group consisting of:
(1) (S)-6-methyloctanoyl-Dab-Thr-D-Dap-cyclo(4-10)[Dab-Dab-D-Phe-Thr-Dab-Dab-Thr];
(2) 6-methylheptanoyl-Dab-Thr-D-Dap-cyclo(4-10)[Dab-Dab-D-Phe-Thr-Dab-Dab-Thr];
(3) Octanoyl-Dab-Thr-D-Dap-cyclo(4-10)[Dab-Dab-D-Phe-Thr-Dab-Dab-Thr];
(4) Heptanoyl-Dab-Thr-D-Dap-cyclo(4-10)[Dab-Dab-D-Phe-Thr-Dab-Dab-Thr];
(5) Nonanoyl-Dab-Thr-D-Dap-cyclo(4-10)[Dab-Dab-D-Phe-Thr-Dab-Dab-Thr];
(6) (S)-6-methyloctanoyl-Dab-Thr-Dap-cyclo(4-10)[Dab-Dab-D-Phe-Thr-Dab-Dab-Thr];
(7) 6-methylheptanoyl-Dab-Thr-Dap-cyclo(4-10)[Dab-Dab-D-Phe-Thr-Dab-Dab-Thr];
(8) Octanoyl-Dab-Thr-Dap-cyclo(4-10)[Dab-Dab-D-Phe-Thr-Dab-Dab-Thr];
(9) Heptanoyl-Dab-Thr-Dap-cyclo(4-10)[Dab-Dab-D-Phe-Thr-Dab-Dab-Thr];
(10) Nonanoyl-Dab-Thr-Dap-cyclo(4-10)[Dab-Dab-D-Phe-Thr-Dab-Dab-Thr];
(11) (S)-6-methyloctanoyl-Dab-Thr-D-Dab-cyclo(4-10)[Dab-Dab-D-Phe-Thr-Dab-Dab-Thr];
(12) 6-methylheptanoyl-Dab-Thr-D-Dab-cyclo(4-10)[Dab-Dab-D-Phe-Thr-Dab-Dab-Thr];
(13) Octanoyl-Dab-Thr-D-Dab-cyclo(4-10)[Dab-Dab-D-Phe-Thr-Dab-Dab-Thr];
(14) Heptanoyl-Dab-Thr-D-Dab-cyclo(4-10)[Dab-Dab-D-Phe-Thr-Dab-Dab-Thr];
(15) Nonanoyl-Dab-Thr-D-Dab-cyclo(4-10)[Dab-Dab-D-Phe-Thr-Dab-Dab-Thr];
(16) (S)-6-methyloctanoyl-Dab-Thr-Dab-cyclo(4-10)[Dab-Dab-D-Phe-Thr-Dab-Dab-Thr];
(17) 6-methylheptanoyl-Dab-Thr-Dab-cyclo(4-10)[Dab-Dab-D-Phe-Thr-Dab-Dab-Thr];
(18) Octanoyl-Dab-Thr-Dab-cyclo(4-10)[Dab-Dab-D-Phe-Thr-Dab-Dab-Thr];
(19) Heptanoyl-Dab-Thr-Dab-cyclo(4-10)[Dab-Dab-D-Phe-Thr-Dab-Dab-Thr];
(20) Nonanoyl-Dab-Thr-Dab-cyclo(4-10)[Dab-Dab-D-Phe-Thr-Dab-Dab-Thr];
(21) (S)-6-methyloctanoyl-Dab-Thr-D-Orn-cyclo(4-10)[Dab-Dab-D-Phe-Thr-Dab-Dab-Thr];
(22) 6-methylheptanoyl-Dab-Thr-D-Orn-cyclo(4-10)[Dab-Dab-D-Phe-Thr-Dab-Dab-Thr];
(23) Octanoyl-Dab-Thr-D-Orn-cyclo(4-10)[Dab-Dab-D-Phe-Thr-Dab-Dab-Thr];
(24) Heptanoyl-Dab-Thr-D-Orn-cyclo(4-10)[Dab-Dab-D-Phe-Thr-Dab-Dab-Thr];
(25) Nonanoyl-Dab-Thr-D-Orn-cyclo(4-10)[Dab-Dab-D-Phe-Thr-Dab-Dab-Thr];
(26) (S)-6-methyloctanoyl-Dab-Thr-Orn-cyclo(4-10)[Dab-Dab-D-Phe-Thr-Dab-Dab-Thr];
(27) 6-methylheptanoyl-Dab-Thr-Orn-cyclo(4-10)[Dab-Dab-D-Phe-Thr-Dab-Dab-Thr];
(28) Octanoyl-Dab-Thr-Orn-cyclo(4-10)[Dab-Dab-D-Phe-Thr-Dab-Dab-Thr];
(29) Heptanoyl-Dab-Thr-Orn-cyclo(4-10)[Dab-Dab-D-Phe-Thr-Dab-Dab-Thr];
(30) Nonanoyl-Dab-Thr-Orn-cyclo(4-10)[Dab-Dab-D-Phe-Thr-Dab-Dab-Thr];
(31) (S)-6-methyloctanoyl-Dab-Thr-D-Dap-cyclo(4-10)[Dab-Dab-D-Phe-Leu-Dab-Dab-Thr];
(32) 6-methylheptanoyl-Dab-Thr-D-Dap-cyclo(4-10)[Dab-Dab-D-Phe-Leu-Dab-Dab-Thr];
(33) Octanoyl-Dab-Thr-D-Dap-cyclo(4-10)[Dab-Dab-D-Phe-Leu-Dab-Dab-Thr];
(34) Heptanoyl-Dab-Thr-D-Dap-cyclo(4-10)[Dab-Dab-D-Phe-Leu-Dab-Dab-Thr];
(35) Nonanoyl-Dab-Thr-D-Dap-cyclo(4-10)[Dab-Dab-D-Phe-Leu-Dab-Dab-Thr];
(36) (S)-6-methyloctanoyl-Dab-Thr-Dap-cyclo(4-10)[Dab-Dab-D-Phe-Leu-Dab-Dab-Thr];
(37) 6-methylheptanoyl-Dab-Thr-Dap-cyclo(4-10)[Dab-Dab-D-Phe-Leu-Dab-Dab-Thr];
(38) Octanoyl-Dab-Thr-Dap-cyclo(4-10)[Dab-Dab-D-Phe-Leu-Dab-Dab-Thr];
(39) Heptanoyl-Dab-Thr-Dap-cyclo(4-10)[Dab-Dab-D-Phe-Leu-Dab-Dab-Thr];
(40) Nonanoyl-Dab-Thr-Dap-cyclo(4-10)[Dab-Dab-D-Phe-Leu-Dab-Dab-Thr];
(41) (S)-6-methyloctanoyl-Dab-Thr-D-Orn-cyclo(4-10)[Dab-Dab-D-Phe-Leu-Dab-Dab-Thr];
(42) 6-methylheptanoyl-Dab-Thr-D-Orn-cyclo(4-10)[Dab-Dab-D-Phe-Leu-Dab-Dab-Thr];
(43) Octanoyl-Dab-Thr-D-Orn-cyclo(4-10)[Dab-Dab-D-Phe-Leu-Dab-Dab-Thr];
(44) Heptanoyl-Dab-Thr-D-Orn-cyclo(4-10)[Dab-Dab-D-Phe-Leu-Dab-Dab-Thr];
(45) Nonanoyl-Dab-Thr-D-Orn-cyclo(4-10)[Dab-Dab-D-Phe-Leu-Dab-Dab-Thr];
(46) (S)-6-methyloctanoyl-Dab-Thr-Orn-cyclo(4-10)[Dab-Dab-D-Phe-Leu-Dab-Dab-Thr];
(47) 6-methylheptanoyl-Dab-Thr-Orn-cyclo(4-10)[Dab-Dab-D-Phe-Leu-Dab-Dab-Thr];
(48) Octanoyl-Dab-Thr-Orn-cyclo(4-10)[Dab-Dab-D-Phe-Leu-Dab-Dab-Thr];
(49) Heptanoyl-Dab-Thr-Orn-cyclo(4-10)[Dab-Dab-D-Phe-Leu-Dab-Dab-Thr];
(50) Nonanoyl-Dab-Thr-Orn-cyclo(4-10)[Dab-Dab-D-Phe-Leu-Dab-Dab-Thr];
(51) (S)-6-methyloctanoyl-Dap-Thr-D-Ser-cyclo(4-10)[Dab-Dab-D-Phe-Thr-Dab-Dab-Thr];
(52) 6-methylheptanoyl-Dap-Thr-D-Ser-cyclo(4-10)[Dab-Dab-D-Phe-Thr-Dab-Dab-Thr];
(53) Octanoyl-Dap-Thr-D-Ser-cyclo(4-10)[Dab-Dab-D-Phe-Thr-Dab-Dab-Thr];
(54) Heptanoyl-Dap-Thr-D-Ser-cyclo(4-10)[Dab-Dab-D-Phe-Thr-Dab-Dab-Thr];
(55) Nonanoyl-Dap-Thr-D-Ser-cyclo(4-10)[Dab-Dab-D-Phe-Thr-Dab-Dab-Thr];
(56) (S)-6-methyloctanoyl-Orn-Thr-D-Ser-cyclo(4-10)[Dab-Dab-D-Phe-Thr-Dab-Dab-Thr];
(57) 6-methylheptanoyl-Orn-Thr-D-Ser-cyclo(4-10)[Dab-Dab-D-Phe-Thr-Dab-Dab-Thr];
(58) Octanoyl-Orn-Thr-D-Ser-cyclo(4-10)[Dab-Dab-D-Phe-Thr-Dab-Dab-Thr];
(59) Heptanoyl-Orn-Thr-D-Ser-cyclo(4-10)[Dab-Dab-D-Phe-Thr-Dab-Dab-Thr];
(60) Nonanoyl-Orn-Thr-D-Ser-cyclo(4-10)[Dab-Dab-D-Phe-Thr-Dab-Dab-Thr];
(61) (S)-6-methyloctanoyl-Dab-Thr-D-Ser-cyclo(4-10)[Dab-Dap-D-Phe-Thr-Dab-Dab-Thr];
(62) 6-methylheptanoyl-Dab-Thr-D-Ser-cyclo(4-10)[Dab-Dap-D-Phe-Thr-Dab-Dab-Thr];
(63) Octanoyl-Dab-Thr-D-Ser-cyclo(4-10)[Dab-Dap-D-Phe-Thr-Dab-Dab-Thr];
(64) Heptanoyl-Dab-Thr-D-Ser-cyclo(4-10)[Dab-Dap-D-Phe-Thr-Dab-Dab-Thr];
(65) Nonanoyl-Dab-Thr-D-Ser-cyclo(4-10)[Dab-Dap-D-Phe-Thr-Dab-Dab-Thr];
(66) (S)-6-methyloctanoyl-Dab-Thr-D-Ser-cyclo(4-10)[Dab-Orn-D-Phe-Thr-Dab-Dab-Thr];
(67) 6-methylheptanoyl-Dab-Thr-D-Ser-cyclo(4-10)[Dab-Orn-D-Phe-Thr-Dab-Dab-Thr];
(68) Octanoyl-Dab-Thr-D-Ser-cyclo(4-10)[Dab-Orn-D-Phe-Thr-Dab-Dab-Thr];
(69) Heptanoyl-Dab-Thr-D-Ser-cyclo(4-10)[Dab-Orn-D-Phe-Thr-Dab-Dab-Thr];
(70) Nonanoyl-Dab-Thr-D-Ser-cyclo(4-10)[Dab-Orn-D-Phe-Thr-Dab-Dab-Thr];
(71) (S)-6-methyloctanoyl-Dab-Thr-D-Ser-cyclo(4-10)[Dab-Dab-D-Phe-Thr-Dap-Dab-Thr];
(72) 6-methylheptanoyl-Dab-Thr-D-Ser-cyclo(4-10)[Dab-Dab-D-Phe-Thr-Dap-Dab-Thr];
(73) Octanoyl-Dab-Thr-D-Ser-cyclo(4-10)[Dab-Dab-D-Phe-Thr-Dap-Dab-Thr];
(74) Heptanoyl-Dab-Thr-D-Ser-cyclo(4-10)[Dab-Dab-D-Phe-Thr-Dap-Dab-Thr];
(75) Nonanoyl-Dab-Thr-D-Ser-cyclo(4-10)[Dab-Dab-D-Phe-Thr-Dap-Dab-Thr];
(76) (S)-6-methyloctanoyl-Dab-Thr-D-Ser-cyclo(4-10)[Dab-Dab-D-Phe-Thr-Orn-Dab-Thr];
(77) 6-methylheptanoyl-Dab-Thr-D-Ser-cyclo(4-10)[Dab-Dab-D-Phe-Thr-Orn-Dab-Thr];
(78) Octanoyl-Dab-Thr-D-Ser-cyclo(4-10)[Dab-Dab-D-Phe-Thr-Orn-Dab-Thr];
(79) Heptanoyl-Dab-Thr-D-Ser-cyclo(4-10)[Dab-Dab-D-Phe-Thr-Orn-Dab-Thr];
(80) Nonanoyl-Dab-Thr-D-Ser-cyclo(4-10)[Dab-Dab-D-Phe-Thr-Orn-Dab-Thr];
(81) (S)-6-methyloctanoyl-Dab-Thr-D-Ser-cyclo(4-10)[Dab-Dab-D-Phe-Thr-Dab-Dap-Thr];
(82) 6-methylheptanoyl-Dab-Thr-D-Ser-cyclo(4-10)[Dab-Dab-D-Phe-Thr-Dab-Dap-Thr];
(83) Octanoyl-Dab-Thr-D-Ser-cyclo(4-10)[Dab-Dab-D-Phe-Thr-Dab-Dap-Thr];
(84) Heptanoyl-Dab-Thr-D-Ser-cyclo(4-10)[Dab-Dab-D-Phe-Thr-Dab-Dap-Thr];
(85) Nonanoyl-Dab-Thr-D-Ser-cyclo(4-10)[Dab-Dab-D-Phe-Thr-Dab-Dap-Thr];
(86) (S)-6-methyloctanoyl-Dab-Thr-D-Ser-cyclo(4-10)[Dab-Dab-D-Phe-Thr-Dab-Orn-Thr];
(87) 6-methylheptanoyl-Dab-Thr-D-Ser-cyclo(4-10)[Dab-Dab-D-Phe-Thr-Dab-Orn-Thr];
(88) Octanoyl-Dab-Thr-D-Ser-cyclo(4-10)[Dab-Dab-D-Phe-Thr-Dab-Orn-Thr];
(89) Heptanoyl-Dab-Thr-D-Ser-cyclo(4-10)[Dab-Dab-D-Phe-Thr-Dab-Orn-Thr];
(90) Nonanoyl-Dab-Thr-D-Ser-cyclo(4-10)[Dab-Dab-D-Phe-Thr-Dab-Orn-Thr];
(91) (S)-6-methyloctanoyl-Dap-Thr-D-Dab-cyclo(4-10)[Dab-Dab-D-Phe-Leu-Dab-Dab-Thr];
(92) 6-methylheptanoyl-Dap-Thr-D-Dab-cyclo(4-10)[Dab-Dab-D-Phe-Leu-Dab-Dab-Thr];
(93) Octanoyl-Dap-Thr-D-Dab-cyclo(4-10)[Dab-Dab-D-Phe-Leu-Dab-Dab-Thr];
(94) Heptanoyl-Dap-Thr-D-Dab-cyclo(4-10)[Dab-Dab-D-Phe-Leu-Dab-Dab-Thr];
(95) Nonanoyl-Dap-Thr-D-Dab-cyclo(4-10)[Dab-Dab-D-Phe-Leu-Dab-Dab-Thr];
(96) (S)-6-methyloctanoyl-Dap-Thr-D-Dab-cyclo(4-10)[Dab-Dab-D-Leu-Leu-Dab-Dab-Thr];
(97) 6-methylheptanoyl-Dap-Thr-D-Dab-cyclo(4-10)[Dab-Dab-D-Leu-Leu-Dab-Dab-Thr];
(98) Octanoyl-Dap-Thr-D-Dab-cyclo(4-10)[Dab-Dab-D-Leu-Leu-Dab-Dab-Thr];
(99) Heptanoyl-Dap-Thr-D-Dab-cyclo(4-10)[Dab-Dab-D-Leu-Leu-Dab-Dab-Thr];
(100) Nonanoyl-Dap-Thr-D-Dab-cyclo(4-10)[Dab-Dab-D-Leu-Leu-Dab-Dab-Thr];
(101) (S)-6-methyloctanoyl-Dab-Thr-D-Dap-cyclo(4-10)[Dab-Dab-Phe-Thr-Dab-Dab-Thr];
(102) 6-methylheptanoyl-Dab-Thr-D-Dap-cyclo(4-10)[Dab-Dab-Phe-Thr-Dab-Dab-Thr];
(103) 6-methylheptanoyl-Dab-Thr-D-Ser(CH₂SO₃H)-cyclo(4-10)[Dab-Dab-D-Phe-Thr-Dab-Dab-Thr];
(104) 6-methylheptanoyl-Dab-Thr(CH₂SO₃H)-D-Ser-cyclo(4-10)[Dab-Dab-D-Phe-Thr-Dab-Dab-Thr];
(105) 6-methylheptanoyl-Dab-Thr-D-Ser-cyclo(4-10)[Dab-Dab-D-Phe-Thr(CH₂SO₃H)-Dab-Dab-Thr];
(106) 6-methylheptanoyl-Dab-Thr-D-Ser-cyclo(4-10)[Dab-Dab-D-Phe-Thr-Dab-Dab-Thr(CH₂SO₃H)];
(107) 6-methylheptanoyl-Dab-Thr-D-Ser-cyclo(4-10)[Dab-Dab-D-Phe(4-CH2SO3H)-Thr-Dab-Dab-Thr];
(108) 6-methylheptanoyl-Dab(CH₂SO₃H)-Thr-D-Ser-cyclo(4-10)[Dab-Dab-D-Phe-Thr-Dab-Dab-Thr];
(109) 6-methylheptanoyl-Dab-Thr-D-Ser-cyclo(4-10)[Dab-Dab(CH₂SO₃H)-D-Phe-Thr-Dab-Dab-Thr];
(110) 6-methylheptanoyl-Dab-Thr-D-Ser-cyclo(4-10)[Dab-Dab-D-Phe-Thr-Dab(CH₂SO₃H)-Dab-Thr];
(111) 6-methylheptanoyl-Dab-Thr-D-Ser-cyclo(4-10)[Dab-Dab-D-Phe-Thr-Dab-Dab(CH₂SO₃H)-Thr];
(112) 6-methylheptanoyl-Dab-Thr-Dap(CH₂SO₃H)-cyclo(4-10)[Dab-Dab-D-Phe-Thr-Dab-Dab-Thr];
(113) 6-methylheptanoyl-Dab-Thr(CH₂SO₃H)-Dap-cyclo(4-10)[Dab-Dab-D-Phe-Thr-Dab-Dab-Thr];
(114) 6-methylheptanoyl-Dab-Thr-Dap-cyclo(4-10)[Dab-Dab-D-Phe-Thr(CH₂SO₃H)-Dab-Dab-Thr];
(115) 6-methylheptanoyl-Dab-Thr-Dap-cyclo(4-10)[Dab-Dab-D-Phe-Thr-Dab-Dab-Thr(CH₂SO₃H)];
(116) 6-methylheptanoyl-Dab-Thr-Dap-cyclo(4-10)[Dab-Dab-D-Phe(4-CH₂SO₃H)-Thr-Dab-Dab-Thr];
(117) 6-methylheptanoyl-Dab(CH₂SO₃H)-Thr-Dap-cyclo(4-10)[Dab-Dab-D-Phe-Thr-Dab-Dab-Thr];
(118) 6-methylheptanoyl-Dab-Thr-Dap-cyclo(4-10)[Dab-Dab(CH₂SO₃H)-D-Phe-Thr-Dab-Dab-Thr];
(119) 6-methylheptanoyl-Dab-Thr-Dap-cyclo(4-10)[Dab-Dab-D-Phe-Thr-Dab(CH₂SO₃H)-Dab-Thr];
(120) 6-methylheptanoyl-Dab-Thr-Dap-cyclo(4-10)[Dab-Dab-D-Phe-Thr-Dab-Dab(CH₂SO₃H)-Thr].

3. The cyclic alkaline lipopeptide compound having the structure represented by general formula I or the pharmaceutically acceptable salt thereof of claim 1 or 2, wherein the pharmaceutically acceptable salt of the compound of general formula I comprises a salt formed by the compound of general formula I and an acid, and the acid is selected from the group consisting of inorganic acids and organic acids, examples of the inorganic acids are perchloric acid, hydroiodic acid, hydrobromic acid, hydrochloric acid, sulfuric acid, nitric acid or phosphoric acid; examples of the organic acids are acetic acid, trifluoroacetic acid, lactic acid, succinic acid, fumaric acid, maleic acid, citric acid, benzoic acid, methanesulfonic acid or p-toluenesulfonic acid.

4. A pharmaceutical composition, wherein the pharmaceutical composition comprises the cyclic alkaline lipopeptide compound having the structure represented by general formula I or the pharmaceutically acceptable salt thereof of claims 1 to 3, and a pharmaceutically acceptable carrier or excipient.

5. Use of the cyclic alkaline lipopeptide compound having the structure represented by general formula I or the pharmaceutically acceptable salt thereof of claims 1 to 4 in the preparation of an antibacterial medicament.

6. The use of claim 5, wherein the antibacterial medicament is a medicament against Gram-negative bacteria.

7. A method for preparing the compound or the pharmaceutically acceptable salt thereof of claims 1 to 3, comprising the following steps:
(1) reacting an Fmoc-AA-OP side chain free amino group of a protected alkaline amino acid with a halogenated resin to obtain Fmoc-AA-OP-resin; wherein P is a carboxyl-protecting group selected from allyl and benzyl (Bn); when Fmoc-AA-OP is Fmoc-Dab-OP, the structure thereof is represented by formula II; when Fmoc-AA-OP is Fmoc-Dap-OP, the structure thereof is represented by formula III; when Fmoc-AA-OP is Fmoc-Om-OP, the structure thereof is represented by formula IV: 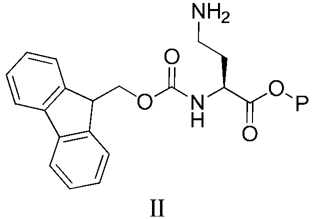 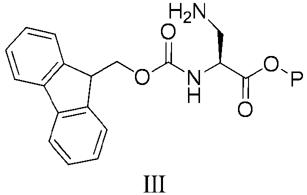 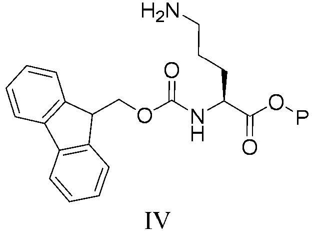
(2) coupling Fmoc-AA-OP-resin one by one to obtain a linear polypeptide-resin;
(3) selectively removing a protective group from the linear polypeptide-resin and carrying out solid-phase cyclization on the linear polypeptide-resin to obtain a cyclic polypeptide-resin;
(4) carrying out acidolysis on the cyclic polypeptide-resin to obtain a cyclic polypeptide crude product;
(5) carrying out purification and/or salt conversion and lyophilization on the cyclic polypeptide crude product to obtain a cyclic polypeptide pure product.

8. The method of claim 7, comprising the following steps:
(1) preparation of Fmoc-AA-OP-resin: adding a halogenated resin into a solid-phase polypeptide synthesis tube, adding DCM for swelling; after swelling, washing three times with DMF and three times with DCM; dissolving the protected starting amino acid Fmoc-AA-OP and a base in DCM, adding the solution into the peptide synthesis tube, reacting at room temperature for 2 h; vacuumizing to remove the reaction solution; washing the resin with DMF three times and DCM three times to obtain Fmoc-AA-OP-resin;
(2) coupling synthesis method: treating the Fmoc-AA-OP-resin obtained in the step (1) with 20% piperidine/DMF twice for 10 min each time to remove the α-amino Fmoc-protecting group; washing three times with DMF and three times with DCM; dissolving an amino acid or a side-chain carboxylic acid, DIEA and HCTU in DMF, adding the solution into the peptide synthesis tube, reacting at room temperature for 120 min; vacuumizing to remove the reaction solution; washing three times with DMF and three times with DCM; sequentially coupling amino acids from position 8 to position 1, and then coupling the side-chain carboxylic acid to the protected polypeptide-resin; removing the ivDde or Dde protecting group on the side-chain amino group of the amino acid at position 4 with 2% hydrazine hydrate/DMF solution; washing three times with DMF and three times with DCM; coupling the carboxyl group of the amino acid at position 10 to the side-chain amino group of the amino acid at position 4; or sequentially coupling amino acids from position 7 to position 1, and then coupling the side-chain carboxylic acid to the protected polypeptide-resin; removing the ivDde or Dde protecting group on the side-chain amino group of the amino acid at position 4 with 2% hydrazine hydrate/DMF solution; washing three times with DMF and three times with DCM; coupling the carboxyl group of the amino acid at position 10 to the side-chain amino group of the amino acid at position 4, and then coupling the carboxyl group of the amino acid at position 9 to the α-amino group of the amino acid at position 10;
(3) selective removal of protecting groups and solid-phase cyclization: treating the fully protected linear polypeptide-resin obtained in the step (2) with 20% piperidine/DMF twice for 10 min each time to remove the α-amino Fmoc-protecting group; washing three times with DMF and three times with DCM to form a free amino group; removing the carboxyl allyl-protecting group with tetrakis(triphenylphosphine)palladium/phenylsilane in a mixed solution of DCM and DMF to form a free carboxyl group; dissolving PyAOP and HOAT in DMF, adding NMM, adding the mixture into a peptide synthesis tube, reacting at room temperature for 3 h; vacuumizing to remove the reaction solution; washing the mixture three times with DMF and three times with DCM to obtain a fully protected cyclic polypeptide-resin;
(4) obtaining a cyclic alkaline lipopeptide crude product by acid hydrolysis: adding an acidolysis solution with a TFA/H₂O volume ratio of 95:5 into the peptide synthesis tube, reacting at room temperature for 120 min; adding the TFA cleavage solution into cold diethyl ether at a volume ratio of 1:20 to precipitate the peptide; centrifuging and drying to obtain a crude product;
(5) purification, salt conversion and lyophilization of the crude product: dissolving the crude product in water, filtering through a filter membrane with a pore size of 0.22 µm for later use; performing preparative high-performance liquid chromatography using a reversed-phase C18 packing material with a particle size of 10 µm, with mobile phase A of 0.08% TFA/water solution and mobile phase B of 0.08% TFA/acetonitrile solution; performing gradient elution with a gradient system and cyclic sample injection for purification using a 22 mm×250 mm chromatographic column with a flow rate of 8 mL/min and a detection wavelength of 214 nm; loading the crude product solution onto the chromatographic column, starting mobile phase elution, collecting fractions corresponding to the main chromatographic peak; evaporating acetonitrile from the fractions to obtain an aqueous polypeptide solution; lyophilizing to obtain the product.

9. The method of claim 7 or 8, wherein the halogenated resin is selected from the group consisting of trityl chloride resin, 4-methyltrityl chloride resin, 4-methoxytrityl chloride resin, 2-chlorotrityl chloride resin, bromo-(4-methylphenyl)-methyl resin or bromo-(4-methoxyphenyl)-methyl resin; preferably, the resin is 2-chlorotrityl chloride resin.

10. The method of claim 7 or 8, wherein in the step (1), the degree of substitution of the halogenated resin is 0.1-1.6 mmol/g, preferably 0.5-1.0 mmol/g;
the dosage of each Fmoc-protected amino acid is 1.2-6 times the total molar amount of the loaded resin, preferably 2.0-4.0 times;
the base is selected from at least one of N,N-diisopropylethylamine (DIEA), triethylamine (TEA) and pyridine, the molar dosage of the base is 1.5-3 times the molar amount of the Fmoc-protected amino acid;
the substitution reaction time is 1-12 h.
